# EUROPEAN PATENT APPLICATION

(11) **EP 4 506 348 A1**
(43) Date of publication of application: **12.02.2025**
(21) Application number: 23778071.3
(22) Date of filing: 27.03.2023
(51) Int. Cl.: C07D 487/04, C07D 487/14, C07D 498/04, C07D 513/04, A61K 31/497, A61K 31/4985, A61K 31/501, A61K 31/5365, A61K 31/542, A61P 25/28

(54) **ISOXAZOLE-HETEROCYCLIC DERIVATIVE, PHARMACEUTICAL COMPOSITION AND USE**

(30) Priority: 28.03.2022 CN 202210312824
(71) Applicant: Shanghai Simrd Biotechnology Co., Ltd, Shanghai 201318 (CN)
(72) Inventor: WANG, Fei, Shanghai 201318 (CN); WU, Jinhua, Shanghai 201318 (CN); LIU, Yang, Shanghai 201318 (CN); YANG, Fei, Shanghai 201318 (CN)
(74) Representative: Zanoli, Enrico
(86) International application number: PCT/CN2023/083976
(87) International publication number: WO 2023/185704

(57) **Abstract**

The invention relates to an isoxazole-heterocyclic derivative, a pharmaceutical composition and use, and specifically provides a compound represented by formula (I) or a pharmaceutically acceptable salt, a stereoisomer, a tautomer, a prodrug, an amorphous form, an isotopologue, a polymorph or a solvate thereof, a pharmaceutical composition comprising the compound and use of the compound as α5-GABA_{A} receptor regulator.

## Description

### Cross-reference

The invention claims the priority to the Chinese patent application with the application No. 202210312824.7 and the title "isoxazole-heterocyclic derivative, pharmaceutical composition and use" filed with the China National Intellectual Property Administration on March 28, 2022, which is incorporated herein by reference in its entirety.

### Technical field

The invention relates to isoxazole-heterocyclic derivative having regulatory function on α5-GABA_{A} receptor, pharmaceutical composition comprising the same, and use thereof.

### Background art

γ-Aminobutyric acid (GABA) is an important inhibitory neurotransmitter in the mammalian central nervous system, and there are two classes of GABA receptors present in nature, one of which is GABA_{A} receptor that is a member of the ligand-gated ion channel superfamily, and the other of which is GABA_{B} receptor that is a member of the G protein-coupled receptor superfamily. GABA_{A} receptor subunits in mammals have been found to include the subunits α1-6, β1-4, γ1-3, δ, ε, θ and ρ1-3, among which the α, β and γ subunits are essential for the formation of a fully functional GABA_{A} receptor, while the α subunit is critical for the binding of benzodiazepine to the GABA_{A} receptor.

The α5-containing GABA_{A} receptor (α5-GABA_{A} receptor) accounts for less than 5% of GABA_{A} receptors in the mammalian brain and is expressed at a very low level in the cerebral cortex, but accounts for more than 20% of GABA_{A} receptors in hippocampal tissues of the brain, and is hardly expressed in other brain regions. In view of the studies on the specific distribution and function of α5-GABA_{A} receptors in hippocampal tissues of the brain, many pharmaceutical companies, including Roche, MSD and the like, engaged in the study of α5-GABA_{A} receptor ligands, and subsequently a large number of compounds were synthesized, in particular, inverse regulators (or negative regulators, inverse agonists) of α5-GABA_{A} receptors in hippocampal tissues of the brain, wherein α5IA and MRK-016 showed good cognitive improvement effects in animal disease models. It is generally recognized that α5-GABA_{A} receptor inverse regulators may be useful in the treatment of cognitive disorders, in particular the treatment of Alzheimer's disease. Patent application US20110224278 discloses that inverse regulators of α5-GABA_{A} receptors can be used for the treatment of multi-infarct dementia and stroke related diseases.

Depression is a serious mental disease that can be life threatening (such as, suicide). The current standard therapy for antidepressant treatment is selective 5-hydroxytryptamine reuptake inhibitors (SSRI), but these drugs generally reach their maximum efficacy after 6-8 weeks of medication. Moreover, their effectiveness is limited, with only about half of patients showing therapeutic effects. Other marketed and clinically under-developed antidepressant drugs also have varying degrees of side effects that limit the use of them. Therefore, an antidepressant with a rapid onset function and lower side effects is urgently needed in clinic to relieve the burden of depressed patients and society.

Fishell *et al.* reports the antidepressant effect of α5-GABA_{A} receptor inverse regulators (J. Fischell et al., Neuropsychopharmacology, 2015, 40(11), 2499-2509), and believes that selective α5-GABA_{A} receptor inverse regulators can achieve a rapid onset effect different from traditional antidepressants, and their safety is superior to existing ketamine therapy with the same rapid onset ability. Thus, selective α5-GABA_{A} receptor inverse regulators may become a new mechanism of antidepressant therapy with rapid onset ability, meeting the serious unmet needs in the current clinical treatment of depression.

There have been many studies on determining whether a compound is an inverse regulator or antagonist of GABA_{A} receptors containing α5 subunit. For example, in international patent applications WO 1992022652 and WO 1994013799, a combination of α5, β3, and γ2 of GABA_{A} receptor is used to determine whether a compound binds to the receptor. In the process of drug screening, the method described by Goeders *et al.* (Goeders, N. E. and Kuhar, M. J. Life Sci. 1985, 37(4), 345-355) is commonly used. There are also many studies on determining whether a ligand that can bind to the GABA_{A} receptor α5 subunit is an antagonist, agonist, or inverse regulator, and reference can be made to the method described by Wafford *et al.* (Wafford, K. A., Mol. Pharmacol. 1993, 43, 240-244).

Recent findings indicate that GABA_{A} receptors mediate at least 2 modes of inhibition, i.e., phasic inhibition and tonic inhibition. The GABA_{A} receptors inside the synapse, due to action potential, cause synchronous release of GABA containing vesicles inside the synapse, resulting in a sharp increase in millimolar GABA concentration in the synaptic cleft, which leads to synchronous activation and rapid desensitization of the postsynaptic GABA_{A} receptors, to form the phasic inhibition. While the GABA_{A} receptors outside the synapse are usually in a sustained low concentration GABA_{A} environment ranging from tens of nanomoles to several millimoles, the GABA_{A} receptors with high affinity for GABA are continuously activated asynchronously, to form the tonic inhibition. Phasic inhibition and tonic inhibition jointly regulate neuronal excitability and signal transduction (Farrant, M. et al., Nat Rev Neurosci, 2005, 6, 215-229). Yeung JY *et al.* disclose that GABA at low concentrations more readily activates α5-GABA_{A} receptor (Yeung JY et al., Mol Pharmacol, 2003, 63, 2-8). K. Y. Lee reports that a persistent high-affinity GABA_{A} current activated by low concentrations of GABA is detected in isolated DRG cells cultured for 24 hours (Lee, K.Y. et al., Neuroscience, 2012, 208, 133-142). I. Lecker *et al.* disclose, in 2013, that the α5-GABA_{A} receptor inverse regulator L-655,708 dose-dependently inhibits the current induced by low concentrations (5, 50 and 500 nM) of GABA, with the highest concentration of L-655,708 being only able to inhibit 15% of the current when the GABA concentration increases to 1 µM, and having no inhibitory effect on the current induced by GABA as the GABA concentration continues to increase (Lecker, I. et al., British Journal of Anaestheia, 2013, 110(S1), i73-i81).

Target subtype selectivity is also important for the development of α5-GABA_{A} receptor inverse regulators. Because benzodiazepines, as non-selective GABA_{A} receptor positive regulators, have been used clinically for anti-anxiety and anti-epileptic treatment, the inverse regulators of this target may have the effect of promoting anxiety and epilepsy (H. J. Little, et al., Br J Pharmacol, 1984, 951-958). Therefore, the development of α5-GABA_{A} receptor inverse regulators needs to improve the binding and functional selectivity of α5-GABA_{A} receptors and avoid potential side effects of promoting anxiety and epilepsy (mainly caused by α2-GABA_{A} receptor activity) (Theresa M. Ballard et al., Psychopharmacography, 2009, 207-223). The previously disclosed α5-GABA_{A} receptor inverse regulators have insufficient stability in gastric juice and poor solubility in intestinal juice, and are not good in druggability when being developed as an oral drug and difficult to popularize and use.

### Summary

In one aspect, the invention provides a compound represented by formula (I) or a pharmaceutically acceptable salt, stereoisomer, tautomer, prodrug, amorphous form, isotopologue, polymorph or solvate thereof, which has good stability in the gastric juice and solubility for α5-GABA_{A}: 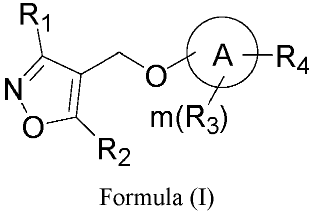
wherein, R₁ is selected from phenyl or pyridyl, wherein phenyl or pyridyl is optionally substituted with 1, 2 or 3 halogens;
R₂ is selected from H, linear or branched C1-C4 alkyl, C1-C4 alkoxy or C3-C6 cycloalkyl;
ring A is selected from a 5-10 membered heterocyclic group containing 1, 2 or 3 ring heteroatoms selected from N, P, O or S;
R₄ is selected from a fused group formed by heterocycle and heterocycle, the heterocycle for forming the fused group being selected from a 5-10 membered heterocyclic group containing 1, 2 or 3 heteroatoms selected from N, P, O or S; the fused group is unsubstituted or is substituted with one or more substituents selected from: optionally substituted alkyl, optionally substituted cycloalkyl, optionally substituted formyl, optionally substituted sulfonyl, or optionally substituted heterocycle containing 1, 2 or 3 heteroatoms; wherein the heteroatom is selected from N, P, O or S;
R₃ is selected from halogen, linear or branched C1-C4 alkyl, C1-C4 alkoxy, C3-C6 cycloalkyl;
m is 0, 1 or 2.

Unless otherwise specified, the following definitions are provided to illustrate and define the meanings and scopes of various terms used herein to describe the invention.

The following definitions of general terms apply whether alone or in combination.

The nomenclature used in the invention is based on AutoNomTM 2000, a Beilstein Institute computerized system used to generate IUPAC systematic nomenclature. Any open valency appearing on a carbon, oxygen, sulfur or nitrogen atom in the structures given herein indicates the presence of a hydrogen atom.

Unless otherwise specified, the term "substituted" refers to the substitution of any one or more hydrogen atoms on a specific atom by substituent(s), which can include heavy hydrogen and hydrogen variants, as long as the valence state of the specific atom is normal and the substituted compound is stable. The term "optionally substituted" refers to being substituted or not, unless otherwise specified, the type and number of substituents can be arbitrary on a chemically available basis.

The term "unsubstituted" refers to that there are no substituents on the specified group.

The term "optionally substituted" refers to that the specified group is unsubstituted or substituted with one or more substituents independently selected from among the possible substituents.

When specifying the number of substituent(s), the term "one or more" refers to one to the maximum possible number of substitutions, i.e. one hydrogen to all hydrogens substituted with substituent(s). Unless otherwise specified, 1, 2, 3, 4 or 5 substituents are preferred. When a group can have multiple substituents and multiple possible substituents are given, the substituents are independently selected and do not necessarily have to be the same.

When any variable substituent appears more than once in the composition or structure of a compound, its definition is independent in each case. Therefore, for example, if a group is substituted with 0 to 2 substituents, the group can optionally be substituted with up to two substituents, and the substituents in each case have independent options. In addition, combinations of substituents and/or their variants are only allowed if such combinations result in stable compounds.

The term "optional" or "optionally" refers to that the subsequently described event or condition may, but not necessarily, occur, and that the description includes the circumstances where the event or condition occurs and the circumstances where the event or condition does not occur.

When the listed linking group does not specify its linking direction the linking direction can be arbitrary. For example, in 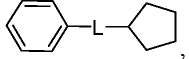 the linking group L is 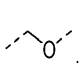 In this case, 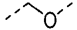 can be linked to the phenyl and cyclopentyl groups in the same direction as the reading order from left to right to form 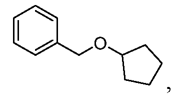 or it can be linked to the phenyl and cyclopentyl groups in the direction opposite to the reading order from left to right to form 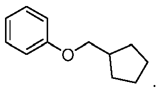 Combinations of linking groups, substituents, and/or their variants are only allowed if such combinations result in stable compounds.

Unless otherwise specified, the number of atoms on a ring is generally defined as the number of the members in the ring, e.g., "4-14 membered ring" refers to a "ring" around which 4-14 atoms are arranged.

Unless otherwise specified, Cn-n+m or Cn-Cn+m includes any specific case of n to n+m carbons, such as C1-C6 including C1, C2, C3, C4, C5, C6, and also any range of n to n+m, such as C1-C6 including C1-C3, C1-C6, C3-C6 and the like. Similarly, n to n+m membered ring includes any atom number of n to n+m on the ring, for example, a 4-14 membered ring including 4-, 6-, 8-, 10- and 14-membered rings, and also any range of n to n+m, such as 4-14 membered ring including 4-6 membered ring, 4-7 membered ring, 5-10 membered ring, 5-7 membered ring and the like.

The term "halogen" or "halo" refers to fluorine, chlorine, bromine, iodine. The term "oxy" refers to a -O-group. The term "acyloxy" refers to a -C(=O)O-group". The term "carbonyl" or "acyl" refers to a -C(=O)-group. The term "nitro" refers to a -NO₂ group. The term "cyano" refers to -CN. The term "amino" refers to -NH₂. The term "sulfonyl" refers to a -S(=O)₂- group.

The term "alkyl" refers to a linear or branched saturated hydrocarbon group, e.g., having 1 to 20 carbon atoms, preferably 1 to 12 carbon atoms. The term "optionally substituted alkyl" includes unsubstituted alkyl and substituted alkyl, wherein unsubstituted alkyl refers to alkyl that is unsubstituted with substituents. Examples of "unsubstituted alkyl" include, but are not limited to, methyl, ethyl, n-propyl, isopropyl, n-butyl, isobutyl, t-butyl and the like. Substituted alkyl refers to alkyl that is substituted with 1 to 4 substituents such as halo, alkyl, substituted alkyl, cycloalkyl, substituted cycloalkyl, alkenyl, substituted alkenyl, alkynyl, substituted alkynyl, aryl, substituted aryl, heteroaryl, substituted heteroaryl, heterocycle, substituted heterocycle, alkylsulfonyl, substituted alkylsulfonyl and the like.

The term "cycloalkyl" refers to a non-aromatic, saturated or unsaturated cyclic hydrocarbon group consisting of carbon atoms and hydrogen atoms, which may include fused ring systems, bridged ring systems or spiro ring systems, for example having 3 to 20 carbon atoms, preferably 3 to 12 carbon atoms. The term "optionally substituted cycloalkyl" includes unsubstituted cycloalkyl and substituted cycloalkyl, wherein unsubstituted cycloalkyl refers to cycloalkyl that is unsubstituted with substituents. Examples of "unsubstituted cycloalkyl" include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, cycloheptyl, cyclooctyl, bicyclo[2.2.1]heptane, bicyclo[2.2.2]octane, bicyclo[3.2.2]nonane, adamantine and the like. Substituted cycloalkyl refers to cycloalkyl that is substituted with 1 to 4 substituents such as halo, nitro, cyano, hydroxy, alkyl, aryl, substituted aryl, heteroaryl, substituted heteroaryl, alkoxy, aryloxy, alkanoyloxy, aroyloxy, amino, alkylamino, arylamino, arylalkylamino, di-substituted amino (wherein the two substituents for the amino are selected from alkyl, aryl or arylalkyl).

The term "heterocyclic group" or "heterocycle" refers to a saturated or partially unsaturated monocyclic or polycyclic group containing heteroatoms, preferably a saturated or partially unsaturated monocyclic or polycyclic group containing 1, 2, 3, 4, 5 or 6 ring heteroatoms selected from N, P, O or S. It may be a non-aromatic or aromatic ring, e.g., having 3 to 20 carbon atoms, e.g., it may be a 3-8 membered monocyclic ring, a 7-14 membered bicyclic ring (also referred to as "bicyclic heterocycle") or a 11-20 membered tricyclic ring (also referred to as "tricyclic heterocycle") and the like, and it may also be a 3-8 membered cyclic lactam, which has at least one nitrogen atom in at least one carbon atom-containing ring. Examples include, but are not limited to, pyridine ring, pyrimidinonyl, pyridonyl, imidazolyl, triazolyl, pyrrolidinyl, tetrahydrofuranyl, tetrahydropyranyl, morpholino, thiomorpholino, piperazinyl, homopiperazinyl, oxiranyl, imidazolidinyl, 3-azabicyclo[3.1.0]hexanyl, 3-azabicyclo[4.1.0]heptanyl, azabicyclo[2.2.2]hexanyl, N-pyridyl urea, pyrimidinonyl, 1,1-dioxo-thiomorpholinyl and the like.

The term "ring heteroatom" refers to a heteroatom which constitutes ring in the heterocycle.

In the term "fused group formed by heterocycle and heterocycle", the heterocycle is as previously defined, and the fused group may be formed by fusing together two or more (e.g., three or four) single heterocyclic rings. Two single heterocyclic rings are fused to form a diheterocyclic ring. It may be unsubstituted fused group or substituted fused group. Unsubstituted fused group is, for example but not limited to, thieno[3,2-c]pyridine, imidazopyridyl, pyrazolopyridyl, triazolopyridyl or pyrimidoimidazooxazine. Substituted fused group refers to a fused group that is substituted with 1 or more substituents such as alkyl, substituted alkyl, cycloalkyl, substituted cycloakyl, formyl, substituted formyl, sulfonyl, substituted sulfonyl, heterocycle containing 1 to 3 heteroatoms, substituted heterocycle containing 1 to 3 heteroatoms and the like, and the heteroatom being selected from N, O, P or S. 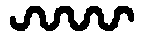 and * both represent linking sites.

The term "pharmaceutically acceptable" is directed to those compounds, materials, compositions and/or dosage forms that are suitable for use in contact with human and animal tissues within the regions of reliable medical judgment, without excessive toxicity, irritation, allergic reactions or other problems or complications, and are commensurate with a reasonable benefit/risk ratio.

The compounds of the invention may contain asymmetric centers or chiral centers, and therefore exist in different stereoisomeric forms. All stereoisomeric forms of the compounds of the invention, including but not limited to diastereomers, enantiomers, and steric hindrance isomers, as well as mixtures thereof such as racemic mixtures, will form a part of the invention. In this context, all stereoisomers are contemplated when the stereochemistry of any particular chiral atom is not determined. In addition, the invention relates to all geometric and positional isomers. The compounds of the invention can exist in different tautomeric forms, and all of these forms are included within the scope of the invention. All stereoisomers of the compounds of the invention are expected to include mixtures or pure or essentially pure forms. The resolution may be achieved by physical methods such as fractional crystallization, separation or crystallization of diastereomeric derivatives, or separation by chiral column chromatography.

The term "prodrug" refers to a functional derivative of a compound of formula (I) which is readily converted *in vivo* to the compound of formula (I). Suitable derivatives may be selected and prepared by conventional techniques well known to those skilled in the art, see, for example, Design of Prodrugs, ed. H. Bundgaard, Elsevier, 1985.

The term "pharmaceutically acceptable salt" as used herein refers to a pharmaceutically acceptable organic or inorganic salt of the compound of the invention. Exemplary salts include, but are not limited to, sulfate, citrate, acetate, oxalate, chloride, bromide, iodide, nitrate, acid sulfate, isonicotinate, lactate, salicylate, acid citrate, succinate, maleate, fumarate, gluconate, formate, methanesulfonate and pamoate. "Pharmaceutically acceptable salt" may involve inclusion of another molecule such as maleate or other counterion. Counterion acts to stabilize charge(s) in the parent compound. "Pharmaceutically acceptable salt" may have more than one charged atom, and multiple charged atoms may have multiple counterions.

If the compound of the invention is a base, the desired "pharmaceutically acceptable salt" may be prepared by a suitable method, for example, by treating the free base with an inorganic acid such as hydrochloric acid, hydrobromic acid, sulphuric acid, nitric acid, phosphoric acid, or with an organic acid such as acetic acid, maleic acid, succinic acid, mandelic acid, fumaric acid, malonic acid, pyruvic acid, salicylic acid, pyranosidyl acid such as glucuronic acid or galacturonic acid, alpha-hydroxy acid such as citric acid or tartaric acid, amino acid such as glutamic acid, aromatic acid such as benzoic acid or cinnamic acid, sulphonic acid such as methanesulphonic acid or p-toluenesulphonic acid.

If the compound of the invention is an acid, the desired "pharmaceutically acceptable salt" may be prepared by a suitable method, for example, by treating the free acid with an inorganic base or organic base such as amine, alkali metal hydroxide or alkaline earth metal hydroxide and the like. Examples of suitable salts include, but are not limited to, organic salts obtained from amino acids, primary, secondary, and tertiary amine salts, as well as salts of cyclic amines such as piperidine, morpholine, and piperazine, and inorganic salts obtained from sodium, calcium, potassium, magnesium, manganese, iron, copper, zinc, aluminum and lithium.

The compound of the invention can exist in a continuous solid state from completely amorphous to completely crystalline. The term "amorphous" refers to a state in which the material lacks long-range ordering at the molecular level and, depending on temperature, can exhibit physical properties of a solid or liquid. Typically, such material does not exhibit a distinct X-ray diffraction pattern and is more formally described as a liquid while displaying solid properties. Upon heating, such material undergoes a change from solid properties to liquid properties, characterized by a change in state, usually at the second order ("glass transition"). The term "crystal" refers to a solid phase in which the material has a regularly ordered internal structure at the molecular level and exhibits a unique X-ray diffraction pattern with determined peaks. Such material will also display liquid properties when sufficiently heated, but the change from solid to liquid is characterized by a phase change, usually at the first order ("melting point").

As used herein, the term "polymorph" refers to distinct solid crystalline phases of certain compounds of the invention in the solid state resulting from the presence of two or more different molecular arrangements. Certain compounds of the invention may exist in more than one crystal form, and the invention is intended to include various crystal forms and their mixtures.

The term "solvate" as used herein refers to a combination or a complex of one or more solvent molecules with the compound of the invention. Examples of solvents for forming the solvate include, but are not limited to, water, isopropanol, ethanol, methanol, dimethyl sulfoxide, ethyl acetate, acetic acid, and ethanolamine. The compound of the invention can exist in non solvated form or in solvated form with pharmaceutically acceptable solvents such as water, ethanol and the like. Therefore, the invention will include both solvated and non solvated forms.

The compound of the invention may contain an unnatural proportion of atomic isotopes on one or more atoms constituting the compound. The term "isotopologue" refers to a substance having the same number of atoms but different atomic masses or mass numbers than those dominant in nature. For example, the compound can be labeled with a radioactive isotope such as deuterium (²H), tritium (³H), iodine-125 (¹²⁵I), or C-14 (¹⁴C). The transformation of all isotopic compositions of the compound of the invention, whether radioactive or not, is included within the scope of the invention. Isotopic variants may enhance certain therapeutic benefits, for example, deuterium enrichment may increase the half-life *in vivo* or reduce dosage requirements, or may provide standard compounds that can be used for characterization of biological samples. Isotopically enriched compounds within formula (I) can be prepared by using appropriate isotope-enriched reagents and/or intermediates through conventional techniques known to those skilled in the art, or through methods analogous to those described in the schemes and examples herein, without excessive experiments.

Unless otherwise specified, the term "stereoisomer" may mean "diastereomer", or "enantiomer" or "cis-trans isomer".

The term "enantiomer" or "optical isomer" refers to stereoisomers that are in a mirror image relationship with each other. The term "diastereomer" refers to stereoisomers whose molecules have two or more chiral centers and are in a non-mirror image relationship with each other.

Unless otherwise specified, the term "cis-trans isomer" or "geometric isomer" refers to stereoisomers caused by the inability of double bonds or single bonds of cyclic carbon atoms to rotate freely.

Unless otherwise specified, "(D)" or "(+)" refers to dextrorotation, "(L)" or "(-)" refers to levorotation, "(DL)" or "(±) refers to racemization.

Unless otherwise specified, wedge solid line linkage 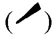 and wedge dashed line linkage 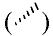 are used to represent the absolute configuration of a stereocenter, straight solid line linkage 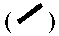 and straight dashed line linkage 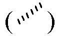 are used to represent the relative configuration of a stereocenter, wavy line 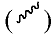 is used to represent wedge solid line linkage 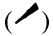 or wedge dashed line linkage 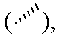 or wavy line 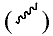 is used to represent straight solid line linkage 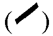 and straight dashed line linkage 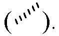

Asterisk * or 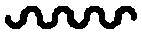 represents the linking site.

The compound of the invention may be present specific forms. Unless otherwise specified, the term "tautomer" or "tautomeric form" refers to that different functional isomers are in dynamic equilibrium and can interconvert rapidly at room temperature. If tautomers are available (e.g., in solution), then chemical equilibrium between/among the tautomers can be achieved. For example, proton tautomers, also known as prototropic tautomers, involve interconversion by proton migration, such as keto-enol isomerization and imine-enamine isomerization. Valence tautomers include interconversion by recombination of certain bonding monads. A specific example of keto-enol tautomerization is the interconversion between the two tautomers: pentane-2,4-dione and 4-hydroxy-3-en-2-one.

Further, ring A is selected from 5-6 membered heterocyclic group containing 1, 2 or 3 nitrogen atoms as ring heteroatoms.

In a preferred embodiment, ring A is selected from pyridine, pyridazine, pyrazine, pyrimidine, piperidine or piperazine.

In a more preferred embodiment, ring A is selected from pyridine, pyridazine or pyrazine.

Further, R₃ is selected from methyl or methoxy.

Further, m is 0 or 1, and in a preferred embodiment, m is 0.

Further, R₄ has a structure represented by formula (II) or (III): 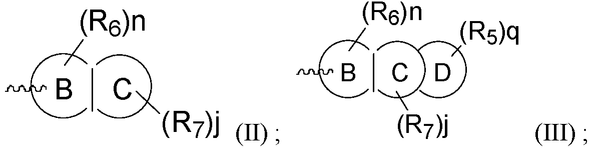
wherein, ring B, ring C or ring D is independently selected from a 5-10 membered heterocyclic group containing 1, 2 or 3 heteroatoms, and the heteroatom being selected from N, P, O or S;
R₅, R₆ or R₇ is independently selected from oxo, optionally substituted alkyl, optionally substituted cycloalkyl, optionally substituted formyl, optionally substituted sulfonyl, or optionally substituted heterocycle containing 1, 2 or 3 heteroatoms; wherein the heteroatom is selected from N, O, S;
n, j or q is independently selected from 1 or 2.
(Rs)q means that hydrogen atoms on ring D are substituted with q instances of R₅, wherein R₅ can be the same or different;
(R₆)n means that hydrogen atoms on ring B are substituted with n instances of R₅, wherein R₆ can be the same or different;
(R₇)j means that hydrogen atoms on ring C are substituted with j instances of R₅, wherein R₇ can be the same or different.

In a preferred embodiment, R₅, R₆ or R₇ is independently selected from oxo, C1-C6 alkyl, C3-C8 cycloalkyl, formyl, sulfonyl or C3-C8 heterocycle containing 1, 2 or 3 heteroatoms, wherein the heteroatom is selected from N, O, S; wherein the alkyl, cycloalkyl, formyl, sulfonyl or heterocycle is unsubstituted or substituted with 1, 2 or 3 substituents selected from halogen or C1-C3 alkyl.

Further, ring B is selected from a 5-7 membered heterocycle containing 1, 2 or 3 nitrogen atoms as the heteroatoms constituting the ring.

In certain preferred embodiments, ring B is selected from triazole, pyrazole, imidazole, 6-membered cyclic lactam or diazacyclopentane, wherein the 6-membered cyclic lactam contains two nitrogen atoms as the heteroatoms constituting the ring.

In certain more preferred embodiments, ring B is

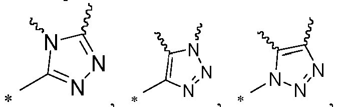

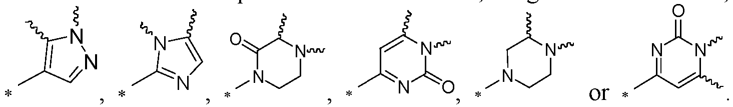

Further, ring C is selected from a 5-7 membered heterocycle containing 1-2 heteroatoms independently selected from nitrogen atom, oxygen atom or sulfur atom as the heteroatoms constituting the ring.

In certain preferred embodiments, ring C is selected from the heterocycles as described in any one of 1)-2) below:
1) pyrrole, imidazole, triazole or 6-membered cyclic lactam, wherein the 6-membered cyclic lactam contains two nitrogen atoms as the heteroatoms constituting the ring;
2) 5-7 membered heterocycloalkanes or heterocycloalkenes containing 1-2 heteroatoms independently selected from nitrogen atom, oxygen atom or sulfur atom as the heteroatoms constituting the ring.

In certain more preferred embodiments, ring C is selected from 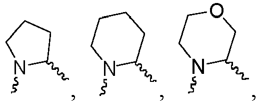 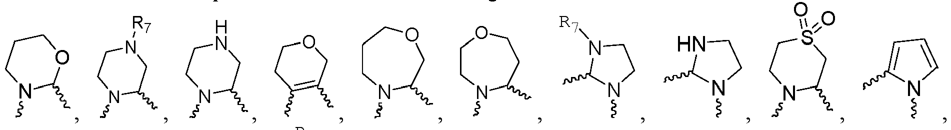 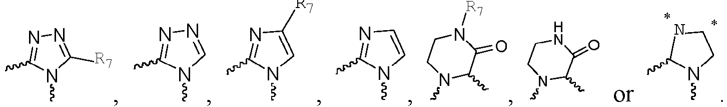 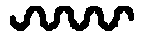 represents the linking site of ring C to ring B, * represents the linking site of ring C to ring D.

Further, ring D is selected from a 5-7 membered heterocycle, containing 1-2 heteroatoms independently selected from nitrogen atom, oxygen atom or sulfur atom as the heteroatoms constituting the ring.

In certain preferred embodiments, ring D is selected from a 5-7 membered bridged ring containing 1 nitrogen atom and 1 oxygen atom or a 5-6 membered bridged ring containing 1 nitrogen atom.

In certain more preferred embodiments, ring D is selected from

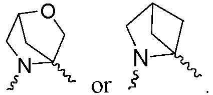

Further, R₇ is selected from C1-C6 alkyl, C3-C8 cycloalkyl, formyl substituted with C1-C3 alkyl, sulfonyl substituted with C1-C3 alkyl, C3-C6 heterocyclic group containing one O atom or one S atom; wherein the alkyl, cycloalkyl or heterocyclic group is unsubstituted or substituted with 1, 2 or 3 halogens.

In certain preferred embodiments, R₇ is selected from methyl, ethyl, -CF₃, -COCH₃,

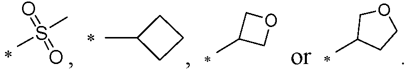

In certain preferred embodiments, when R₄ has the structure of formula (II), n is 0 or 1; when R₄ has the structure of formula (III), n is 0 or 1, and q is 0.

In a preferred embodiment, R₁ is selected from phenyl substituted with 1, 2 or 3 halogens.

In a preferred embodiment, R₂ is selected from H, linear or branched C1-C4 alkyl.

In a more preferred embodiment, R₁ is selected from 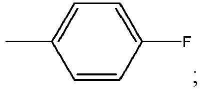 R₂ is selected from methyl.

Further, the compound has a structure represented by the following formula (VI), (V), (IV) or (IIV):

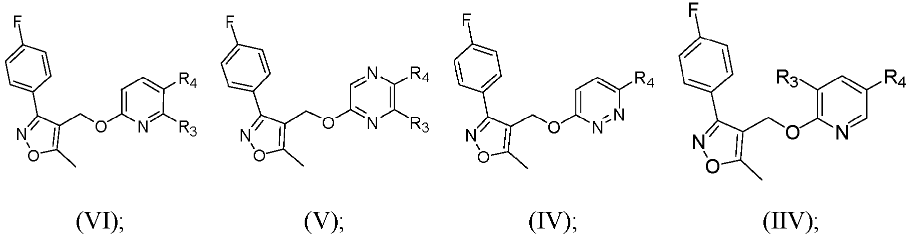

R₃ is as defined above, R₄ is as defined above.

In a more preferred embodiment, the compound is selected from any one of the following compounds:

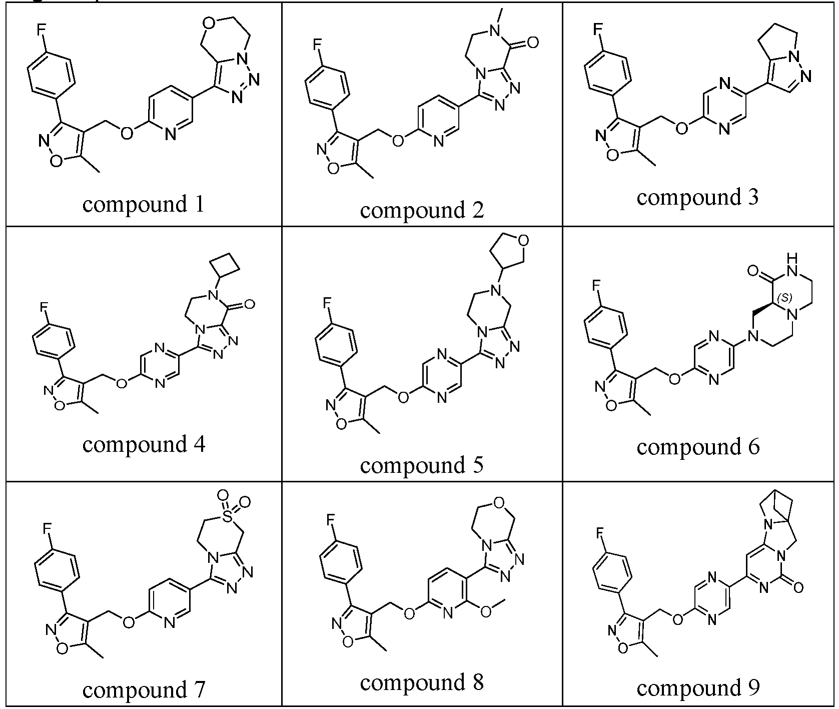

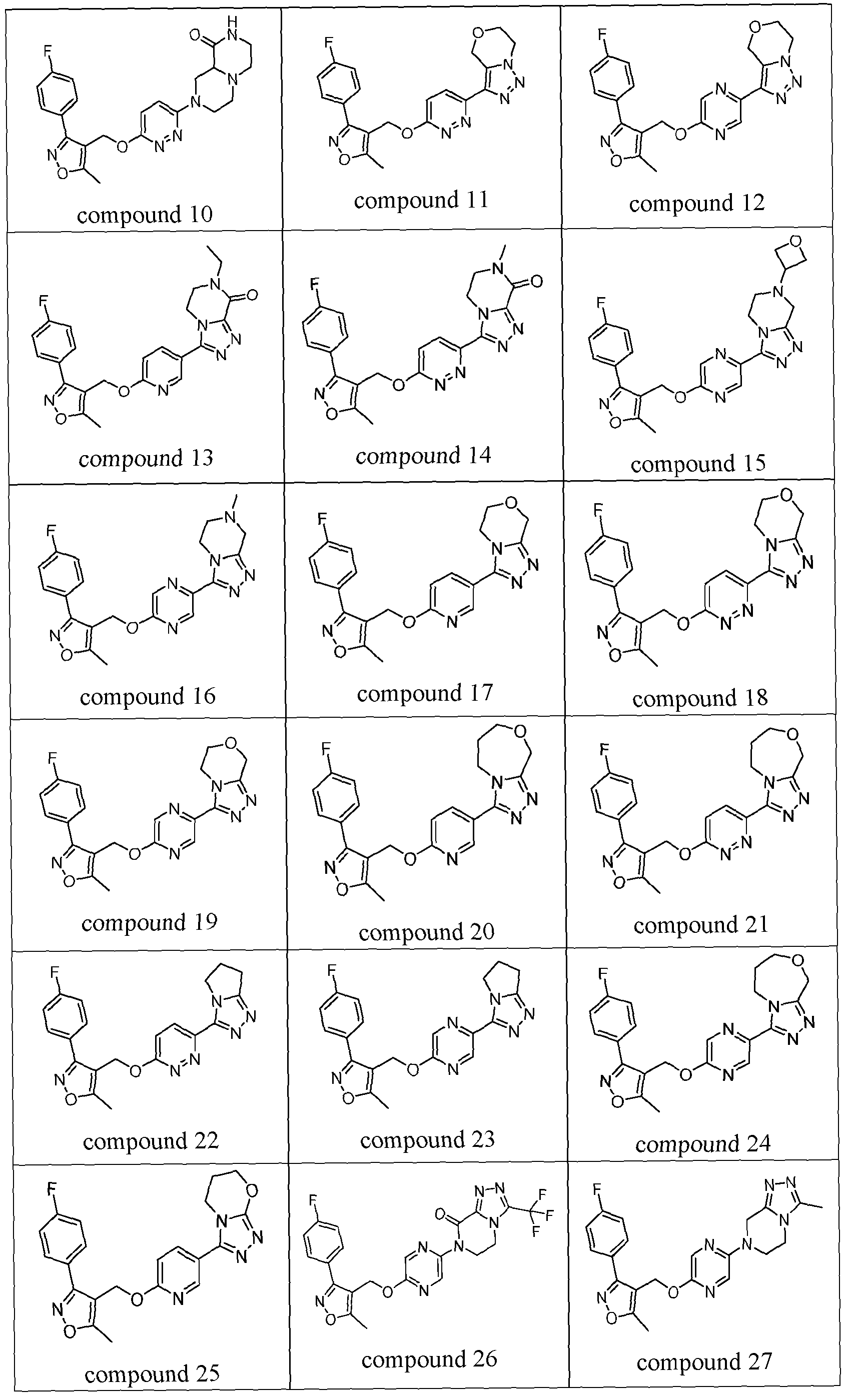

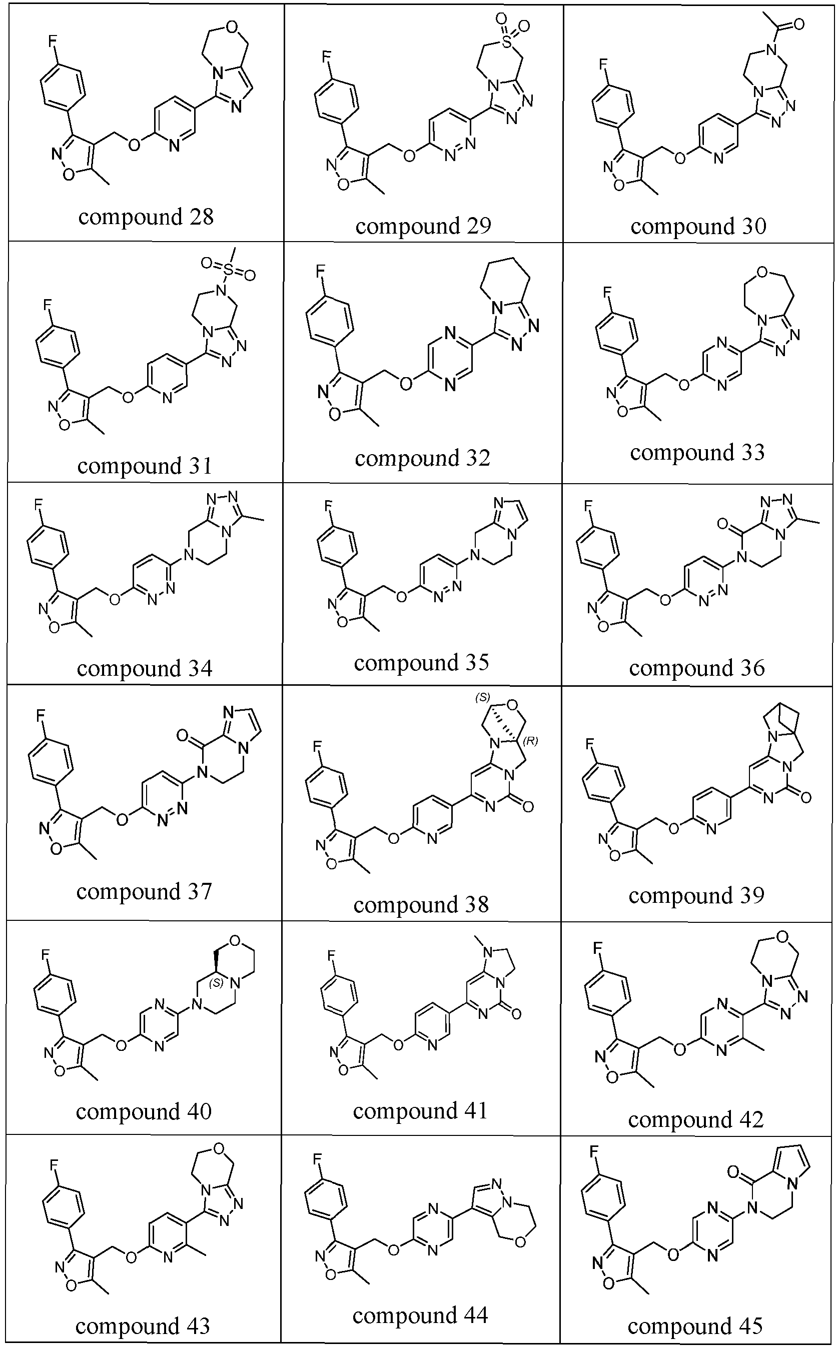

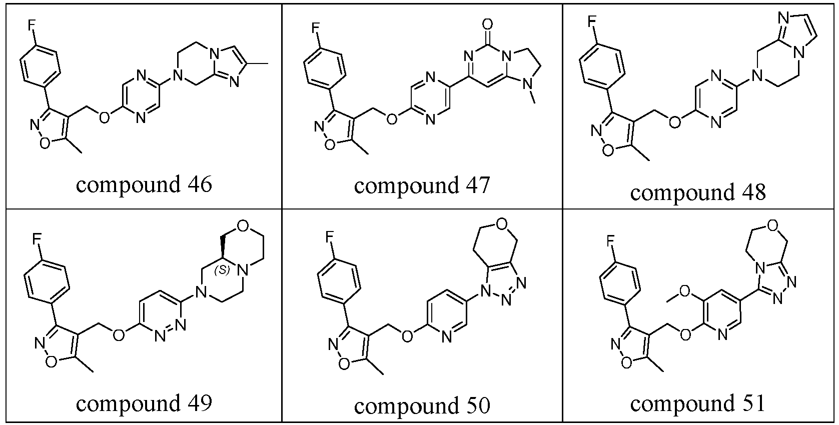

The invention also provides a pharmaceutical composition comprising at least one of a compound or a stereoisomer, tautomer, prodrug, pharmaceutically acceptable salt, amorphous form, isotopologue, polymorph or solvate thereof according to any item of the invention, and a pharmaceutically acceptable carrier and/or adjuvant.

The invention also provides use of a compound or a stereoisomer, tautomer, prodrug, pharmaceutically acceptable salt, amorphous form, polymorph or solvate thereof, the pharmaceutical mixture, or the pharmaceutical composition according to any item of the invention for the preparation of a medicament for treating or preventing a disease associated with α5-GABA_{A} receptor.

Further, the disease associated with α5-GABA_{A} receptor is at least one selected from: depression, pain, Alzheimer's disease, multi-infarct dementia, stroke, anxiety disorder, generalized anxiety disorder, panic disorder, agoraphobia, post-traumatic stress disorder, premenstrual dysphoric disorder, fibromyalgia, attention deficit hyperactivity disorder, obsessive-compulsive disorder, social anxiety disorder, autism, autistic disorder, schizophrenia, obesity, bulimia nervosa or anorexia nervosa, Tourette's syndrome, vasomotor flushing, sexual dysfunction, borderline personality disorder, chronic fatigue syndrome, Reynaud's syndrome, Parkinson's disease, epilepsy, and mood disorder following head injury.

Further, the depression is bipolar depression, postpartum depression, major depression, deprementia, atypical depression, melancholia, treatment resistant depression, depression associated with Huntington's disease, depression associated with multiple sclerosis, or depression associated with anxiety disorder.

The invention also provides use of a compound or a stereoisomer, tautomer, prodrug, pharmaceutically acceptable salt, amorphous form, polymorph or solvate thereof, the pharmaceutical mixture, or the pharmaceutical composition according to any item of the invention for the preparation of a medicament for treating or preventing the following diseases: depression, pain, Alzheimer's disease, multi-infarct dementia, stroke, anxiety disorder, generalized anxiety disorder, panic disorder, agoraphobia, post-traumatic stress disorder, premenstrual dysphoric disorder, fibromyalgia, attention deficit hyperactivity disorder, obsessive-compulsive disorder, social anxiety disorder, autism, autistic disorder, schizophrenia, obesity, bulimia nervosa or anorexia nervosa, Tourette's syndrome, vasomotor flushing, sexual dysfunction, borderline personality disorder, chronic fatigue syndrome, Reynaud's syndrome, Parkinson's disease, epilepsy, and mood disorder following head injury.

The invention also provides a method for treating or preventing a disease associated with α5-GABA_{A} receptor by administering to a patient an effective dose of a compound or a stereoisomer, tautomer, prodrug, pharmaceutically acceptable salt, amorphous form, polymorph or solvate thereof, the pharmaceutical mixture, or the pharmaceutical composition according to any item of the invention.

The invention also provides a method for treating or preventing the following diseases: depression, pain, Alzheimer's disease, multi-infarct dementia, stroke, anxiety disorder, generalized anxiety disorder, panic disorder, agoraphobia, post-traumatic stress disorder, premenstrual dysphoric disorder, fibromyalgia, attention deficit hyperactivity disorder, obsessive-compulsive disorder, social anxiety disorder, autism, autistic disorder, schizophrenia, obesity, bulimia nervosa or anorexia nervosa, Tourette's syndrome, vasomotor flushing, sexual dysfunction, borderline personality disorder, chronic fatigue syndrome, Reynaud's syndrome, Parkinson's disease, and epilepsy, or mood disorder following head injury, by administering to a patient an effective dose of a compound or a stereoisomer, tautomer, prodrug, pharmaceutically acceptable salt, amorphous form, polymorph or solvate thereof, the pharmaceutical mixture, or the pharmaceutical composition according to any item of the invention.

The technical and scientific terms as used herein that are not specifically defined have the meanings as commonly understood by those skilled in the art to which the invention pertains.

The term "treating/treatment" as used herein means administering one or more pharmaceutical substances, particularly the compound of formula (I) and/or the pharmaceutically acceptable salt thereof according to the invention, to an individual suffering from a disease or having symptoms of the disease, for the purpose of curing, alleviating, relieving, altering, treating, improving, enhancing or affecting the disease or the symptoms of the disease. The term "preventing" as used herein means administering one or more pharmaceutical substances, particularly the compound of formula (I) and/or the pharmaceutically acceptable salt thereof according to the invention, to an individual having a predisposition to the disease, for preventing the individual from developing the disease. When referring to a chemical reaction, the terms "treatment", "contact", and "reaction" mean the addition or mixing of two or more reagents under appropriate conditions to produce the specified and/or desired product. It is to be understood that the reaction that produces the specified and/or desired product may not necessarily result directly from the combination of the two reagents that are initially added, i.e., there may be one or more intermediates produced in the mixture that ultimately result in the formation of the specified and/or desired product.

The term "patient" as used herein is defined as any warm-blooded animal, for example, but not limited to mouse, guinea pig, dog, horse or human, with the patient preferably being a human.

The invention provides use of a pharmaceutical compound comprising a therapeutically effective amount of an α5-GABA_{A} receptor inverse regulator. Although the α5-GABA_{A} receptor inverse regulator for use in the treatment of the invention may be administered as the starting compound, it is preferred that the active ingredient, optionally in the form of a physiologically acceptable salt, is mixed with one or more additives, excipients, carriers, buffers, diluents and/or other conventional pharmaceutical adjuvants to form a pharmaceutical composition.

The term "effective amount" as used herein means an amount generally sufficient to produce a beneficial effect on an individual. An effective amount of the compound according to the invention can be determined by conventional methods (e.g., modeling, dose escalation studies, or clinical trials) in combination with conventional influencing factors (e.g., administration mode, pharmacokinetics of the compound, severity and course of the disease, medical history of the individual, health status of the individual, the extent of responsiveness of the individual to the drug and the like).

In a preferred embodiment, the invention provides a pharmaceutical composition comprising an α5-GABA_{A} receptor inverse regulator, wherein the α5-GABA_{A} receptor inverse regulator is in admixture with one or more pharmaceutically acceptable carriers and optionally other therapeutic and/or prophylactic components known or used in the art. The carrier must be "acceptable" in the sense of being compatible with other ingredients in the formulation and not harmful to its recipient.

The compound for use in the invention may thus be formulated together with conventional additives, or diluents, into pharmaceutical compositions and unit dose forms thereof. Such forms include solids (especially in the forms of tablets, filled capsules, powders and pills), and liquids (especially aqueous or non-aqueous solutions, suspensions, emulsions, elixirs), and capsules filled with the above forms, all forms for oral administration, suppositories for rectal administration, and sterile injectable solutions for parenteral administration. Such pharmaceutical compositions and unit dose forms thereof may comprise conventional ingredients in conventional proportions, with or without additional active compounds or ingredients, and such unit dose forms may contain any suitable effective amount of the active ingredient equivalent to the required daily dosage range.

The compound for use in the invention may be administered in a variety of oral and parenteral dosage forms. It will be apparent to those skilled in the art that the dosage forms described below may contain, as the active ingredient, a compound or a pharmaceutically acceptable salt thereof according to the invention.

To formulate the compound for use in the invention into a pharmaceutical composition, the pharmaceutically acceptable carrier can be either a solid or a liquid. Solid form formulations include powders, tablets, pills, capsules, cachets, suppositories, and dispersible granules. The solid carrier can be one or more substances that also function as diluents, flavors, solubilizers, lubricants, suspending agents, binders, preservatives, tablet disintegrating agents, or encapsulating materials.

In powders, the carrier is a finely divided solid which is in admixture with the finely divided active ingredient.

In tablets, the active ingredient is mixed with a carrier with necessary binding properties in appropriate proportions and compressed into the desired shape and size.

Powders and tablets preferably contain from 5% or 10% to about 70% of the active compound. Suitable carriers are magnesium carbonate, magnesium stearate, talc, saccharide, lactose, pectin, dextrin, starch, gelatin, tragacanth, methylcellulose, sodium carboxymethylcellulose, a low melting wax, cocoa butter and the like. The term "formulation" includes an active compound formulated with an encapsulating material as a carrier, wherein the encapsulating material provides a capsule in which the active ingredient, with or without a carrier, is surrounded by the carrier and thus bound therewith. Similarly, formulations include cachets and lozenges. Tablets, pulvis, capsules, pills, cachets, and lozenges can be used as solid forms suitable for oral administration.

For preparing suppositories, a low melting wax, such as a mixture of fatty acid glyceride or cocoa butter, is first melted and the active ingredient is then dispersed homogeneously therein by stirring. The molten homogeneous mixture is then poured into a mold of appropriate size, allowed to cool and thereby solidify.

Compositions suitable for vaginal administration may exist in the form of vaginal suppository, tampon, cream, gel, paste, foam or spray. In addition to active ingredients, the composition also contains suitable carriers known in the art.

Liquid formulations include solutions, suspensions and emulsions, for example, aqueous solutions or water-propylene glycol solutions. For example, parenteral injection liquid preparations can be formulated as water-polyethylene glycol solutions.

The compound for use in the invention may thus be formulated into formulations for parenteral administration (e.g. by injection, such as bolus injection or continuous infusion), and may be present in unit dose form with added preservatives in ampoules, pre-filled syringes, small volume infusion bags, or multi-dose containers. The compositions may take such forms as suspensions, solutions or emulsions in oily or aqueous carriers, and may contain formulation components such as suspending agents, stabilizers, and/or dispersants. Alternatively, the active ingredient can be in powder form, which can be obtained by sterile separation of sterilized solids or freeze-drying of solutions, and used for reconstitution with a suitable carrier such as sterile, pyrogen free water before use.

Aqueous solutions suitable for oral administration can be prepared by dissolving the active ingredient in water and adding the required colorants, flavors, stabilizers, and thickening agents.

Aqueous suspensions suitable for oral administration can be made by dispersing the finely divided active component in water containing viscous material, such as natural or synthetic gums, resins, methylcellulose, sodium carboxymethylcellulose, or other well-known suspending agents.

Also included are solid formualtions designed to be converted, shortly before use, to liquid formulations for oral administration. Such liquid formulations include solutions, suspensions and emulsions. Such formulations may contain, in addition to the active ingredient, colorants, flavors, stabilizers, buffers, artificial and natural sweeteners, dispersants thickeners, solubilizing agents and the like.

For topical application to the epidermis, the compound of the invention may be formulated into ointments, creams, lotions, or transdermal patches. For example, ointments and creams may be formulated with an aqueous or oily base with the addition of suitable thickening and/or gelling agents. Lotions may be formulated with an aqueous or oily base and in general also contain one or more emulsifying agents, stabilizing agents, dispersants, suspending agents, thickening agents, or colorants.

Compositions suitable for oral local administration include lozenges containing active ingredients in a flavoring base, usually sucrose and acacia or tragacanth gum; pastilles containing active ingredients in an inert base such as gelatin and glycerol or sucrose and acacia; and mouthwashes containing active ingredients in a suitable liquid carrier.

The solution or suspension can be applied directly to the nasal cavity by conventional means, such as with a dropper, pipette or nebulizer. The composition may be in single or multiple dose form.

Administration to the respiratory tract may also be effected by means of an aerosol in which the active ingredient is packaged in a pressurised pack with a suitable propellant, including chlorofluorocarbons (CFCs) such as dichlorodifluoromethane, trichlorofluoromethane or dichlorotetrafluoroethane, carbon dioxide or other suitable gas. The aerosol may also suitably contain a surfactant, such as lecithin. The dosage of the drug may be controlled by a metering valve.

Alternatively, the active ingredient may be in the form of a dry powder, for example a powder mixture of the compound with a suitable powder base such as lactose, starch, starch derivatives such as hydroxypropylmethyl cellulose and polyvinylpyrrolidone (PVP). The powder carrier can conveniently form a gel in the nasal cavity. The powder compositions may be present in unit dose form, for example in capsules or cartridges (such as capsule or cartridge of gelatin), or in blister packs where the powder may be administered by means of an inhaler.

In compositions for administration to the respiratory tract, including intranasal compositions, the compound typically has a small particle size, for example on the order of 5 microns or less. Such particle size may be obtained by methods known in the art, for example by micronization.

If desired, compositions suitable for sustained release of the active ingredient may be employed.

The pharmaceutical formulation is preferably in unit dose form. In such form, the formulation is subdivided into unit doses containing appropriate amounts of the active ingredient. The unit dose form may be an encapsulated formulation, wherein the sealed package contains a large amount of discrete formulations, such as encapsulated tablets, capsules, and powders in vials or ampoules. Further, the unit dose form may be capsules, tablets, cachets or lozenges per se, or may be an appropriate amount of the above-mentioned capsules, tablets or the like in any encapsulated form.

Tablets or capsules for oral administration and liquids for intravenous administration as well as continuous infusion are preferred compositions.

More detailed information on formulations and administration techniques can be found in the latest version of Remington's Pharmaceutical Sciences (Maack Publishing Co., Easton, PA).

The amount of the active ingredient in a unit dose formulation may vary depending on the particular application and the potency of the active ingredient, and may be adjusted from 0.01 mg to about 0.1 g. For example, in pharmaceutical application, the drug may be administered thrice per day in a capsule of 0.01 to about 100 mg, and the composition may also contain other compatible therapeutic agents as necessary.

### Method of treatment

In therapeutic use, the compound for use in the invention is administered at a starting dose of 0.001 mg/kg to 10 mg/kg body weight per day. However, the dose may vary depending on the patient's needs, the severity of the condition being treated, and the compound being used. Generally, the treatment is started with a smaller dose than the optimal dose of the compound, then this dose is increased in small amounts to achieve the best effect. For convenience, if necessary, the total daily dose can be subdivided into multiple doses within one day.

The pharmaceutical compositions of the invention may also be used simultaneously with other drugs for the treatment of pain, epilepsy, anxiety and depression, including but not limited to morphine, gabapentin and the like. Accordingly, the invention provides a drug for treating pain, epilepsy, anxiety and depression, which is not only effective but also free from significant side effects. Another object of the invention is to provide a highly safe drug for special patient groups, such as the elderly, patients with liver or kidney dysfunction, or patients with cardiovascular diseases.

The invention also provides a method of treating or preventing a disease by administering to a patient an effective dose of a compound or a composition as described above.

The invention also provides a method of treating or preventing a disease associated with α5-GABA_{A} receptor by administering to a patient an effective dose of a compound as described above or a composition as described above.

The compound of the invention may be prepared by a variety of synthetic methods well known to those skilled in the art, including the specific embodiments listed below. The embodiments formed by combining them with other chemical synthesis methods and equivalences well known to those skilled in the art, and preferred embodiments including but not limited to the examples of the invention are all included within the scope of the invention.

### Beneficial effects:

The isoxazole-heterocyclic derivative (i.e., the above-mentioned compound of formula (I) or a pharmaceutically acceptable salt, stereoisomer, tautomer, prodrug, amorphous form, isotopologue, polymorph or solvate thereof) of the invention has important pharmacological properties and is an α5-GABA_{A} receptor inverse regulator. The compound of the invention not only has excellent affinity activity and inverse regulation activity to α5-GABA_{A} receptor, but, more importantly, also has good gastric juice stability and solubility, and is more suitable for development of oral drugs and oral absorption. Accordingly, the compound or a pharmaceutically acceptable salt or prodrug thereof according to the invention can be used alone or in combination with other drugs for treating or preventing a disease associated with α5-GABA_{A} receptor.

### Detailed Description of Embodiments

The following provides a clear and complete description of the technical solution of the invention. Obviously, the examples described are only a part of the examples of the invention, not all of them. Based on the examples of the invention, all other examples obtained by those skilled in the art without creative labor are within the scope of protection of the invention. Wherein, the solvent ratio used in the purification steps (such as preparative thin layer chromatography, column chromatography and the like) in the following examples is the volume ratio.

### Intermediate 1: 3-(6-fluoropyridin-3-yl)-6,7-dihydro-4H-[1,2,3]triazolo[5,1-c][1,4]oxazine First step: 3-(2-azidoethoxy)propyl-1-yne

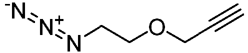

60% sodium hydride (0.22 g, 5.5 mmol) was added to anhydrous tetrahydrofuran (30 mL), and the reaction mixture was cooled to 0°C, to which was slowly added dropwise a solution of 2-azidoethanol (0.4 g, 4.6 mmol) in tetrahydrofuran (10 mL). The reaction mixture was stirred at 0°C for half an hour, to which was slowly added dropwise 3-bromopropyl-1-yne (0.82g, 5.5 mmol, 80% purity). After complete addition dropwise, the reaction mixture was stirred at room temperature for 16 hours. The reaction was quenched with water (20 mL), the reaction solution was extracted with ethyl acetate (10 mL) three times, and then the organic phases were concentrated to obtain the title compound as a light yellow oil (0.86 g crude product). LC-MS: m/z [M+H]⁺ =126.

### Second step: 3-(6-fluoropyridin-3-yl)-6,7-dihydro-4H-[1,2,3]triazolo[5,1-c][1,4]oxazine

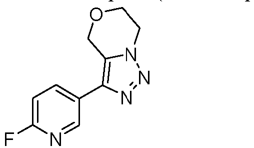

2-Fluoro-5-iodopyridine (268 mg, 1.2 mmol), cuprous iodide (23 mg, 0.12 mmol) and tetra(triphenylphosphine)palladium (69 mg, 0.06 mmol) were added to triethylamine (15mL), and the reaction mixture was stirred at room temperature for 1 hour under argon protection, to which was added 3-(2-azidoethoxy)propyl-1-yne (150 mg crude product). The reaction mixture was stirred at 60°C for 16 hours under argon protection. The reaction solution was cooled to room temperature, quenched with saturated ammonium chloride solution (50 mL), and extracted with ethyl acetate (30 mL) three times, and then the organic phases were concentrated, and separated by preparative thin layer chromatography (dichloromethane/methanol = 100/1) to obtain title compound as a light yellow solid (40 mg). ¹H NMR (400MHz, CDCl₃) δ 8.32 (s, 1 H), 8.29 -8.21 (m, 1 H), 7.07 (dd, J = 2.9, 8.3 Hz, 1 H), 5.12 (s, 2 H), 4.54 (t, J = 5.1 Hz, 2 H), 4.18 (t, J = 5.1 Hz, 2 H); LC-MS: m/z [M+H]⁺ =221.

Referring to the table below, the intermediates 2-3 were prepared according to the preparation method of intermediate 1 except that the same molar amount of the starting material in the column "Starting material" was used instead of the corresponding starting material (2-fluoro-5-iodopyridine).

| Intermediate No. | Intermediate name | Structural formula | ¹H NMR (400 MHz) | Starting material |
|---|---|---|---|---|
| 2 | 3-(6-chloropyridazin-3-yl)-6,7-dihydro-4H-[1,2,3]triazolo[5,1-c][1,4]oxazine | 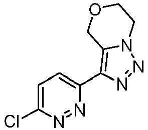 | CDCl₃: δ 8.34 (d, J = 8.8 Hz, 1H), 7.61 (d, J = 8.8 Hz, 1H), 5.33 (s, 2H), 4.54 (t, J = 4.9 Hz, 2H), 4.18 (t,J= 4.9 Hz, 2H) | 3-chloro-6-iodopyridazine |
| 3 | 3-(5-chloropyrazin-2-yl)-6,7-dihydro-4H-[1,2,3]triazolo[5,1-c][1,4]oxazine | 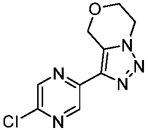 | DMSO-d6: δ 9.24 (s, 1H), 8.53 (s, 1H), 5.21 (s, 2H), 4.52 (t, J = 4.9 Hz, 2H), 4.16 (t,J= 4.9 Hz, 2H) | 2-chloro-5-iodopyrazine |

### Intermediate 4: 4-(((5-bromopyridin-2-yl)oxy)methyl)-3-(4-fluorophenyl)-5-methylisoxazole

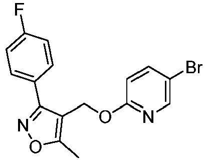

(3-(4-Fluorophenyl)-5-methylisoxazol-4-yl)methanol (0.2 g, 0.97 mmol, synthesized in accordance with the method disclosed in US20130102778A1) was dissolved in anhydrous tetrahydrofuran (3 mL), to which was added at 0°C 60% sodium hydride (0.07 g, 2.91 mmol), and after stirring for 15 minutes, slowly added dropwise a solution of 5-bromo-2-fluoropyridine (0.19 g, 1.07 mmol) in tetrahydrofuran (3 mL). After complete addition dropwise, the mixture was reacted at room temperature for 2 hours. The reaction was quenched with water (25 mL), and extracted with ethyl acetate (25 mL) twice, and then the organic phases were combined, dried over anhydrous sodium sulfate, concentrated, and separated by preparative thin layer chromatography (dichloromethane/methanol = 100/1) to obtain the title compound as an oil (150 mg, yield 43%). LC-MS: m/z [M+H]⁺ =363.

Referring to the table below, the intermediates 5-6 were prepared according to the preparation method of intermediate 4 except that the same molar amount of the starting material in the column "Starting material" was used instead of the corresponding starting material (5-bromo-2-fluoropyridine).

| Intermediate No. | Intermediate name | Structural formula | LC-MS (m/z [M+H]⁺) | Starting material |
|---|---|---|---|---|
| 5 | 4-(((5-bromopyrazin-2-yl)oxy)methyl)-3-(4-fluorophenyl)-5-methylisoxazole | 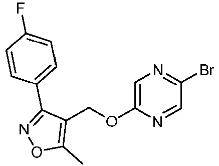 | 364 | 2,5-dibromo pyrazine |
| 6 | 4-(((6-chloropyridazin-3-yl)oxy)methyl)-3-(4-fluorophenyl)-5-methylisoxazole | 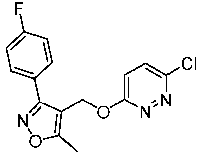 | 320 | 3,6-dichloro pyridazine |

### Intermediate 7: 3-(4-fluorophenyl)-5-methyl-4-(((5-(4,4,5,5-tetramethyl-1,3,2-dioxoboran-2-yl) pyridin-2-yl)oxy)methyl)isoxazole

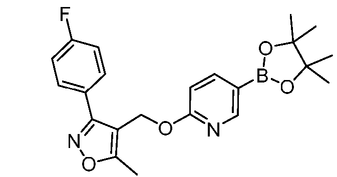

4-(((5-Bromopyridin-2-yl)oxy)methyl)-3-(4-fluorophenyl)-5-methylisoxazole (0.270 g, 0.74 mmol), bis(pinacolato)diboron (0.2 g, 0.777 mmol), [1,1'-bis(diphenylphosphine)ferrocene]palladium dichloride dichloromethane complex (0.061 g, 0.074 mmol), potassium acetate (0.15 g, 1.48 mmol) were dissolved in 1,4-dioxane (6 mL), and reacted under nitrogen protection at 100°C for 5 hours. The reaction mixture was filtered over diatomaceous earth, and then the filtrate was concentrated, and separated by preparative thin layer chromatography (dichloromethane/methanol = 50/1) to obtain the title compound as a yellow solid (140 mg, yield 52%). LC-MS: m/z [M+H]⁺ =329.

### Intermediate 8: 3-bromo-6,7-dihydro-[1,2,4]triazolo[4,3-a]pyrazin-8(5H)-one

### First step: tert-butyl 3-bromo-8-oxo-5,6-dihydro-[1,2,4]triazolo[4,3-a]pyrazin-7(8H)-carboxylate

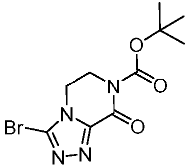

tert-butyl 3-bromo-5,6-dihydro-[1,2,4]triazolo[4,3-a]pyrazin-7(8H)-carboxylate (250 mg, 0.82 mmol, CAS: 723286-80-4) was dissolved in chloroform (3 mL) and acetonitrile (3 mL), to which were added sodium periodate (807 mg, 3.77 mmol) aqueous solution (6 ml), and then ruthenium dioxide hydrate (31 mg, 0.2 mmol). The mixture was stirred at room temperature for 1 hour. The reaction was quenched with water (6 mL), and extracted with chloroform (5 mL) three times, the organic phases were combined, dried, and filtered over diatomaceous earth, and then the filtrate was concentrated, and separated and purified by column chromatography (dichloromethane/methanol = 50/1) to obtain the title compound as a white solid (140 mg, yield 54%). LC-MS: m/z [M+H]⁺ =317.

### Second step: 3-bromo-6,7-dihydro-[1,2,4]triazolo[4,3-a]pyrazin-8(5H)-one

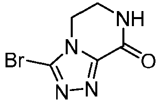

tert-butyl 3-bromo-8-oxo-5,6-dihydro-[1,2,4]triazolo[4,3-a]pyrazin-7(8H)-carboxylate (140 mg, 0.44 mmol) was dissolved in dichloromethane (3 mL), to which was added trifluoroacetic acid (150.51 mg, 1.32 mmol), and the mixture was stirred at room temperature overnight. The reaction solution was directly concentrated to obtain the title compound as a light green solid (100 mg crude product), which, without further purification, was directly used for the next step. LC-MS: m/z [M+H]⁺ =217.

### Intermediate 9: 3-bromo-7-methyl-6,7-dihydro-[1,2,4]triazolo[4,3-a]pyrazin-8(5H)-one

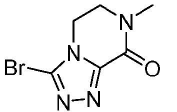

3-Bromo-6,7-dihydro-[1,2,4]triazolo[4,3-a]pyrazin-8(5H)-one (100 mg, 0.46 mmol) was dissolved in tetrahydrofuran (1 ml), to which were added, under ice bath condition, 60% sodium hydride (22 mg, 0.92 mmol), and after stirring for 15 minutes, iodomethane (131 mg, 0.92 mmol), and the reaction mixture was stirred at room temperature overnight. The reaction was quenched with water (20 mL), and extracted with ethyl acetate (15 mL) three times, and then the organic phases were combined, concentrated, and purified by column chromatography (dichloromethane/methanol = 20/1) to obtain the title compound as a white solid (42 mg, yield over two steps: 29%). LC-MS: m/z [M+H]⁺ =231.

Referring to the table below, the intermediate 10 was prepared according to the preparation method of intermediate 9 except that the same molar amount of the starting material in the column "Starting material" was used instead of the corresponding starting material (iodomethane).

| Intermediate No. | Intermediate name | Structural formula | LC-MS(m/z [M+H]⁺) | Starting material |
|---|---|---|---|---|
| 10 | 3-bromo-7-ethyl-6,7-dihydro-[1,2,4] triazolo[4,3-a]pyrazin-8(5H)-one | 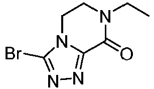 | 245 | iodoethane |

### Intermediate 11: methyl 5-chloro-3-methylpyrazin-2-carboxylate

### First step: 3-(methoxycarbonyl)-2-methylpyrazine 1-oxide

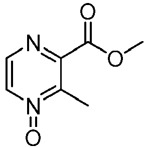

Methyl 3-methylpyrazin-2-carboxylate (2.0 g, 13.1 mmol) was dissolved in chloroform (20 mL), to which was added m-chloroperbenzoic acid (2.49 g, 14.5 mmol), and the mixture was reacted at 70°C for 5 hours. The reaction solution was diluted with water (50 mL), and extracted with dichloromethane (50 mL) twice, and then the organic phases were combined, concentrated, and purified by column chromatography (dichloromethane/methanol = 10/1) to obtain the title compound product as a white solid (1.5 g). LC-MS: m/z [M+1]⁺ =169.

### Second step: methyl 5-chloro-3-methylpyrazin-2-carboxylate

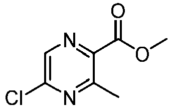

3-(Methoxycarbonyl)-2-methylpyrazine 1-oxide (1.5 g, 9.28 mmol) was dissolved in N,N-dimethylformamide (15 mL), to which was slowly added dropwise phosphorus oxychloride (2.54 mL), and the reaction mixture was stirred at 100°C for 1 hour. The reaction solution was slowly added dropwise to saturated sodium bicarbonate solution till weakly alkaline, and extracted with dichloromethane (50 mL) twice, and then the organic phases were combined, concentrated, and purified by column chromatography (petroleum ether/ethyl acetate = 5/1) to obtain the title compound product as a pale yellow liquid (1.0g). LC-MS: m/z [M+1]⁺ =187.

### Intermediate 12: methyl 6-((3-(4-fluorophenyl)-5-methylisoxazol-4-yl)methoxy)-2-methoxynicotinate

### First step: methyl 6-fluoro-2-methoxynicotinate

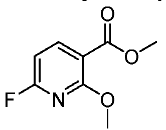

Methyl 2,6-difluoronicotinate (2.0 g, 11.6 mmol) was dissolved in tetrahydrofuran (35 mL), to which was added sodium methoxide (660 mg, 12.1 mmol), and the reaction mixture was reacted at 5°C for half an hour. The reaction solution was diluted with water (50 mL), and extracted with ethyl acetate (50 mL) twice, and then the organic phases were combined, concentrated, and purified by column chromatography (petroleum ether/ethyl acetate = 10/1) to obtain the title compound product as a white solid (1.58 g). LC-MS: m/z [M+1]⁺ =186.

### Second step: methyl 6-((3-(4-fluorophenyl)-5-methylisoxazol-4-yl)methoxy)-2-methoxynicotinate

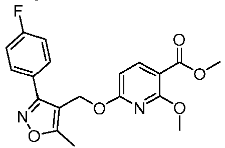

Methyl 6-fluoro-2-methoxnicotinate (1.5 g, 9.28 mmol) was dissolved in N,N-dimethylformamide (20 mL), to which were added potassium carbonate (2.24 g, 16.2 mmol) and (3-(4-fluorophenyl)-5-methylisoxazol-4-yl)methanol (1.68 g, 10.25 mmol, synthesized in accordance with the method disclosed in US20130102778A1), and the reaction mixture was reacted at 80°C for 12 hours. The reaction solution was diluted with water (100 mL), and extracted with ethyl acetate (50 mL) twice, and then the organic phases were combined, concentrated, and purified by column chromatography (dichloromethane/methanol = 50/1) to obtain the title compound product as a pale yellow liquid (1.5 g). LC-MS: m/z [M+1]⁺ =373.

### Intermediate 13: 4-(bromomethyl)-3-(4-fluorophenyl)-5-methylisoxazole

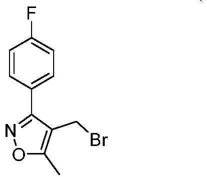

(3-(4-Fluorophenyl)-5-methylisoxazol-4-yl)methanol (2.4 g, 11.59 mmol, synthesized in accordance with the method disclosed in US20130102778A1) was added to anhydrous dichloromethane (30 mL), and cooled to 0°C, to which was slowly added phosphorus tribromide (1.5 mL), and the reaction mixture was stirred at room temperature for 1 hour. The mixture was slowly added dropwise to saturated sodium bicarbonate solution, and after further stirring the mixture for 15 minutes, the organic layer was separated, dried over anhydrous sodium sulfate, and filtered, and then the filtrate was concentrated to obtain the title compound (2 g crude product). LC-MS: m/z [M+H]⁺ =270.

### Intermediate 14: methyl 6-((3-(4-fluorophenyl)-5-methylisoxazol-4-yl)methoxy) -5-methoxynicotinate

### First step: 6-hydroxy-5-methoxynicotinic acid

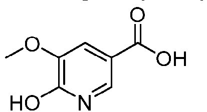

Methyl 6-chloro-5-methoxynicotinate (0.9 g, 4.46 mmol), potassium hydroxide (0.3 g, 5.35 mmol), 2-di-tert-butylphosphino-2',4',6'-triisopropylbiphenyl (0.19 g, 0.45 mmol) and tris(dibenzylideneacetone)dipalladium (0.41 g, 0.45 mmol) were added in sequence to a mixed solvent of 1,4-dioxane (10 mL) and water (10 mL), and, under argon protection, the reaction mixture was reacted at 90°C for 12 hours. The reaction solution was added to water (50 mL), and extracted with ethyl acetate (50 mL) twice, and the organic phases were discarded. The water phase was adjusted with 1M HCl to pH=5-6, and extracted with ethyl acetate (50 mL) three times, and then the combined organic phase was directly concentrated to obtain the title compound as a yellow solid (0.9 g crude product). LC-MS: m/z [M+H]⁺ =170.

### Second step: methyl 6-hydroxy-5-methoxynicotinate

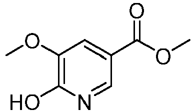

6-Chloro-5-methoxynicotinic acid (0.8 g crude product) was dissolved in methanol (20 mL), to which, at 0°C, was added sulfonyl chloride(0.84 g, 7.05 mmol), and the reaction mixture was then stirred at 68°C for 12 hours. The reaction solution was directly concentrated, and the residue was diluted with water (50 mL), and extracted with dichloromethane (50 mL) three times, and then the organic phases were combined, concentrated, and separated and purified by column chromatography (dichloromethane/methanol = 20/1) to obtain the title compound as a white solid (0.8 g). LC-MS: m/z [M+H]⁺ =184.

### Third step: methyl 6-((3-(4-fluorophenyl)-5-methylisoxazol-4-yl)methoxy)-5-methoxynicotinate

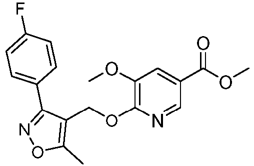

Methyl 6-hydroxy-5-methoxynicotinate (0.7 g, 2.59 mmol) was dissolved in 1,2-dichloroethane (20 mL), to which were added silver oxide (0.9 g, 3.89 mmol) and 4-(bromomethyl)-3-(4-fluorophenyl)-5-methylisoxazole (617 mg, 3.37 mmol), and the reaction mixture was stirred at 80°C for 12 hours. The reaction solution was diluted with water (50 mL), and extracted with ethyl acetate (50 mL) three times, and then the organic phases were combined, concentrated, and separated and purified by column chromatography (dichloromethane/methanol = 50/1) to obtain the title compound as a yellow solid (46 mg, yield 5%). LC-MS: m/z [M+H]⁺ =373.

### Intermediate 15: 6-chloronicotinohydrazine

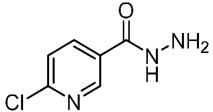

Methyl 6-chloronicotinate (5 g, 3.4 mmol) was added to ethanol (30 mL), to which was added hydrazine hydrate (222 mg, 10.2 mmol), and the reaction mixture was stirred at room temperature overnight. A white solid was precipitated, and the reaction mixture was filtered to obtain the title compound as a white solid (5 g crude product). LC-MS: m/z [M+H]⁺ =172.

Referring to the table below, the intermediates 16-21 were synthesized according to the preparation method of intermediate 15 except that the same molar amount of the starting material in the column "Starting material" was used instead of the corresponding starting material (methyl 6-chloronicotinate).

| Intermediate No. | Intermediate name | Structural formula | LC-MS: m/z [M+H]⁺ | Starting material |
|---|---|---|---|---|
| 16 | 6-chloropyridazin-3-carbohydrazide | 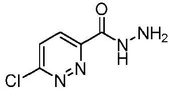 | 173 | methyl 6-chloropyridazin -3-carboxylate |
| 17 | 5-chloropyrazin-2-carbohydrazide | 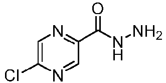 | 173 | methyl 5-chloropyrazin -2-carboxylate |
| 18 | 5-chloro-3-methylpyrazin -2-carbohydrazide | 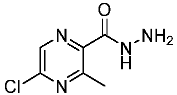 | 187 | methyl 5-chloro-3-methyl pyrazin-2-carboxylate |
| 19 | 6-chloro-2-methylnicotinohydrazine | 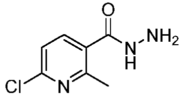 | 186 | methyl 6-chloro-2-methyl nicotinate |
| 20 | 6-((3-(4-fluorophenyl)-5-methyl isoxazol-4-yl)methoxy)-2 -methoxynicotinohydrazine | 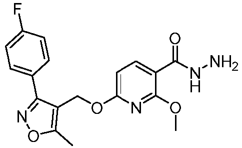 | 373 | methyl 6-((3-(4-fluoro phenyl)-5-methylisoxazol-4-yl)methoxy) -2-methoxvnicotinate |
| 21 | 6-((3-(4-fluorophenyl)-5-methyl isoxazol-4-yl)methoxy)-5 -methoxynicotinohydrazine | 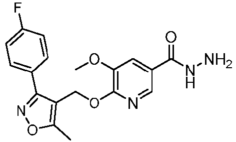 | 373 | methyl 6-((3-(4-fluoro phenyl)-5-methylisoxazol-4-yl)methoxy) -5-methoxynicotinate |

### Intermediate 22: tert-butyl 5-methoxy-3,6-dihydropyrazin-1(2H)-carboxylate

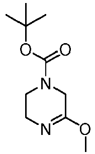

tert-butyl 3-oxopiperazin-1-carboxylate (10 g, 50 mmol) and trimethyloxonium tetrafluoroborate (10.34 mg, 70 mmol) were added to dichloromethane (100 mL), and the reaction mixture was stirred at room temperature overnight. With the addition of saturated sodium bicarbonate solution, the reaction solution was further stirred for 1 hour, and extracted with dichloromethane multiple times, and then the organic phases were combined, dried, concentrated to obtain the title compound as a light yellow gum (10 g crude product), which, without further purification, was directly used for the next reaction.

### Intermediate 23: 3-(4-fluorophenyl)-5-methyl-4-(((5-(5,6,7,8-tetrahydro-[1,2,4]triazolo [4,3-a]pyrazin-3-yl)pyrazin-2-yl)oxy)methyl)isoxazole

### First step: tert-butyl 3-(5-chloropyrazin-2-yl)-5,6-dihydro-[1,2,4]triazolo[4,3-a] pyrazin-7(8H)-carboxylate

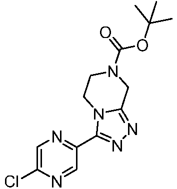

tert-butyl 5-methoxy-3,6-dihydropyrazin-1(2H)-carboxylate (1.24 g, 5.79 mmol, Intermediate 22) and 5-chloropyrazin-2-carbohydrazide (1 g, 5.79 mmol, Intermediate 17) were dissolved in ethanol (20 mL), and the mixture was heated to 120°C and stirred overnight. The reaction solution was directly concentrated, and purified by column chromatography (dichloromethane/methanol = 50/1) to obtain the title compound as a light yellow solid (1 g, yield 51%). LC-MS: m/z [M+H]⁺ =337.

### Second step: tert-butyl 3-(5-((3-(4-fluorophenyl)-5-methylisoxazol-4-yl)methoxy) pyrazin-2-yl)-5,6-dihydro-[1,2,4]triazolo[4,3-a]pyrazin-7(8H)-carboxylate

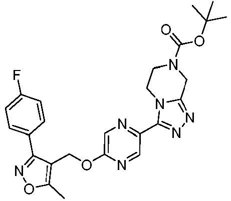

(3-(4-Fluorophenyl)-5-methylisoxazol-4-yl)methanol (500 mg, 2.4 mmol, synthesized in accordance with the method disclosed in US20130102778A1) was dissolved in tetrahydrofuran (15 mL), to which, under ice bath condition, were added 60% sodium hydride (193 mg, 4.8 mmol), and, after stirring the mixture for 30 minutes, tert-butyl 3-(5-chloropyrazin-2-yl)-5,6-dihydro-[1,2,4]triazolo[4,3-a]pyrazin-7(8H)-carboxylate (812 mg, 2.4 mmol), and the mixture was stirred at room temperature overnight. The reaction solution was quenched with saturated ammonium chloride solution, and extracted with ethyl acetate multiple times, and then the organic phases were combined, dried, concentrated, and purified by column chromatography (dichloromethane/methanol = 50/1) to obtain the title compound as a light yellow oil (600 mg, yield 49%). LC-MS: m/z [M+H]⁺ =508.

### Third step: 3-(4-fluorophenyl)-5-methyl-4-(((5-(5,6,7,8-tetrahydro-[1,2,4]triazolo[4,3-a] pyrazin-3-yl)pyrazin-2-yl)oxy)methyl)isoxazole

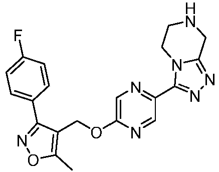

tert-butyl 3-(5-((3-(4-fluorophenyl)-5-methylisoxazol-4-yl)methoxy)pyrazin-2-yl)-5,6-dihydro-[1,2,4]triazolo[4,3-a]pyrazin-7(8H)-carboxylate (500 mg, 0.99mol) was dissolved in methanol (5 mL) and water (5 mL), and the mixture was subjected to microwave reaction at 150°C for 1.5 hours in a microwave reactor. The mixture was directly concentrated to obtain the title compound as a light yellow oil (400 mg, yield 99%). LC-MS: m/z [M+H]⁺ =408.

Referring to the table below, the intermediate 24 was synthesized according to the preparation method of intermediate 23 except that the same molar amount of the starting material in the column "Starting material" was used instead of the corresponding starting material (5-chloropyrazin-2-carbohydrazide).

| Intermediate No. | Intermediate name | Structural formula | LC-MS (m/z [M+H]⁺) | Starting material |
|---|---|---|---|---|
| 24 | 3-(4-fluorophenyl)-5-methyl -4-(((6-(5,6,7,8-tetrahydro-[1,2,4]triazolo [4,3-a]pyrazin-3-yl)pyridazine -3 -yl)oxy)methyl)isoxazole | 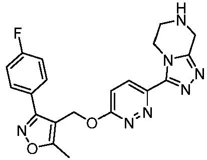 | 408 | 6-chloropyridin -3-carbohydrazide |

### Intermediate 25: 4-((5-(7-cyclobutyl-5,6,7,8-tetrahydro-[1,2,4]triazolo[4,3-a]pyrazin-3-yl) pyrazin-2-yl)oxy)methyl)-3-(4-fluorophenyl)-5-methylisoxazole

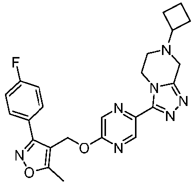

3-(4-Fluorophenyl)-5-methyl-4-(((5-(5,6,7,8-tetrahydro-[1,2,4]triazolo[4,3-a]pyrazin-3-yl )pyrazin-2-yl)oxy)methyl)isoxazole (200 mg, 0.49 mmol, Intermediate 23) was dissolved in methanol (12 mL), to which were added cyclobutanone (84 mg, 0.98 mmol), and, after stirring the mixture at room temperature for 30 minutes, sodium cyanoborohydride (185 mg, 2.94 mmol), and then the mixture was heated to 60°C and stirred overnight. The mixture was purified by column chromatography (dichloromethane/methanol = 50/1) to obtain the title compound as a white solid (100 mg, yield 44%). LC-MS: m/z [M+H]⁺ =462.

Referring to the table below, the intermediate 26 was synthesized according to the preparation method of intermediate 26 except that the same molar amount of the starting material in the column "Starting material" was used instead of the corresponding starting material (cvclobutanone).

| Intermediate No. | Intermediate name | Structural formula | LC-MS(m/z [M+H]⁺) | Starting material |
|---|---|---|---|---|
| 26 | 3-(4-fluorophenyl)-5-methyl-4-(((6-(7-methyl-5,6,7,8-tetrahydro-[1,2,4]triazolo[4,3-a]pyrazin -3-yl)pyridazin-3-yl)oxy)methyl)isoxazole | 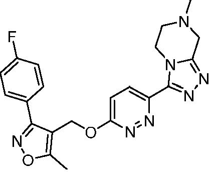 | 422 | formaldehyde |

### Intermediate 27: 1,4-oxazepan-3-one

### First step: 2-(3-((tert-butoxycarbonyl)amino)propoxy)acetic acid

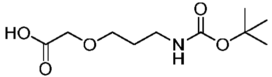

tert-butyl (3-hydroxypropyl)carbamate (4 g, 23 mmol) was added to anhydrous tetrahydrofuran (40 mL), and cooled to 0°C, to which was slowly added 60% sodium hydride (2.47 g, 68 mmol), and after stirring for 15 minutes, further added methyl bromoacetate (5.24 g, 34 mmol), and then the reaction mixture was stirred at room temperature overnight. The reaction was quenched with water, and extracted with ethyl acetate multiple times, and then the organic phases were combined, dried over anhydrous sodium sulfate, and concentrated to obtain the title compound (3 g crude product). LC-MS: m/z [M+1]⁺ =234.

### Second step: methyl 2-(3-aminopropoxy)acetate

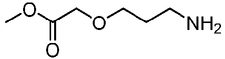

2-(3-((tert-butoxycarbonyl)amino)propoxy)acetic acid (3 g crude product) was dissolved in methanol (30 mL), and cooled to 0°C, to which was slowly added dropwise sulfonyl chloride (4.6 g, 39 mmol), and the reaction mixture was stirred at room temperature overnight. The reaction solution was concentrated to obtain the title compound (1 g crude product). LC-MS: m/z [M+1]⁺ =148.

### Third step: 1,4-oxazean-3-one

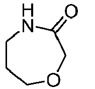

Methyl 2-(3-aminopropoxy)acetate (1 g crude product) was dissolved in methanol (20 mL), to which was added potassium carbonate (1.9 g, 13.6 mmol), and the reaction mixture was heated to 60°C and stirred for 1 hour. The reaction mixture was filtered over diatomaceous earth, and then the filtrate was concentrated, and purified by column chromatography (petroleum ether/ethyl acetate = 2/1) to obtain the title compound (0.16 g, yield over three steps: 5%). LC-MS: m/z [M+H]⁺ =116.

### Intermediate 28: 5-chloro-3,6-dihydro-2H-1,4-oxazine

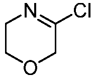

3-Morpholone (4g) was dissolved in a mixed solvent of dichloromethane (5 mL), to which was added phosphorus oxychloride, and the reaction mixture was stirred at 40°C for 1 hour. The reaction solution was concentrated to obtain the title compound (4.5 g crude product), which, without further purification, was directly used for the next reaction.

Referring to the table below, the intermediates 29-33 were synthesized according to the preparation method of intermediate 28 except that the same molar amount of the starting material in the column "Starting material" was used instead of the corresponding starting material (3-morpholone).

| Intermediate No. | Intermediate name | Structural formula | LC-MS: m/z [M+H]+ | Starting material |
|---|---|---|---|---|
| 29 | 5-chloro-3,6-dihydro-2H-1,4-thiazine | 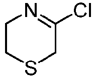 | crude product was directly used into the next step | thiomorpholin-3 -one |
| 30 | 3-chloro-2,5,6,7-tetrahydro-1,4-oxazepane | 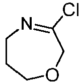 | crude product was directly used into the next step | 1,4-oxazepan-3 -one |
| 31 | 5-chloro-3,4-dihydro-2H-pyrrole | 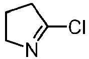 | crude product was directly used into the next step | 2-pyrrolidone |
| 32 | 6-chloro-2,3,4,5 -tetrahydropyridine | 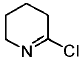 | crude product was directly used into the next step | piperidin-2-one |
| 33 | 5-chloro-2,3,6,7-tetrahydro-1,4-oxazepane | 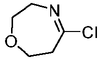 | crude product was directly used into the next step | 1,4-oxazepan-5-one |

### Intermediate 34: 3-(6-chloropyridin-3-yl)-5,6-dihydro-8H-[1,2,4]triazolo[3,4-c][1,4]oxazine

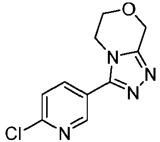

5-Chloro-3,6-dihydro-2H-1,4-oxazine (5 g, 29 mmol, Intermediate 28) was dissolved in toluene (250 mL), to which were added 6-chloronicotinohydrazine (4.5 g, 37 mmol, Intermediate 15) and N,N-diisopropylethyl amine (3.7 g, 28.9 mmol) separately, and then the reaction mixture was stirred at 130°C for 4 hours, and cooled to room temperature. The reaction mixture was adjusted with saturated sodium bicarbonate solution to pH=10-11, stirred at 100°C for 16 hours, and extracted with ethyl acetate multiple times, and then the organic phases were combined, concentrated to obtain the crude title compound (0.36 g, yield over three steps: 38%). LC-MS: m/z [M+H]⁺ =237.

Referring to the table below, the intermediates 35-47 were synthesized according to the preparation method of intermediate 34 except that the same molar amount of the starting material 1 and the same molar amount of the starting material 2 in the column "Starting material" were used instead of the corresponding starting materials (6-chloronicotinohydrazine and 5-chloro-3,6-dihydro-2H-1,4-oxazine) respectively.

| Intermediate No. | Intermediate name | Structural formula | LC-MS: m/z [M+H]⁺ | Starting material 1 | Starting material 2 |
|---|---|---|---|---|---|
| 35 | 3-(6-chloropyridazin-3-yl)-5,6-dihydro-8H-[1,2,4]triazolo [3,4-c][1,4]oxazine | 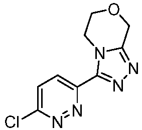 | 238 | 6-chloropyridazin-3-carbohydrazide | 5-chloro-3 ,6-dihydro-2H-1,4-oxazine |
| 36 | 3-(6-chloropyridin-3-yl)-5,6-dihydro-8H-[1,2,4]triazolo [3,4-c][1,4]thiazine | 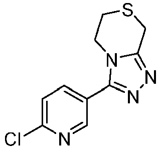 | 253 | 6-chloronicotino hydrazine | 5-chloro-3 ,6-dihydro-2H-1,4-thiazine |
| 37 | 3-(5-chloropyrazin-2-yl)-5,6-dihydro-8H-[1,2,4]triazolo [3,4-c][1,4]oxazine | 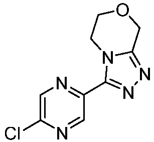 | 238 | 5-chloropyrazin-2-carbohydrazide | 5-chloro-3 ,6-dihydro-2H-1,4-oxazine |
| 38 | 3-(6-chloropyridin-3-yl)-6,7-dihydro-5H,9H-[1,2,4]triazolo [3,4-c][1,4]oxazepane | 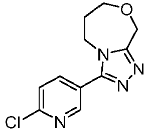 | 251 | 6-chloronicotino hydrazine | 3-chloro-2,5,6,7-tetrahydro-1,4-oxazepane |
| 39 | 3-(6-chloropyridazin-3-yl)-6,7-dihydro-5H,9H-[1,2,4]triazolo [3,4-c][1,4]oxazepane | 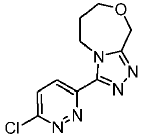 | 252 | 6-chloropyridazin-3-carbohydrazide | 3-chloro-2,5,6,7-tetrahydro-1,4-oxazepane |
| 40 | 3-(6-chloropyridazin-3-yl)-6,7-dihydro-5H-pyrrolo [2,1-c][1,2,4]triazole | 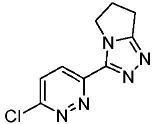 | 222 | 6-chloropyridazin-3-carbohydrazide | 5-chloro-3 ,4-dihydro-2H-pyrrole |
| 41 | 3-(5-chloropyrazin-2-yl)-6,7-dihydro-5H-pyrrolo [2,1-c][1,2,4]triazole | 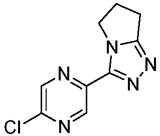 | 222 | 5-chloropyrazin-2-carbohydrazide | 5-chloro-3 ,4-dihydro-2H-pyrrole |
| 42 | 3-(5-chloropyrazin-2-yl)-6,7-dihydro-5H,9H-[1,2,4]triazolo [3,4-c][1,4]oxazepane | 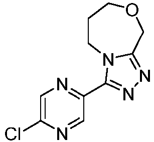 | 252 | 5-chloropyrazin-2-carbohydrazide | 3-chloro-2,5,6,7-tetrahydro-1,4-oxazepane |
| 43 | 3-(6-chloropyridazin-3-yl)-5,6-dihydro-8H-[1,2,4]triazolo [3,4-c][1,4]thiazine | 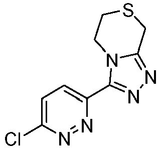 | 254 | 6-chloropyridazin-3-carbohydrazide | 5-chloro-3 ,6-dihydro-2H-1,4-thiazine |
| 44 | 3-(5-chloropyrazin-2-yl)-5,6,7,8-tetrahydro-[1,2,4]triazolo[4,3-a]pyridine | 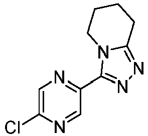 | 236 | 5-chloropyrazin-2-carbohydrazide; | 6-chloro-2,3,4,5-tetrahydropyridine |
| 45 | 3-(5-chloropyrazin-2-yl)-5,6,8,9-tetrahydro-[1,2,4]triazolo [4,3-d][1,4]oxazepane | 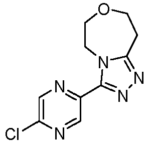 | 252 | 5-chloropyrazin-2-carbohydrazide | 5-chloro-2,3,6,7-tetrahydro-1,4-oxazepane |
| 46 | 3-(5-chloro-3-methylpyrazin-2-yl)-5,6-dihydro-8H-[1,2,4]triazolo [3,4-c][1,4]oxazine | 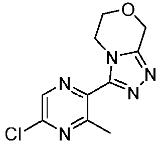 | 252 | 5-chloro-3 -methy 1 pyrazin-2 -carbohydrazide | 5-chloro-3 ,6-dihydro-2H-1,4-oxazine |
| 47 | 3-(6-chloro-2-methylpyridin-3-yl)-5,6-dihydro-8H-[1,2,4]triazolo [3,4-c][1,4]oxazine | 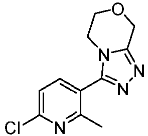 | 251 | 6-chloro-2-methyl nicotinohydrazine | 5-chloro-3 ,6-dihydro-2H-1,4-oxazine |

### Intermediate 48: 4-(((5-(5,6-dihydro-8H-[1,2,4]triazolo[3,4-c][1,4]thiazin-3-yl)pyridin-2-yl) oxy)methyl)-3-(4-fluorophenyl)-5-methylisoxazole

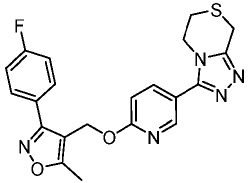

(3-(4-Fluorophenyl)-5-methylisoxazol-4-yl)methanol (141 mg, 0.68 mmol, synthesized in accordance with the method disclosed in US20130102778A1) was added to anhydrous tetrahydrofuran (10 mL), and cooled to 0°C, to which were added 60% sodium hydride (82 mg, 2.04 mmol), and, after stirring at 0°C for 30 minutes, 3-(6-chloropyridin-3-yl)-5,6-dihydro-8H-[1,2,4]triazolo[3,4-c][1,4]thiazine (172 mg, 0.68 mmol, Intermediate 36), and the reaction mixture was stirred at room temperature overnight. The reaction solution was concentrated, and separated by preparative thin layer chromatography (petroleum ether/ethyl acetate=1/1) to obtain the title compound as an orange solid (160 mg, yield 56%). LC-MS: m/z [M+H]⁺ =424.

Referring to the table below, the intermediate 49 was synthesized according to the preparation method of intermediate 48 except that the same molar amount of the starting material in the column "Starting material" was used instead of the corresponding starting material (3-(6-chloropyridin-3-yl)-5,6-dihydro-8H-[1,2,4]triazolo[3,4-c][1,4]thiazine).

| Intermediate No. | Intermediate name | Structural formula | LC-MS: m/z [M+H]⁺ | Starting material |
|---|---|---|---|---|
| 49 | 4-(((6-(5,6-dihydro-8H-[1,2,4]triazolo[3,4-c][1,4]thiazin-3-yl) pyridazin-3-yl)oxy)methyl)-3-(4-fluorophenyl)-5-methylisoxazole | 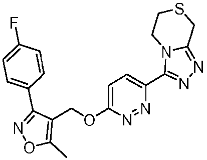 | 425 | 3-(6-chloropyridazin-3-yl)-5,6-dihydro-8H-[1,2,4]triazolo [3,4-c][1,4]thiazine |

### Intermediate 50: 3-(6-chloropyridin-3-yl)-6,7-dihydro-5H-[1,2,4]triazolo[3,4-b][1,3]oxazine

### First step: 5-(6-chloropyridin-3-yl)-1,3,4-oxadiazole-2-thiol

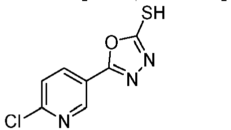

6-Chloronicotinohydrazine (0.7 g, 4.06 mmol, Intermediate 15) was dissolved in ethanol (16 mL) and water (8 mL), to which were further added carbon disulfide (3.09 g, 40.6 mmol) and potassium hydroxide (0.34 g, 6.09 mmol), and the mixture was heated to 90°C and stirred overnight. The reaction solution was concentrated to obtain the title compound (0.84 g crude product). LC-MS: m/z [M+1]⁺ =214.

### Second step: 2-(6-chloropyridin-3-yl)-5-(methylthio)-1,3,4-oxadiazole

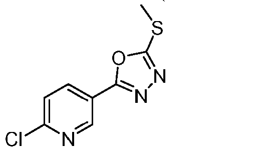

5-(6-Chloropyridin-3-yl)-1,3,4-oxadiazole-2-thiol (0.84 g, 3.9 mmol) was dissolved in methanol (20 mL), to which was further added iodomethane (1.12 g, 7.84 mmol), and the reaction mixture was stirred at room temperature overnight. The reaction solution was concentrated, and purified by column chromatography (dichloromethane/methanol = 100/1) to obtain the title compound (0.7 g, yield 78%). LC-MS: m/z [M+H]⁺ =228.

### Third step: 2-(6-chloropyridin-3-yl)-5-(methylsulfonyl)-1,3,4-oxadiazole

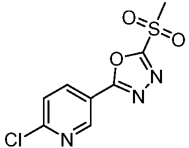

2-(6-Chloropyridin-3-yl)-5-(methylthio)-1,3,4-oxadiazole (0.7 g, 3.08 mmol) was dissolved in dichloromethane (10 mL), to which was further added m-chloroperbenzoic acid (1.59 g, 9.24 mmol), and the reaction mixture was stirred at room temperature overnight. The reaction solution was concentrated, and purified by column chromatography (dichloromethane/methanol = 50/1) to obtain the title compound (280 mg, yield 35%). LC-MS: m/z [M+H]⁺ =260.

### Fourth step: 6-chloro-N'-(5,6-dihdro-4H-1,3-oxazin-2-yl)nicotinic hydrazide

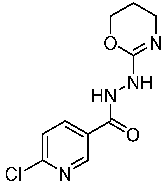

2-(6-Chloropyridin-3-yl)-5-(methylsulfonyl)-1,3,4-oxadiazole (280 mg, 1.05 mmol) was dissolved in tetrahydrofuran (10 mL), to which was further added 3-aminopropanol (94.64 mg,1.26 mmol), and then slowly added dropwise triethylamine (319 mg, 3.15 mmol), and the reaction mixture was stirred at room temperature overnight. The reaction solution was concentrated, and purified by column chromatography (dichloromethane/methanol = 10/1) to obtain the title compound (120 mg, yield 37.5%). LC-MS: m/z [M+H]⁺ =255.

### Fifth step: 3-(6-chloropyridin-3-yl)-6,7-dihydro-5H-[1,2,4]triazolo[3,4-b][1,3]oxazine

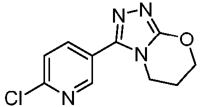

6-Chloro-N'-(5,6-dihydro-4H-1,3-oxazin-2-yl)nicotinic hydrazide (120 mg, 0.47 mmol) was added to dichlorobenzene (8mL), and subjected to microwave reaction at 180°C for 4 hours. The reaction solution was separated by preparative thin layer chromatography (dichloromethane/methanol = 20/1) to obtain the title compound (50 mg, yield 45%). LC-MS: m/z [M+H]⁺ =237.

### Intermediate 51: 3-(4-fluorophenyl)-5-methyl-4-((5-(3-(trifluoromethyl)-5,6-dihydro-[1,2,4]triazolo[4,3-a]pyrazin-7(8H)-yl)pyrazin-2-yl)oxy)methyl)isoxazole

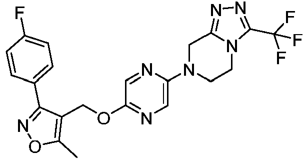

4-((5-Bromopyrazin-2-yl)oxy)methyl)-3-(4-fluorophenyl)-5-methylisoxazole (300 mg, 0.82 mmol) was added to 1,4-dioxane (10 mL), to which were further added 3-(trifluoromethyl)-5,6,7,8-tetrahydro-[1,2,4]triazolo[4,3-a]pyrazine hydrochloride (158 mg, 0.82 mmol), tris(dibenzylideneacetone)dipalladium (75 mg, 0.082 mmol), 4,5-bis(diphenylphosphino)-9,9-dimethylxanthene (51 mg, 0.088 mmol) and cesium carbonate (430 mg, 1.32 mmol), and the reaction mixture was stirred under nitrogen protection at 100°C overnight. The reaction solution was concentrated, and purified by column chromatography (dichloromethane/methanol = 20/1) to obtain the title compound (80 mg, yield 20.5%). LC-MS: m/z [M+1]⁺ =476.

### Intermediate 52: 3-(6-fluoropyridin-3-yl)-5,6-dihydro-8H-imidazo[5,1-c][1,4]oxazine

### First step: (6-fluoropyridin-3-yl)(3-(hydroxymethyl)morpholino)methanone

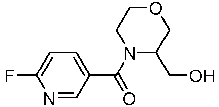

6-Fluoronicotinic acid (1.5 g, 10.6 mmol), morpholin-3-ylmethanol (1.25 g, 10.6 mmol) and N,N-diisopropylethylamine (2.75 g, 21.3 mmol) were added to dichloromethane (20 mL), to which was added 2-(7-azabenzotriazolyl)-N,N,N',N'-tetramethylurea hexafluorophosphate (4.85 g, 12.8 mmol), at 0°C and the reaction mixture was stirred at room temperature overnight. The reaction mixture was purified by column chromatography (dichloromethane/methanol = 50/1) to obtain the title compound as a yellow liquid (2.1 g, yield 82%). LC-MS: m/z [M+1]⁺ =241.

### Second step: 4-(6-fluoronicotinoyl)morpholine-3-formaldehyde

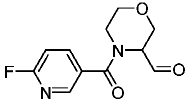

(4-(6-Fluoropyridin-3-carbonyl)morpholin-3-yl)methanol (0.5 g, 2.08 mmol) and Dess-Martin reagent (0.88 g, 2.08 mmol) were dissolved in dichloromethane (20 mL), and the reaction mixture was stirred at room temperature for 2 hours. The reaction mixture was filtered over diatomaceous earth, and then the filtrate was concentrated, and separated by preparative thin layer chromatography (dichloromethane/methanol = 50/1) to obtain the title compound as an oily liquid (90 mg, yield 18%). LC-MS: m/z [M+1]⁺ =239.

### Third step: 3-(6-fluoropyridin-3-yl)-5,6-dihydro-8H-imidazo[5,1-c][1,4]oxazine

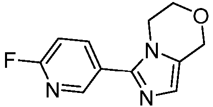

4-(6-Fluoropyridin-3-carbonyl)morpholine-3-formaldehyde (0.09 g, 0.38 mmol) and ammonium acetate (0.059 g, 0.76 mmol) were dissolved in acetic acid (4 mL), and reacted at 80°C for 2 hours. The reaction was quenched with saturated sodium bicarbonate solution, and extracted with ethyl acetate multiple times, and then the organic phases were combined, dried over anhydrous sodium sulfate, concentrated, and separated by preparative thin layer chromatography (dichloromethane/methanol = 20/1) to obtain the colorless oily title compound (10 mg, yield 12%). LC-MS: m/z [M+1]⁺=220.

### Intermediate 53: 3-bromo-5,6,7,8-tetrahydro-[1,2,4]triazolo[4,3-a]pyrazine

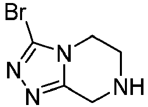

tert-butyl 3-bromo-5,6-dihydro-[1,2,4]triazolo[4,3-a]pyrazin-7(8H)-carboxylate (0.2 g, 0.66 mmol) was dissolved in dichloromethane (4 mL), to which was added trifluoroacetic acid (2 mL), and the reaction mixture was stirred at room temperature overnight. An excess of sodium bicarbonate solid was added to the reaction mixture. The reaction mixture was filtered over diatomaceous earth, and then the filtrate was concentrated to obtain the the yellow oily title compound (0.1 g, yield 77%). LC-MS: m/z [M+1]⁺ =203.

### Intermediate 54: 1-(3-bromo-5,6-dihydro-[1,2,4]triazolo[4,3-a]pyrazin-7(8H)-yl)ethan-1-one

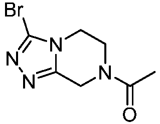

3-Bromo-5,6,7,8-tetrahydro-[1,2,4]triazolo[4,3-a]pyrazine (0.13 g, 0.64 mmol), acetyl chloride (0.098 g, 0.96 mmol) and sodium bicarbonate (0.11 g, 1.28 mmol) were dissolved in dichloromethane (6 mL), and reacted at room temperature for 3 hours. The reaction mixture was filtered over diatomaceous earth, and then the filtrate was concentrated to obtain the title compound (0.12 g crude product). LC-MS: m/z [M+1]⁺ =245.

Referring to the table below, the intermediate 55 was synthesized according to the preparation method of intermediate 54 except that the same molar amount of the starting material in the column "Starting material" was used instead of the corresponding starting material (acetyl chloride).

| Intermediate No. | Intermediate name | Structural formula | LC-MS: m/z [M+H]⁺ | Starting material |
|---|---|---|---|---|
| 55 | 3-bromo-7-(methylsulfonyl)-5,6,7,8-tetrahydro-[1,2,4]triazolo[4,3-a]pyrazine | 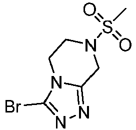 | 281 | methylsulfonyl chloride |

### Intermediate 56: (3S,11aR)-7-chloro-3,4-dihydro-1H,9H,11H-3,11a-methanopyrimido [6', 1' :2,3]imidazo[5,1-C] [1,4]oxazin-9-one

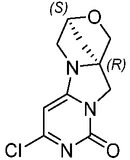

Intermediate 56 was synthesized in accordance with the method of the patent WO2021089032A1.

### Intermediate 57: 3-chloro-7,8-dihydro-1H,6H,9H-7,8a-methanopyrrolo[1',2':3,4]imidazo [1,2-c]pyrimidin-1-one

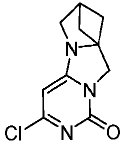

Intermediate 57 was synthesized in accordance with the method of the patent WO2021089032A1.

### Intermediate 58: 7-chloro-1-methyl-2,3-dihydroimidazo[1,2-c]pyrimidin-5(1H)-one

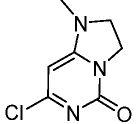

7-Chloro-2,3-dihydroimidazo[1,2-c]pyrimidin-5(1H)-one (0.02 g, 0.12 mmol), iodomethane (0.026 g, 0.18 mmol) and cesium carbonate (0.078 g, 0.24 mmol) were dissolved in N,N-dimethylformamide (2 mL), and stirred at room temperature for 16 hours. The reaction solution was added water (20 mL), and then extracted with ethyl acetate (20 mL) twice, and then the organic phases were combined, dried, and concentrated to obtain the title compound (160 mg). LC-MS: m/z [M+1]⁺ =186.

### Intermediate 59: 3-(4,4,5,5-tetramethyl-1,3,2-dioxaboran-2-yl)-6,7-dihydro-4H-pyrazolo[5,1-c][1,4]oxazine

### First step: 4-bromo-1-(2-((tert-butyldimethylsilyl)oxy)ethyl)-1H-pyrazole

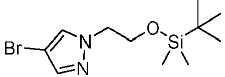

4-Bromo-1H-pyrazole (2.0 g, 13.6 mmol) was dissolved in N,N-dimethylformamide (30 mL), to which were added potassium carbonate (5.64 g, 40.8 mmol) and tert-butyl (2-chloroethoxy)dimethylsilane (4.88 g, 20.4 mmol), and the reaction solution was reacted at 25°C for 12 hours. The reaction solution was diluted with water (50 mL), and extracted with ethyl acetate (50 mL) twice, and then the organic phases were combined, dried, concentrated, and separated and purified by column chromatography (petroleum ether/ethyl acetate = 5/1) to obtain the title compound as a white solid (3.2g). LC-MS: m/z [M+1]⁺ =305.

### Second step: 4-bromo-1-(2-((tert-butyldimethylsilyl)oxy)ethyl)-1H-pyrazole-5-formaldehyde

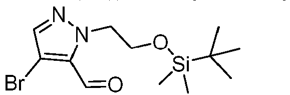

Under nitrogen protection, 4-bromo-1-(2-((tert-butyldimethylsilyl)oxy)ethyl)-1H-pyrazole (3.2 g, 11.7 mmol) was dissolved in tetrahydrofuran (50 mL), to which were added dropwise at -78°C lithium diisopropylamide (11.76 mL, 23.52 mmol, 2M), and after stirring for half an hour, N,N-dimethylformamide (1.72 g, 23.5 mmol). After complete addition dropwise, the reaction mixture was stirred for 1 hour. Dilute hydrochloric acid was added dropwise to the reaction solution till weakly acidic. Then, the reaction solution was diluted with water (50 mL), and extracted with ethyl acetate (50 mL) twice, and then the organic phases were combined, dried, concentrated, and separated and purified by column chromatography (petroleum ether/ethyl acetate = 2/1) to obtain the title compound as a white solid (2.5 g). LC-MS: m/z [M+1]⁺ =333.

### Third step: 3-bromo-6,7-dihydro-4H-pyrazolo[5,1-c][1,4]oxazin-4-ol

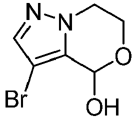

4-Bromo-1-(2-((tert-butyldimethylsilyl)oxy)ethyl)-1H-pyrazole-5-formaldehyde (2.5 g, 7.53 mmol) was added to a mixed solvent of water (5 mL) and 2-methyltetrahydrofuran (5 mL), to which was added trifluoroacetic acid (10 mL), and, after stirring at room temperature for half an hour, saturated sodium bicarbonate solution was added to the reaction solution till weakly alkaline. The reaction solution was diluted with water (50 mL), and extracted with ethyl acetate (50 mL) twice, and then the organic phases were combined, dried, concentrated, and separated and purified by column chromatography (dichloromethane/methanol = 20/1) to obtain the title compound as a pale yellow solid (1.35 g). LC-MS: m/z [M+H]⁺=219.

### Fourth step: 3-bromo-6,7-dihydro-4H-pyrazolo[5,1-c][1,4]oxazine

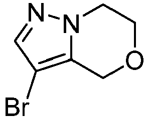

3-Bromo-6,7-dihydro-4H-pyrazolo[5,1-c][1,4]oxazin-4-ol (500 mg, 2.28 mmol) was added to dichloromethane (15 mL), to which were then added trifluoroacetic acid (2.04 mL, 27.3 mmol) and triethylsilane (1.59 g, 13.7 mmol), and the reaction solution was stirred at 25°C for 16 hours. Saturated sodium bicarbonate solution was added to the reaction solution till weakly alkaline. The reaction solution was diluted with water (50 mL), and extracted with ethyl acetate (50 mL) twice, and then the organic phases were combined, dried, concentrated, and separated and purified by column chromatography (dichloromethane/methanol = 20/1) to obtain the title compound as a pale yellow liquid (440 mg). LC-MS: m/z [M+H]⁺ =203.

### Fifth step: 3-(4,4,5,5-tetramethyl-1,3,2-dioxaboran-2-yl)-6,7-dihydro-4H-pyrazolo [5,1-c][1,4]oxazine

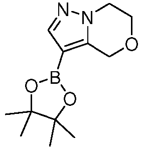

3-Bromo-6,7-dihydro-4H-pyrazolo[5,1-c][1,4]oxazine (200 mg, 0.99 mmol) was dissolved in 1,4-dioxane (10 mL), to which were added potassium carbonate (290 mg, 2.97 mmol), tetrabutylammonium bromide (64 mg, 0.2 mmol), bis(pinacolato)diboron (50 mg, 1.98 mmol) and bis(triphenylphosphine)palladium(II) chloride (140 mg, 0.2 mmol) separately, and, under argon saturation condition, the reaction mixture was stirred at 105°C for 2 hours. The reaction mixture was diluted with water (50 mL), and extracted with ethyl acetate (50 mL) twice, and then the organic phases were combined, dried, concentrated, and purified by column chromatography (dichloromethane/ethyl acetate=20/1) to obtain the title compound (200 mg). LC-MS: m/z [M+H]⁺ =251.

### Intermediate 60: 2-methyl-5,6,7,8-tetrahydroimidazo[1,2-a]pyrazine

### First step: 2-methylimidazo[1,2-a]pyrazine

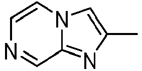

Pyrazin-2-amine (2.0 g, 21.0 mmol) was dissolved in ethanol (25 mL), to which was added 1-bromopropan-2-one (3.46 g, 25.2 mmol), and the mixture was reacted at 80°C for 2 hours. The reaction solution was directly concentrated, and separated and purified by column chromatography (petroleum ether/ethyl acetate = 10/1) to obtain the title compound as a white solid (1.3g). LC-MS: m/z [M+1]⁺ =134.

### Second step: 2-methyl-5,6,7,8-tetrahydroimidazo[1,2-a]pyrazine

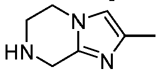

2-Methylimidazo[1,2-a]pyrazine (0.5 g, 3.76 mmol) was dissolved in methanol (10 mL), to which was added Pd/C catalyst (50 mg), and the reaction mixture was stirred in hydrogen environment at 25°C for 2 hours. The reaction liquid was filtered over diatomaceous earth, and then the filtrate was concentrated to obtain the title compound as a pale yellow liquid (0.8 g). LC-MS: m/z [M+1]⁺ =138.

### Intermediate 61: 3-(4-fluorophenyl)-5-methyl-4-(((5-(tributylstannyl)pyrazin-2-yl)oxy) methyl)isoxazole

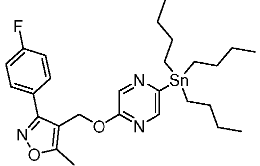

4-(((5-Bromopyrazin-2-yl)oxy)methyl)-3-(4-fluorophenyl)-5-methylisoxazole (1 g, 2.75 mmol, Intermediate 5), tricyclohexylphosphine (77 mg, 0.28 mmol), tris(dibenzylideneacetone)dipalladium (151 mg, 16 mmol) and lithium chloride (525 mg, 12.4 mmol) were added to 1,4-dioxane (40 mL), to which was added hexabutylditin (2.71 g, 4.67 mmol), and then the reaction mixture was heated in an argon atmosphere to 110°C and stirred overnight. An aqueous potassium fluoride saturated solution was added to the reaction solution, followed by stirring for 2 hours. A dark precipitate appeared, the reaction mixture was filtered over diatomaceous earth, and then the filtrate was dried, concentrated, and purified by column chromatography (dichloromethane/methanol=20/1) to obtain the light yellow oily title compound (500 mg, yield 32%). LC-MS: m/z [M+H]⁺ =576.

### Intermediate 62: methyl 1-(2-aminoethyl)-4-(6-((3-(4-fluorophenyl)-5-methylisoxazol-4-yl) methoxy)pyridazin-3-yl)piperazine-2-carboxylate

### First step: 1-tert-butyl 2-methyl 4-[6-((3-(4-fluorophenyl)-5-methylisoxazol-4-yl)methoxy) pyridazin-3-yl]piperazine-1,2-dicarboxylate

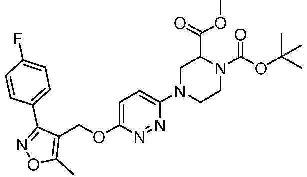

Under nitrogen protection, the compound 4-(((6-chloropyridazin-3-yl)oxy)methyl)-3-(4-fluorophenyl)-5-methylisoxazole (300 mg, 0.94 mmol, Intermediate 6), 1-(tert-butyl) 2-methyl (S)-piperazine-1,2-dicarboxylate (0.34 g, 1.41 mmol, CAS: 129799-15-1), cesium carbonate (0.55 g, 1.69 mmol), (2-dicyclohexylphosphino-2',6'-diisopropoxy-1,1'-biphenyl)(2-amino-1,1'-biphenyl-2-yl)palla dium(II) mesylate (0.079 g, 0.094 mmol) and 2-dicyclohexylphosphino-2',6'-diisopropoxy-1,1'-biphenyl (0.044 g, 0.094 mmol) were added to 1,4-dioxane (20 mL), and the reaction mixture was stirred at 100°C for 16 hours. The reaction liquid was filtered over diatomaceous earth, and then the filtrate was concentrated, and purified by column chromatography (dichloromethane/methanol=20/1) to obtain the title compound as a white solid (450 mg, yield 91%). LC-MS: m/z [M+H]⁺ =528.

### Second step: methyl 4-[6-((3-(4-fluorophenyl)-5-methylisoxazol-4-yl)methoxy)pyridazin-3-yl] piperazine-2-carboxylate

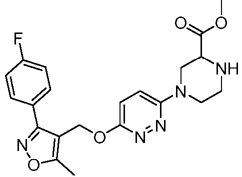

At room temperature, 1-tert-butyl 2-methyl 4-[6-((3-(4-fluorophenyl)-5-methylisoxazol-4-yl)methoxy)pyridazin-3-yl]piperazine-1,2-dicar boxylate (450 mg, 0.85 mmol) was dissolved in dichloromethane (10 mL), to which was added trifluoroacetic acid (3 mL), and the reaction mixture was stirred at room temperature for 3 hours. The reaction solution was poured into saturated sodium bicarbonate solution (50 mL) for quenching, and extracted with ethyl acetate (100 mL) twice. The organic phases were combined, dried over anhydrous sodium sulfate, and concentrated to obtain the title compound (360 mg crude product). LC-MS: m/z [M+H]⁺ =428.

### Third step: methyl 1-(2-((tert-butoxycarbonyl)amino)ethyl)-4-(6-((3-(4-fluorophenyl)-5-methyl isoxazol-4-yl)methoxy)pyridazin-3-yl)piperazine-2-carboxylate

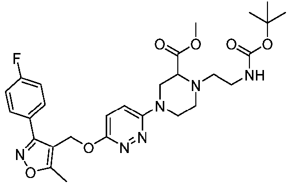

Under nitrogen protection, methyl 4-[6-((3-(4-fluorophenyl)-5-methylisoxazol-4-yl)methoxy)pyridazin-3-yl]piperazine-2-carbox ylate (310 mg, 0.73 mmol), tert-butyl 1,2,3-oxathiazolidine-3-carboxylate 2,2-dioxide (0.20 g, 0.88 mmol, CAS: 459817-82-4) and cesium carbonate (0.36 g, 1.09 mmol) were added to acetonitrile (15 mL), and the reaction mixture was stirred at 70°C for 3 hours. The reaction liquid was filtered over diatomaceous earth, and the filtrate was diluted with water (50 mL), and extracted with ethyl acetate (50 mL) three times. Then, the organic phases were combined, washed with saturated brine (60 mL) once, dried over anhydrous sodium sulfate, and filtered, and then the filtrate was concentrated to obtain the title compound (400 mg crude product). LC-MS: m/z [M+H]⁺ =571.

### Fourth step: methyl 1-(2-aminoethyl)-4-(6-((3-(4-fluorophenyl)-5-methylisoxazol-4-yl)methoxy)pyridazin-3-yl)pi perazine-2-carboxylate

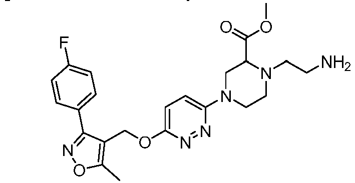

Under nitrogen protection, methyl 1-(2-((tert-butoxycarbonyl)amino)ethyl)-4-(6-((3-(4-fluorophenyl)-5-methylisoxazol-4-yl)met hoxy)pyridazin-3-yl)piperazine-2-carboxylate (350 mg, 0.61 mmol) was dissolved in hydrogen chloride ethyl acetate solution (5 mL), and the reaction mixture was stirred at 25°C for 2 hours. The reaction solution was poured into saturated sodium bicarbonate (150 mL) for quenching, and extracted with ethyl acetate (50 mL) three times. Then, the organic phases were combined, washed with saturated brine (80 mL), dried over anhydrous sodium sulfate, and filtered, and then the filtrate was concentrated to obtain the title compound (200 mg crude product). LC-MS: m/z [M+H]⁺ =471.

### Intermediate 63: (S)-hexahydro-2H-pyrazino[1,2-a]pyrazin-1(6H)-one hydrochloride

### First step: (S)-1-tert-butyl 3-methyl 4-(2-(((benzyloxy)carbonyl)amino)ethyl)piperazine-1,3-dicarboxylate

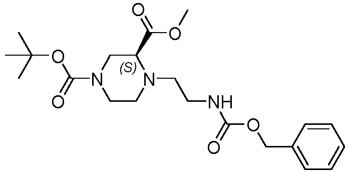

At room temperature, (S)-1-tert-butyl benzyl 3-methylpiperazine-1,3-dicarboxylate (1.0 g, 4.09 mmol) and (2-bromoethyl)carbamate (1.27 g, 4.91 mmol) were dissolved in anhydrous N,N-dimethylformamide (15.0 mL), to which was added potassium carbonate (678 mg, 4.91 mmol), and the reaction mixture was reacted at 100°C for 16 hours. The reaction solution was diluted with water (40 mL), and extracted with dichloromethane (30 mL×3 times), and then the organic phases were combined, washed with saturated brine (40 mL×2 times), dried over anhydrous sodium sulfate and filtered. The filtrate was concentrated under reduced pressure, and then the residue was purified by flush silica gel column chromatography (methanol: dichloromethane= 0:1 ~ 1:10) to obtain the title compound as a yellow oil (370 mg). LC-MS: m/z [M+H]⁺ =422.

### Second step: tert-butyl (S)-9-oxooctahydro-2H-pyrazine[1,2-a]pyrazin-2-carboxylate

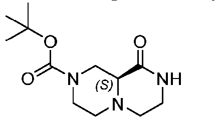

At room temperature, (S)-1-tert-butyl 3-methyl 4-(2-(((benzyloxy)carbonyl)amino)ethyl)piperazine-1,3-dicarboxylate (370 mg, 0.87 mmol) was dissolved in methanol (6.0 mL), to which was added Pd/C (30 mg), and the reaction solution was reacted in hydrogen (15 psi) atmosphere for 16 hours. The reaction liquid was filtered over diatomaceous earth, and then the filtrate was concentrated under reduced pressure to obtain the yellow oily title compound (210 mg). LC-MS: m/z [M+H]⁺ =256.

### Third step: (S)-hexahydro-2H-pyrazino[1,2-a]pyrazin-1(6H)-one hydrochloride

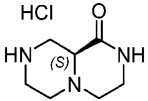

At room temperature, tert-butyl (S)-9-oxooctahydro-2H-pyrazine[1,2-a]pyrazin-2-carboxylate (60 mg, 0.24 mmol) was dissolved in anhydrous dichloromethane (4 mL), to which was slowly added dropwise 4M hydrogen chloride ethyl acetate solution (4 mL), and the reaction solution was reacted with stirring for 1 hour. The reaction solution was concentrated under reduced pressure to obtain the titled product as a yellow solid (50 mg). LC-MS: m/z [M+H]⁺ =156.

### Intermediate 64: 1-(6-fluoropyridin-3-yl)-1,4,6,7-tetrahydropyrano[3,4-d][1,2,3]triazole

### First step: 5-azido-2-fluoropyridine

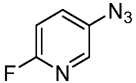

6-fluoro-3-pyridylamine (3 g, 26.7 mmol) was dissolved in acetonitrile (60 mL), and cooled to 0°C, to which, at 0°C, were added tert-butyl nitrite (4.14 g, 40.1 mmol) and azidotrimethylsilane (3.7 g, 32.1 mmol) in sequence, and the reaction mixture was stirred at room temperature overnight. The reaction solution was added to water (200 mL), and extracted with dichloromethane (200 mL) three times. Then, the organic phases were combined, washed with saturated brine once, dried and concentrated to obtain the title compound (3 g, crude product), directly used for the next reaction. LC-MS: m/z [M+H]⁺ =139.

### Second step: 1-(6-fluoropyridin-3-yl)-1,4,6,7-tetrahydropyrano[3,4-d][1,2,3]triazole

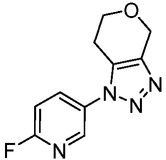

Tetrahydro-4H-pyran-4-one (1.23 g, 12.25 mmol) was added to toluene (50 mL), the mixture was heated to 110°C, to which was slowly added dropwise a solution of 5-azido-2-fluoropyridine (2.2 g, 15.93 mmol) in toluene (10 mL), and then added pyrrolidine (0.96 g, 13.48 mmol), and the reaction solution was stirred at 110°C for 3 hours. After cooling to 0°C, dichloromethane (100 mL), m-chloroperbenzoic acid (4.97 g, 24.5 mmol) and sodium bicarbonate (2.06 g, 24.5 mmol) are added in sequence, and the reaction mixture was stirred at 0°C overnight. The reaction solution was diluted with water (100 mL), and extracted with dichloromethane (100 mL) twice. Then, the organic phases were combined, washed with saturated brine once, dried, concentrated, and purified by column chromatography (dichloromethane/methanol=20/1) to obtain the title compound as a pale yellow solid (310 mg). LC-MS: m/z [M+H]⁺ =221.

### Example 1:

### 3-(6-((3-(4-fluorophenyl)-5-methylisoxazol-4-yl)methoxy)pyridin-3-yl)-6,7-dihydro-4H-[1,2,3]triazolo[5,1-c][1,4]oxazine

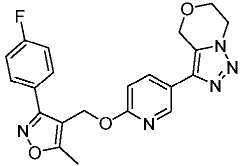

### Method A

(3-(4-Fluorophenyl)-5-methylisoxazol-4-yl)methanol (33 mg, 0.16 mmol, synthesized in accordance with the method disclosed in US20130102778A1) was dissolved in anhydrous tetrahydrofuran (2 mL), to which, under ice bath condition, were added 60% sodium hydride (7 mg, 0.19 mmol), and, after stirring the reaction mixture at 0°C for half an hour, 3-(6-fluoropyridin-3-yl)-6,7-dihydro-4H-[1,2,3]triazolo[5,1-c][1,4]oxazine (35 mg, 0.16 mmol, Intermediate 1), and then the reaction mixture was stirred at room temperature for 16 hours. The reaction was quenched with water (20 mL), and extracted with ethyl acetate (20 mL) three times, and then the organic phases were concentrated, and separated by preparative thin layer chromatography (dichloromethane/methanol = 50/1) to obtain the title compound as a white solid (33 mg, yield 51%), named as Compound 1. ¹H NMR (400MHz, CDCl₃) δ 8.30 (br. s., 1H), 8.03 (d, J = 7.3 Hz, 1H), 7.80 (br. s., 2H), 7.15 (br. s., 2H), 6.88 (d, J = 7.8 Hz, 1H), 5.27 (br. s., 2H), 5.10 (br. s., 2H), 4.52 (br. s., 2H), 4.17 (br. s., 2H), 2.59 (br. s., 3 H); LC-MS: m/z [M+H]⁺ =408.

### Example 2

### 3-(6-((3-(4-fluorophenyl)-5-methylisoxazol-4-yl)methoxy)pyridin-3-yl)-7-methyl-6,7-dih ydro-[1,2,4]triazolo[4,3-a]pyrazin-8(5H)-one

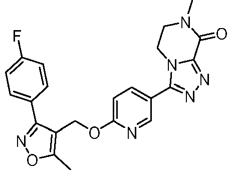

### Method B

3-(4-Fluorophenyl)-5-methyl-4-(((5-(4,4, 5, 5-tetramethyl-1,3,2-dioxoboran-2-yl)pyridin-2 -yl)oxy)methyl)isoxazole (75 mg, 0.18 mmol, Intermediate 7), 3-bromo-7-methyl-6,7-dihydro-[1,2,4]triazolo[4,3-a]pyrazin-8(5H)-one (42 mg, 0.18 mmol, Intermediate 9), [1,1'-bis(diphenyl phosphine)ferrocene]palladium dichloride (13 mg, 0.018 mmol) and sodium carbonate (38 mg, 0.36 mmol) were dissolved in 1,4-dioxane (3 mL). The mixture was heated in argon atmosphere to 110°C and stirred overnight. The reaction liquid was filtered over diatomaceous earth, and then the filtrate was concentrated, and separated and purified by column chromatography (dichloromethane/methanol = 50/1) to obtain the title compound as a white solid (23 mg, yield 29%), named as Compound 2. ¹H NMR (400MHz, CDCl₃) δ 8.49 (br. s., 1H), 8.04 (d, J = 8.3 Hz, 1H), 7.86 -7.69 (m, 2H), 7.16 (t, J = 8.3 Hz, 2H), 6.95 (d, J = 8.3 Hz, 1H), 5.32 (s, 2H), 4.38 (br. s., 2H), 3.81 (br. s., 2H), 3.23 (s, 3 H), 2.60 (s, 3 H); LC-MS: m/z [M+H]⁺ =435.

### Example 3

### 4-((5-(5,6-dihydro-4H-pyrrolo[1,2-b]pyrazol-3-yl)pyrazin-2-yl)oxy)methyl)-3-(4-fluorop henyl)-5-methylisoxazole

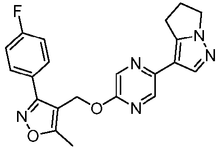

### Method C

4-(((5-Bromopyrazin-2-yl)oxy)methyl)-3-(4-fluorophenyl)-5-methylisoxazole (200 mg, 0.55 mmol, Intermediate 5), 3-3-(4,4,5,5-tetramethyl-1,3,2-dioxaborolan-2-yl)-5,6-dihydro-4H-pyrrolo(1,2-b)pyrazole (129 mg, 0.55 mmol, CAS: 1314138-13-0) were dissolved in 1,4-dioxane (6 mL), to which were added 1,1'-bis(diphenylphosphino)ferrocenyl palladium dichloride (40 mg, 0.055 mmol) and sodium carbonate (117 mg, 1.1 mmol). The mixture was heated in argon atmosphere to 100°C and stirred overnight, and then directly purified by column chromatography (dichloromethane/methanol = 50/1) to obtain the title compound as a light yellow solid (15 mg, yield 7%), named as Compound 3. ¹H NMR (400MHz, CDCl₃) δ 8.21 (s, 2H), 7.97 (br. s., 1H), 7.78 (t, J = 6.6 Hz, 2H), 7.16 (t, J = 8.3 Hz, 2H), 5.24 (s, 2H), 4.22 (d, J = 6.8 Hz, 2H), 3.16 (br. s., 2H), 2.80 -2.65 (m, 2H), 2.59 (s, 3 H); LC-MS: m/z [M+H]⁺ =392.

### Example 4

### 7-cyclobutyl-3-(5-((3-(4-fluorophenyl)-5-methylisoxazol-4-yl)methoxy)pyrazin-2-yl)-6,7 -dihydro -[1,2,4]triazolo[4,3-a]pyrazin-8(5H)-one

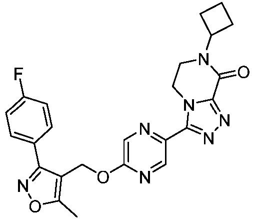

### Method D

4-((5-(7-Cyclobutyl-5,6,7,8-tetrahydro-[1,2,4]triazolo[4,3-a]pyrazin-3-yl)pyrazin-2-yl)ox y)methyl)-3-(4-fluorophenyl)-5-methylisoxazole (100 mg, 0.22 mmol, Intermediate 25) was dissolved in a mixed solvent of chloroform (1 mL), acetonitrile (1 mL) and water (2 mL), to which were added sodium periodate (141 mg, 0.66 mmol) and ruthenium dioxide hydrate(7 mg, 0.044 mmol), and the mixture was stirred at room temperature for 10 minutes. The reaction solution was added with water (4 mL), and then extracted with dichloromethane (3 mL) three times, the organic phases were combined, and filtered over diatomaceous earth, and then the filtrate was dried, concentrated, and purified by column chromatography (dichloromethane/methanol = 20/1) to obtain the title compound as a white solid (19 mg, yield 18%), named as Compound 4. ¹H NMR (400MHz, CDCl₃) δ 9.22 (br. s., 1H), 8.25 (br. s., 1H), 7.78 (br. s., 2H), 7.18 (br. s., 2H), 5.38 (br. s., 2H), 5.22 (br. s., 1H), 4.82 (br. s., 2H), 3.82 (br. s., 2H), 2.62 (br. s., 3 H), 2.30 (br. s., 2H), 2.18 (br. s., 2H), 1.80 (br. s., 2H); LC-MS: m/z [M+H]⁺ =476.

### Example 5

### 3-(4-fluorophenyl)-5-methyl-4-((5-(7-(tetrahydrofuran-3-yl)-5,6,7,8-tetrahydrofuran-[1,2, 4]triazolo [4,3-a]pyrazin-3-yl)pyrazin-2-yl)oxy)methyl)isoxazole

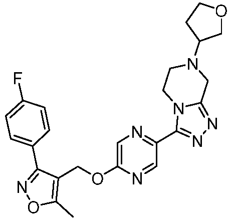

### Method E

3-(4-Fluorophenyl)-5-methyl-4-(((5-(5,6,7,8-tetrahydro-[1,2,4]triazolo[4,3-a]pyrazin-3-yl )pyrazin-2-yl)oxy)methyl)isoxazole (100 mg, 0.245 mmol, Intermediate 23) was dissolved in methanol (10 mL), to which were added dihydro-3(2H)-furanone (63 mg, 0.73 mmol), and, after stirring the mixture at room temperature for 30 minutes, sodium cyanoborohydride (46 mg, 0.73 mmol), and then the mixture was heated to 60°C and stirred overnight. Then, the mixture was purified by column chromatography (dichloromethane/methanol = 20/1) to obtain the title compound as a white solid (41 mg, yield 35%), named as Compound 5. ¹H NMR (400MHz, CDCl₃) δ 9.09 (s, 1H), 8.22 (s, 1H), 7.78 (t, J = 6.1 Hz, 2H), 7.18 (t, J = 8.1 Hz, 2H), 5.35 (s, 2H), 4.49 (br. s., 2H), 4.14 -4.01 (m, 2H), 4.01 -3.89 (m, 2H), 3.89 -3.69 (m, 2H), 3.33 (br. s., 1H), 2.99 (br. s., 1H), 2.92 (br. s., 1H), 2.61 (s, 3 H), 2.19 (d, J = 6.8 Hz, 1H), 1.99 (d, J = 6.8 Hz, 1H); LC-MS: m/z [M+H]⁺ =478.

### Example 6

### (S)-8-(5-((3-(4-fluorophenyl)-5-methylisoxazol-4-yl)methoxy)pyrazin-2-yl)hexahydro-2 H-pyrazino [1,2-a]pyrazin-1(6H)-one

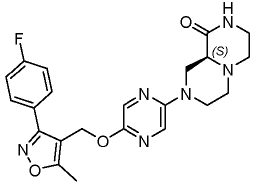

### Method F

4-(((5-bromopyrazin-2-yl)oxy)methyl)-3-(4-fluorophenyl)-5-methylisoxazole (50 mg, 0.135 mmol, Intermediate 5), cesium carbonate (110 mg, 0.34 mmol), (S)-hexahydro-2H-pyrazino[1,2-a]pyrazin-1(6H)-one hydrochloride (46 mg, 0.24 mmol, Intermediate 63), 2-dicyclohexylphosphino-2',6'-diisopropoxy-1,1'-biphenyl (13 mg, 0.027 mmol), (2-dicyclohexylphosphino-2',6'-diisopropoxy-1,1'-biphenyl)(2-amino-1,1'-biphenyl-2-yl)palla dium(II) mesylate (23 mg, 0.027 mmol) were dissolved in 1,4-dioxane (5 mL), and the reaction mixture was heated in an argon atmosphere to 110°C and stirred overnight. The mixture was filtered over diatomaceous earth, saturated ammonium chloride solution was added to the filtrate, then the filtrate was extracted with ethyl acetate three times, and then the organic phases were combined, dried, concentrated, and purified by column chromatography (dichloromethane/methanol = 20/1) to obtain the title compound as a light yellow solid (8 mg, yield 7%), named as Compound 6. ¹H NMR (400MHz, CDCl₃) δ 7.88 (s, 1H), 7.84 -7.76 (m, 2H), 7.74 (s, 1H), 7.16 (t, J = 8.3 Hz, 2H), 5.84 (br. s., 1H), 5.16 (s, 2H), 4.44 (d, J = 11.2 Hz, 1H), 4.16 (d, J = 13.2 Hz, 1H), 3.66 (br. s., 1H), 3.30 (d, J = 11.7 Hz, 1H), 3.11 -2.84 (m, 4 H), 2.65 (br. s., 1H), 2.57 (s, 3 H), 2.49 (br. s., 1H); LC-MS: m/z [M+H]⁺ =439.

### Example 7

### 3-(6-((3-(4-fluorophenyl)-5-methylisoxazol-4-yl)methoxy)pyridin-3-yl)-5,6-dihydro-8H-[1,2,4]triazolo[3,4-c][1,4]thiazine 7,7-dioxide

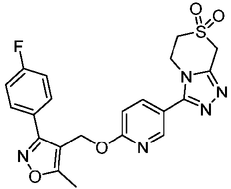

### Method G

4-(((5-(5,6-Dihydro-8H-[1,2,4]triazolo[3,4-c][1,4]thiazin-3-yl)pyridin-2-yl)oxy)methyl)-3-(4-fluorophenyl)-5-methylisoxazole (280 mg, 0.9 mmol, Intermediate 48) and sodium tungstate (650 mg, 4.5 mmol), hydrogen peroxide (620 mg, 4.8 mmol) were added to methanol (8 mL), and stirred for 2 hours. The reaction liquid was filtered, and then the filtrate was concentrated, and separated by preparative thin layer chromatography (petroleum ether/ethyl acetate = 1/2) to obtain the title compound (30 mg, yield 17%), named as Compound 7. ¹H NMR (400 MHz, CDCl₃) δ 8.45 (s, 1H), 7.91 (d, J = 8.8 Hz, 1H), 7.86 - 7.74 (m, 2H), 7.17 (t, J = 8.6 Hz, 2H), 6.95 (d, J = 8.3 Hz, 1H), 5.32 (s, 2H), 4.73 (s, 2H), 4.57 (t, J = 5.4 Hz, 2H), 3.51 (br. s., 2H), 2.60 (s, 3 H); LC-MS: m/z [M+H]⁺ =456.

### Example 8

### Method H

### 3-(6-((3-(4-fluorophenyl)-5-methylisoxazol-4-yl)methoxy)-2-methoxypyridin-3-yl)-5,6-d ihydro-8H-[1,2,4]triazolo[3,4-c][1,4]oxazine

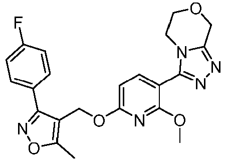

6-((3-(4-Fluorophenyl)-5-methylisoxazol-4-yl)methoxy)-2-methoxynicotinohydrazine (500 mg, 1.34 mmol, Intermediate 20) was added to toluene (50 mL), to which were added triethylamine (2 mL, 10.1 mmol) and 5-chloro-3,6-dihydro-2H-1,4-oxazine (240 mg, 2.01 mmol, Intermediate 28) separately, and, after stirring at 130°C for 4 hours, saturated sodium bicarbonate solution was added till pH=10-11. The reaction solution was stirred at 100°C for 16 hours, diluted with water (100 mL), and extracted with ethyl acetate (100 mL) twice, and then the organic phases were combined, dried, concentrated, and purified by column chromatography (dichloromethane/ethyl acetate= 20/1) to obtain the title compound (5.2 mg), named as Compound 8. ¹H NMR (400 MHz, CD₃OD) δ 2.57 (s, 3 H), 3.96 (s, 3 H), 3.99 (d, J=4.88 Hz, 2H), 4.05 (d, J=4.88 Hz, 2H), 4.99 (s, 2H), 5.40 (s, 2H), 6.55 (d, J=8.32 Hz, 1H), 7.23 (t, J=8.32 Hz, 2H), 7.70 -7.78 (m, 2H), 7.80 (d, J=8.32 Hz, 1H); LC-MS: m/z [M+H]⁺ =438.

### Example 9

### 3-(5-((3-(4-fluorophenyl)-5-methylisoxazol-4-yl)methoxy)pyrazin-2-yl)-7,8-dihydro-1H, 6H,9H-7,8a-methanopyrrolo[1',2':3.4]imidazo[1,2-c]pyrimidin-1-one

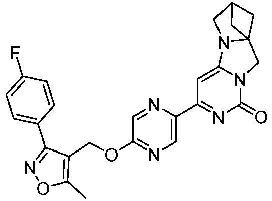

### Method I

3-(4-Fluorophenyl)-5-methyl-4-(((5-(tributylstannyl)pyrazin-2-yl)oxy)methyl)isoxazole (200 mg, 0.35 mmol, Intermediate 61), 3-chloro-7,8-dihydro-1H,6H,9H-7,8a-methanopyrrolo[1',2':3,4]imidazo[1,2-c]pyrimidin-1-on e (78 mg, 0.35 mmol, Intermediate 57) were added to 1,4-dioxane (4 mL), to which were added tetrakis(triphenylphosphine)palladium (40 mg, 0.035 mmol) and cuprous iodide (10 mg, 0.053 mmol), and the reaction mixture was refluxed and stirred overnight in argon atmosphere. The reaction liquid was filtered over diatomaceous earth, and then the filtrate was concentrated, and purified by column chromatography (dichloromethane/methanol = 20/1) to obtain the title compound as a light yellow solid (12 mg, yield 7%), named as Compound 9. ¹H NMR (400 MHz, CDCl₃) δ 9.33 -9.24 (m, 1H), 8.27 -8.19 (m, 1H), 7.87 -7.74 (m, 2H), 7.22 -7.12 (m, 2H), 6.64 (s, 1H), 5.35 (br. s., 2H), 4.22 -4.05 (m, 2H), 3.60 -3.43 (m, 2H), 3.14 -3.04 (m, 1H), 2.60 (s, 3 H), 2.14 (br. s., 2H), 1.76 (br. s., 2H); LC-MS: m/z [M+H]⁺ =473.

### Example 10

### 8-(6-((3-(4-fluorophenyl)-5-methylisoxazol-4-yl)methoxy)pyridazin-3-yl)hexahydro-2H-pyrazino [1,2-a]pyrazin-1(6H)-one

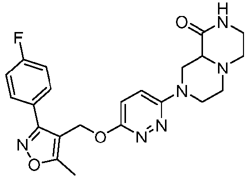

### Method J

Under nitrogen protection, methyl 1-(2-aminoethyl)-4-(6-((3-(4-fluorophenyl)-5-methylisoxazol-4-yl)methoxy)pyridazin-3-yl)pi perazine-2-carboxylate (200 mg, 0.43 mmol, Intermediate 62) was dissolved in methanol (50 mL), to which was added triethylamine (0.44 g, 4.3 mmol), and the reaction mixture was stirred at 60°C for 1 hour. The reaction solution was quenched with water (60 mL), and extracted with ethyl acetate (50 mL) three times. The organic phases were combined, washed with saturated brine (80 mL), dried over anhydrous sodium sulfate, and filtered, and then the filtrate was concentrated under reduced pressure. Then, the residue was purified by column chromatography (dichloromethane/methanol = 20/1) to obtain the title compound (25mg), named as Compound 10. ¹H NMR (400 MHz, DMSO-d6) δ 7.78-7.75 (m, 3 H), 7.39 -7.34 (m, 3 H), 7.03 (d, J = 9.6 Hz, 1H), 5.28 (s, 2H), 4.47 - 4.45 (m, 1H), 4.04 - 4.01 (m, 1H), 3.35 - 3.32 (m, 1H), 3.10 - 3.07 (m, 1H), 2.96 - 2.89 (m, 3 H), 2.65 - 2.63 (m, 2H), 2.55 (s, 3 H), 2.44-2.38 (m, 1H), 2.31-2.24 (m, 1H); LC-MS: m/z [M+H]⁺ =439.

The compounds in the following table were prepared according to the method (Method A) of Example 1 except that the same molar amount of the starting material in the column "Starting material" was used instead of 3-(6-fluoropyridin-3-yl)-6,7-dihydro-4H-[1,2,3]triazolo[5,1-c][1,4]oxazine in Example 1:

| Compound No. | Name | Structure | Starting material |
|---|---|---|---|
| 11 | 3-(6-((3-(4-fluorophenyl)-5-methylisoxazol-4-yl) methoxy)pyridazin-3-yl)-6,7-dihydro-4H-[1,2,3]triazolo[5,1-c][1,4]oxazine | 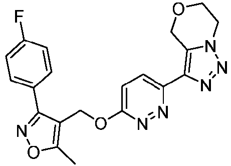 | Intermediate 2 |
| 12 | 3-(5-((3-(4-fluorophenyl)-5-methylisoxazol-4-yl) methoxy)pyrazin-2-yl)-6,7-dihydro-4H-[1,2,3]triazolo[5, 1-c][1,4]oxazine | 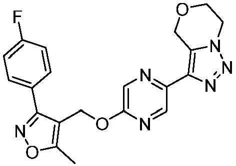 | Intermediate 3 |
| 17 | 3-(6-((3-(4-fluorophenyl)-5-methylisoxazol-4-yl) methoxy)pyridin-3-yl)-5,6-dihydro-8H-[1,2,4]triazolo[3,4-c][1,4]oxazine | 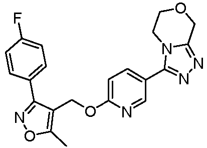 | Intermediate 34 |
| 18 | 3-(6-((3-(4-fluorophenyl)-5-methylisoxazol-4-yl) methoxy)pyridazin-3-yl)-5,6-dihydro-8H-[1,2,4]triazolo[3,4-c][1,4]oxazine | 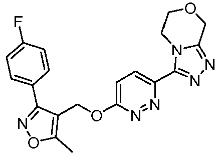 | Intermediate 35 |
| 19 | 3-(5-((3-(4-fluorophenyl)-5-methylisoxazol-4-yl) methoxy)pyrazin-2-yl)-5,6-dihydro-8H-[1,2,4]triazolo[3,4-c][1,4]oxazine | 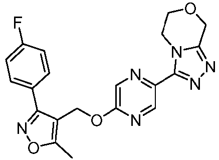 | Intermediate 37 |
| 20 | 3-(6-((3-(4-fluorophenyl)-5-methylisoxazol-4-yl) methoxy)pyridin-3-yl)-6,7-dihydro-5H,9H-[1,2,4]triazolo[3,4-c][1,4]oxazepane | 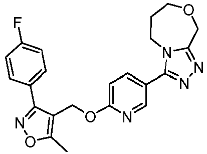 | Intermediate 38 |
| 21 | 3-(6-((3-(4-fluorophenyl)-5-methylisoxazol-4-yl) methoxy)pyridazin-3-yl)-6,7-dihydro-5H, 9H-[1,2,4]triazolo[3,4-c][1,4]oxazepane | 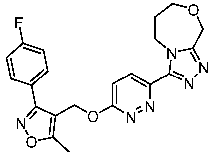 | Intermediate 39 |
| 22 | 4-(((6-(6,7-dihydro-5H-pyrrolo[2,1-c][1,2,4]triazol-3-yl)pyridazin-3-yl)oxy)methyl)-3-(4-fluorophenyl)-5-methylisoxazole | 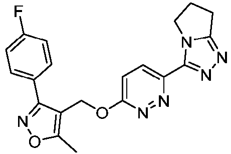 | Intermediate 40 |
| 23 | 4-(((5-(6,7-dihydro-5H-pyrrolo[2,1-c][1,2,4]triazol-3 -yl)pyrazin-2-yl)oxy)methyl)-3-(4-fluorophenyl)-5-methylisoxazole | 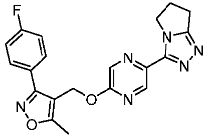 | Intermediate 41 |
| 24 | 3-(5-((3-(4-fluorophenyl)-5-methylisoxazol-4-yl) methoxy)pyrazin-2-yl)-6,7-dihydro-5H,9H-[1,2,4]triazolo[3,4-c][1,4]oxazepane | 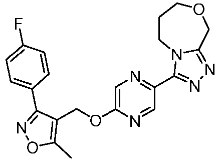 | Intermediate 42 |
| 25 | 3-(6-((3-(4-fluorophenyl)-5-methylisoxazol-4-yl) methoxy)pyridin-3-yl)-6,7-dihydro-5H-[1,2,4]triazolo[3,4-b][1,3]oxazine | 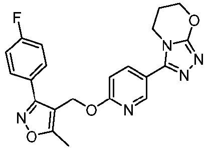 | Intermediate 50 |
| 28 | 3-(6-((3-(4-fluorophenyl)-5-methylisoxazol-4-yl) methoxy)pyridin-3-yl)-5,6-dihydro-8H-imidazo [5,1-c][1,4] oxazine | 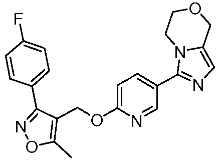 | Intermediate 52 |
| 32 | 3-(4-fluorophenyl)-5-methyl-4-(((5-(5,6,7,8-tetrahydro-[1,2,4]triazolo[4,3-a]pyridin-3-yl) pyrazin-2-yl)oxy)methyl)isoxazole | 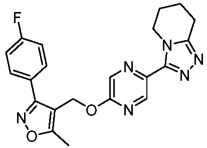 | Intermediate 44 |
| 33 | 3-(5-((3-(4-fluorophenyl)-5-methylisoxazol-4-yl) methoxy)pyrazin-2-yl)-5,6,8,9-tetrahydro-[1,2,4]triazolo [4,3-d][1,4]oxazepane | 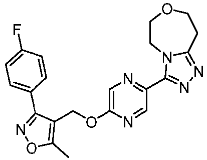 | Intermediate 45 |
| 42 | 3-(5-((3-(4-fluorophenyl)-5-methylisoxazol-4-yl) methoxy)-3-methylpyrazin-2-yl)-5,6-dihydro-8H-[1,2,4]triazolo[3,4-c][1,4]oxazine | 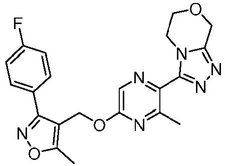 | Intermediate 46 |
| 43 | 3-(6-((3-(4-fluorophenyl)-5-methylisoxazol-4-yl) methoxy)-2-methylpyridin-3-yl)-5,6-dihydro-8H-[1,2,4]triazolo[3,4-c][1,4]oxazine | 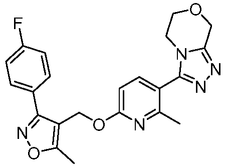 | Intermediate 47 |
| 50 | 1-(6-((3-(4-fluorophenyl)-5-methylisoxazol-4-yl) methoxy)pyridin-3-yl)-1,4,6,7-tetrahydropyran[3,4-d][1,2,3]triazole | 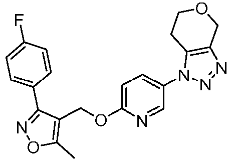 | Intermediate 64 |

Compound 11: ¹H NMR (400 MHz) CDCl₃: δ 8.35 (d, J = 8.8 Hz, 1H), 7.81 -7.68 (m, 2H), 7.22 -7.06 (m, 3 H), 5.47 (s, 2H), 5.33 (s, 2H), 4.54 (t, J = 4.9 Hz, 2H), 4.18 (t, J = 4.9 Hz, 2H), 2.59 (s, 3 H). LC-MS: m/z [M+H]⁺ =409.

Compound 12: ¹H NMR (400 MHz) CDCl₃: δ 8.97 (s, 1H), 8.19 (s, 1H), 7.87 -7.68 (m, 2H), 7.17 (t, J = 8.6 Hz, 2H), 5.31 (s, 2H), 5.21 (s, 2H), 4.51 (t, J = 4.6 Hz, 2H), 4.15 (t, J = 4.9 Hz, 2H), 2.61 (s, 3 H). LC-MS: m/z [M+H]⁺ =409.

Compound 17: ¹H NMR (400 MHz) CDCl₃: δ 8.51 (s, 1H), 8.01 (d, J = 8.8 Hz, 1H), 7.88 -7.71 (m, 2H), 7.17 (t, J = 8.3 Hz, 2H), 6.93 (d, J = 8.8 Hz, 1H), 5.32 (s, 2H), 5.09 (s, 2H), 4.14 (d, J = 4.9 Hz, 2H), 4.09 (d, J = 4.9 Hz, 2H), 2.61 (s, 3 H). LC-MS: m/z [M+H]⁺ =408.

Compound 18: ¹H NMR (400 MHz) CDCl₃: δ 8.46 (d, J = 9.3 Hz, 1H), 7.81 -7.65 (m, 2H), 7.21 -7.09 (m, 3 H), 5.50 (s, 2H), 5.11 (s, 2H), 4.69 (t, J = 4.9 Hz, 2H), 4.11 (t, J = 4.9 Hz, 2H), 2.61 (s, 3 H). LC-MS: m/z [M+H]⁺ =409.

Compound 19: ¹H NMR (400 MHz) CDCl₃: δ 9.11 (s, 1H), 8.23 (s, 1H), 7.85 -7.71 (m, 2H), 7.18 (t, J = 8.6 Hz, 2H), 5.35 (s, 2H), 5.08 (s, 2H), 4.53 (br. s., 2H), 4.08 (br. s., 2H), 2.61 (s, 3 H). LC-MS: m/z [M+H]⁺ =409.

Compound 20: ¹H NMR (400 MHz) CDCl₃: δ 8.37 (s, 1H), 7.93 -7.72 (m, 3 H), 7.16 (t, J = 8.3 Hz, 2H), 6.92 (d, J = 8.8 Hz, 1H), 5.31 (s, 2H), 4.92 (s, 2H), 4.27 -4.15 (m, 2H), 4.15 -4.06 (m, 2H), 2.60 (s, 3 H), 2.04 (br. s., 2H). LC-MS: m/z [M+H]⁺ =422.

Compound 21: ¹H NMR (400 MHz) CDCl₃: δ 8.44 (d, J = 8.8 Hz, 1H), 7.80 -7.66 (m, 2H), 7.22 -7.08 (m, 3 H), 5.50 (s, 2H), 5.14 -4.98 (m, 2H), 4.94 (s, 2H), 4.22 -4.02 (m, 2H), 2.60 (s, 3 H), 2.10 (br. s., 2H). LC-MS: m/z [M+H]⁺ =423.

Compound 22: ¹H NMR (400 MHz) CDCl₃: δ 8.40 (d, J = 9.3 Hz, 1H), 7.73 (dd, J = 5.1, 8.6 Hz, 2H), 7.20 -7.05 (m, 3 H), 5.49 (s, 2H), 4.50 (t, J = 7.3 Hz, 2H), 3.09 (t, J = 7.6 Hz, 2H), 2.86 (t, J = 7.3 Hz, 2H), 2.60 (s, 3 H). LC-MS: m/z [M+H]⁺ =393.

Compound 23: ¹H NMR (400 MHz) CDCl₃: δ 9.07 (s, 1H), 8.21 (s, 1H), 7.85 -7.67 (m, 2H), 7.17 (t, J = 8.6 Hz, 2H), 5.34 (s, 2H), 4.36 (t, J = 7.1 Hz, 2H), 3.05 (t, J = 7.1 Hz, 2H), 2.92 -2.73 (m, 2H), 2.60 (s, 3 H). LC-MS: m/z [M+H]⁺ =393.

Compound 24: ¹H NMR (400 MHz) DMSO-d6: δ 8.83 (s, 1H), 8.45 (s, 1H), 7.78 (d, J = 5.4 Hz, 2H), 7.37 (t, J = 8.1 Hz, 2H), 5.39 (s, 2H), 4.82 (s, 2H), 4.64 (m, 2H), 3.99 (s, 2H), 2.60 (m, 3H), 1.89 (m, 2H). LC-MS: m/z [M+H]⁺ =423.

Compound 25: ¹H NMR (400 MHz) CDCl₃: δ 8.45 (s, 1H), 7.98 (d, J = 8.6 Hz, 1H), 7.78 (t, J = 12 Hz, 2H), 7.15 (t, J = 8.3 Hz, 2H), 6.89 (d, J = 8.7 Hz, 1H), 5.28 (s, 2H), 4.52 - 4.49 (m, 2H), 4.12 (t, J = 5.9 Hz, 2H), 2.59 (s, 3H), 2.27 - 2.24 (m, 2H). LC-MS: m/z [M+H]⁺ =408.

Compound 28: ¹H NMR (400 MHz) CDCl₃: δ 8.43 (br. s., 1H), 7.93 (d, J = 8.3 Hz, 1H), 7.84 -7.76 (m, 2H), 7.16 (t, J = 8.3 Hz, 2H), 6.94 (br. s., 1H), 6.86 (d, J = 8.3 Hz, 1H), 5.28 (s, 2H), 4.95 (s, 2H), 4.22 -3.91 (m, 4 H), 2.69 -2.52 (m, 3 H); LC-MS: m/z [M+1]⁺ =407.

Compound 32: ¹H NMR (400 MHz) DMSO-d6: δ 8.84 (s, 1H), 8.40 (s, 1H), 7.84 -7.71 (m, 2H), 7.35 (t, J = 8.6 Hz, 2H), 5.43 -5.29 (m, 2H), 4.26 (t, J = 5.4 Hz, 2H), 2.91 (t, J = 6.1 Hz, 2H), 2.57 (s, 3 H), 1.98 -1.76 (m, 4 H). LC-MS: m/z [M+H]⁺=407.

Compound 33: ¹H NMR (400 MHz) DMSO-d6: δ 8.80 (s, 1H), 8.41 (s, 1H), 7.81 -7.71 (m, 2H), 7.35 (t, J = 8.6 Hz, 2H), 5.36 (s, 2H), 4.60 (br. s., 2H), 3.79 (br. s., 4 H), 3.19 (br. s., 2H), 2.57 (s, 3 H). LC-MS: m/z [M+H]⁺ =423.

Compound 42: ¹H NMR (400 MHz) CD₃OD: δ 2.53 (s, 3 H), 2.74 (s, 3 H), 4.02 (t, J=4.88 Hz, 2H), 4.25 (t, J=5.12 Hz, 2H), 5.03 (s, 2H), 5.36 (s, 2H), 7.19 (t,J=8.56 Hz, 2H), 7.61 -7.76 (m, 2H), 8.27 (s, 1H). LC-MS: m/z [M+H]⁺ =423.

Compound 43: ¹H NMR (400 MHz) CD₃OD: δ 2.39 (s, 3 H), 2.57 -2.69 (m, 3 H), 3.91 (d, J=4.40 Hz, 2H), 4.05 (d, J=4.40 Hz, 2H), 5.00 (s, 2H), 5.33 -5.43 (m, 2H), 6.79 (d, J=8.32 Hz, 1H), 7.25 (t, J=8.32 Hz, 2H), 7.72 (d, J=8.32 Hz, 1H), 7.83 (t, J=6.12 Hz, 2H). LC-MS: m/z [M+H]⁺ =422.

Compound 50: ¹H NMR (400 MHz) DMSO-d6: δ 8.52 (d, J = 2.5 Hz, 1H), 8.08 (dd, J = 8.9, 2.7 Hz, 1H), 7.86 - 7.73 (m, 2H), 7.37 (t, J = 8.9 Hz, 2H), 7.08 (d, J = 8.8 Hz, 1H), 5.34 (s, 2H), 4.78 (s, 2H), 3.89 (t, J = 5.5 Hz, 2H), 2.92 (t, J = 5.4 Hz, 2H), 2.59 (s, 3H). LC-MS: m/z [M+H]⁺ =408.

The compounds in the following table were prepared according to the method (Method B) of Example 2 except that the same molar amount of the starting material in the column "Starting material" was used instead of 3-bromo-7-methyl-6,7-dihydro-[1,2,4]triazolo[4,3-a]pyrazin-8(5H)-one in Example 2:

| Compound No. | Name | Structure | Starting material |
|---|---|---|---|
| 13 | 7-ethyl-3-(6-((3-(4-fluorophenyl)-5-methylisoxazol-4-yl) methoxy)pyridin-3-yl)-6,7-dihydro-[1,2,4]triazolo[4,3-a]pyrazin-8(5H)-one | 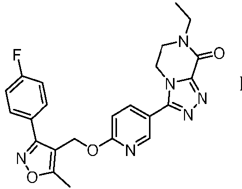 | Intermediate 10 |
| 30 | 1-(3-(6-((3-(4-fluorophenyl)-5-methylisoxazol -4-yl)methoxy)pyridin-3-yl)-5,6-dihydro-[1,2,4]triazolo[4,3-a]pyrazin-7(8H)-yl)ethan-1-one | 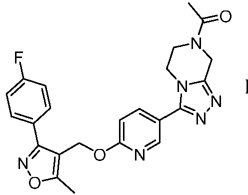 | Intermediate 54 |
| 31 | 3-(4-fluorophenyl)-5-methyl-4-(((5-(7-(methylsulfonyl) -5,6,7,8-tetrahydro-[1,2,4]triazolo[4,3-a]pyrazin-3-yl) pyridin-2-yl)oxy)methyl)isoxazole | 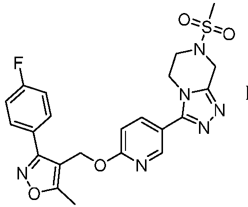 | Intermediate 55 |
| 38 | (3S,11aR)-7-(6-((3-(4-fluorophenyl)-5-methylisoxazol-4-yl) methoxy)pyridin-3-yl-3,4-dihydro-1H,9H,11H-3,11a-methanopyrimido[6',1':2,3]imidazo[5,1-c][1,4]oxazin-9-one | 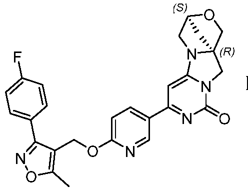 | Intermediate 56 |
| 39 | 3-(6-((3-(4-fluorophenyl)-5-methylisoxazol-4-yl) methoxy)pyridin-3-yl)-7,8-dihydro-1H,6H,9H-7,8a-methanopyrrolo[1 ',2':3,4]imidazo[1,2-c]pyrimidin-1-one | 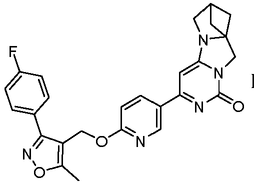 | Intermediate 57 |
| 41 | 7-(6-((3-(4-fluorophenyl)-5-methylisoxazol-4-yl) methoxy)pyridin-3-yl)-1-methyl-2,3-dihydroimidazo[1,2-c]pyrimidin-5(1H)-one | 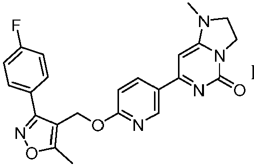 | Intermediate 58 |

Compound 13: ¹H NMR (400 MHz) CDCl₃: δ 8.49 (br. s., 1H), 8.04 (d, J = 8.3 Hz, 1H), 7.79 (br. s., 2H), 7.17 (t, J = 8.1 Hz, 2H), 6.95 (d, J = 8.3 Hz, 1H), 5.32 (s, 2H), 4.37 (br. s., 2H), 3.80 (br. s., 2H), 3.72 (d, J = 6.8 Hz, 2H), 2.60 (s, 3 H), 1.34 (br. s., 3 H). LC-MS: m/z [M+H]⁺ =449.

Compound 30: ¹H NMR (400 MHz) DMSO-d6: δ 8.53 (br. s., 1H), 8.07 (br. s., 1H), 7.79 (br. s., 2H), 7.37 (d, J = 5.9 Hz, 2H), 7.01 (br. s., 1H), 5.33 (br. s., 2H), 4.95 (br. s., 1H), 4.83 (br. s., 1H), 4.20 (br. s., 1H), 4.10 (br. s., 1H), 3.86 (br. s., 2H), 2.58 (d, J = 4.4 Hz, 3 H), 2.15 (br. s., 3 H). LC-MS: m/z [M+H]⁺ =449.

Compound 31: ¹H NMR (400 MHz) DMSO-d6: δ 8.56 (br. s., 1H), 8.11 (d, J = 6.8 Hz, 1H), 7.80 (br. s., 2H), 7.42 -7.30 (m, 2H), 7.02 (d, J = 8.3 Hz, 1H), 5.33 (br. s., 2H), 4.64 (br. s., 2H), 4.26 (br. s., 2H), 3.64 (br. s., 2H), 3.11 (br. s., 3 H), 2.59 (br. s., 3 H). LC-MS: m/z [M+H]⁺ =485.

Compound 38: ¹H NMR (400 MHz) DMSO-d6: δ 8.85 (s, 1H), 8.32 (d, J = 8.3 Hz, 1H), 7.79 (t, J = 6.6 Hz, 2H), 7.35 (t, J = 8.6 Hz, 2H), 6.91 (d, J = 8.8 Hz, 1H), 6.42 (s, 1H), 5.32 (s, 2H), 4.71 (s, 1H), 4.35 (d, J = 12.2 Hz, 1H), 4.01 (d, J = 12.7 Hz, 1H), 3.93 -3.86 (m, 2H), 3.43 (s, 2H), 2.58 (s, 3 H), 1.89 (q, J = 9.9 Hz, 2H). LC-MS: m/z [M+H]⁺ =488.

Compound 39: ¹H NMR (400 MHz) CDCl₃: δ 8.81 (s, 1H), 8.33 (d, J = 8.3 Hz, 1H), 7.87 -7.76 (m, 2H), 7.15 (t, J = 8.6 Hz, 2H), 6.82 (d, J = 8.8 Hz, 1H), 5.98 (s, 1H), 5.29 (s, 2H), 4.11 (s, 2H), 3.48 (s, 2H), 3.09 (br. s., 1H), 2.60 (s, 3 H), 2.15 (br. s., 2H), 1.76 (d, J = 3.9 Hz, 2H). LC-MS: m/z [M+H]⁺ =472.

Compound 41: ¹H NMR (400 MHz) DMSO-d6: δ 8.81 (s, 1H), 8.28 (d, J = 8.8 Hz, 1H), 7.82 -7.74 (m, 2H), 7.35 (t, J = 8.8 Hz, 2H), 6.88 (d, J = 8.3 Hz, 1H), 6.22 (s, 1H), 5.29 (s, 2H), 3.97 (t, J = 8.6 Hz, 2H), 3.68 -3.61 (m, 2H), 2.97 (s, 3 H), 2.56 (s, 3 H). LC-MS: m/z [M+H]⁺ =434.

The compound in the following table was prepared according to the method (Method C) of Example 3 except that the same molar amount of the starting material in the column "Starting material" was used instead of 3-3-(4,4,5,5-tetramethyl-1,3,2-dioxaborolan-2-yl)-5,6-dihydro-4H-pyrrolo(1,2-b)pyrazole in Example 3:

| Compound No. | Name | Structure | Starting material |
|---|---|---|---|
| 44 | 3-(5-((3-(4-fluorophenyl)-5-methylisoxazol-4-yl) methoxy)pyrazin-2-yl)-6,7-dihydro-4H-pyrazolo[5,1-c][1,4]oxazine | 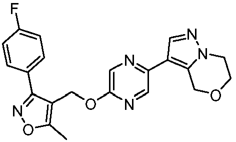 | Intermediate 59 |

Compound 44: ¹H NMR (400 MHz) CD₃OD: δ 2.51 -2.65 (m, 3 H), 4.13 (br. s, 2H), 4.19 (br. s, 2H), 5.12 (br. s, 2H), 5.33 (br. s., 2H), 7.24 (t, J=8.08 Hz, 2H), 7.80 (br. s, 2H), 8.00 (br. s, 1H), 8.19 (br. s, 1H), 8.43 (br. s, 1H). LC-MS: m/z [M+H]⁺ =408.

The compounds in the following table were prepared according to the method (Method D) of Example 4 except that the same molar amount of the starting material in the column "Starting material" was used instead of 4-((5-(7-cyclobutyl-5,6,7,8-tetrahydro-[1,2,4]triazolo[4,3-a]pyrazin-3-yl)pyrazin-2-yl)oxy)me thyl-3-(4-fluorohenyl-5-methylisoxazole in Example 4:

| Compound No. | Name | Structure | Starting material |
|---|---|---|---|
| 14 | 3-(6-((3-(4-fluorophenyl)-5-methylisoxazol-4-yl) methoxy)pyridazin-3-yl)-7-methyl-6,7-dihydro-[1,2,4]triazolo [4,3-a]pyrazin-8(5H)-one | 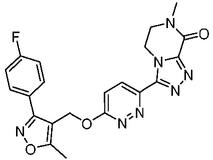 | Intermediate 26 |
| 26 | 7-(5-((3-(4-fluorophenyl)-5-methylisoxazol-4-yl) methoxy)pyrazin-2-yl)-3-(trifluoromethyl)-6,7-dihydro-[1,2,4]triazolo[4,3-a]pyrazin-8(5H)-one | 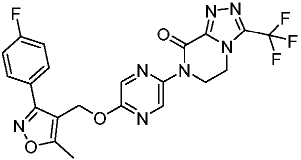 | Intermediate 51 |
| 36 | 7-(6-((3-(4-fluorophenyl)-5-methylisoxazol-4-yl) methoxy)pyridazin-3-yl)-3-methyl-6,7-dihydro-[1,2,4]triazolo[4,3-a]pyrazin-8(5H)-one | 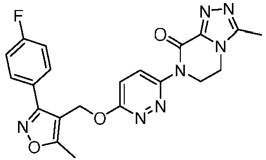 | Compound 34 |
| 37 | 7-(6-((3-(4-fluorophenyl)-5-methylisoxazol-4-yl) methoxy)pyridazin-3-yl)-6,7 - dihydroimidazo[1,2-a]pyrazin-8(5H)-one | 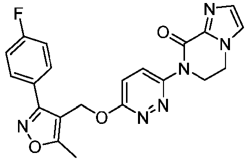 | Compound 35 |

Compound 14: ¹H NMR (400 MHz) CDCl₃: δ 8.60 -8.48 (m, 1H), 7.73 (br. s., 2H), 7.22 -7.11 (m, 3 H), 5.53 -5.47 (m, 2H), 5.05 -4.95 (m, 2H), 3.90 -3.80 (m, 2H), 3.29 -3.23 (m, 3 H), 2.63 -2.58 (m, 3 H). LC-MS: m/z [M+H]⁺=436.

Compound 26: ¹H NMR (400 MHz) CDCl₃: δ 8.95 (s, 1H), 8.08 (s, 1H), 7.81 - 7.73 (m, 2H), 7.17 (t, J = 8.4 Hz, 2H), 5.31 (s, 2H), 4.59 (d, J = 7.8 Hz, 4H), 2.60 (s, 3H). LC-MS: m/z [M+H]⁺ =490.

Compound 36: ¹H NMR (400 MHz) DMSO-d6: δ 8.00 (d, J = 9.3 Hz, 1H), 7.82 -7.73 (m, 2H), 7.40 -7.30 (m, 3 H), 5.43 (s, 2H), 4.46 (d, J = 5.4 Hz, 2H), 4.33 (d, J = 5.9 Hz, 2H), 2.58 (s, 3 H), 2.45 (s, 3 H). LC-MS: m/z [M+H]⁺ =436.

Compound 37: ¹H NMR (400 MHz) DMSO-d6: δ 7.99 (d, J = 9.8 Hz, 1H), 7.78 (t, J = 6.6 Hz, 2H), 7.51 (s, 1H), 7.39 -7.26 (m, 3 H), 7.22 (s, 1H), 5.42 (s, 2H), 4.43 (br. s., 4 H), 2.58 (s, 3 H). LC-MS: m/z [M+H]⁺ =421.

The compounds in the following table were prepared according to the method (Method E) of Example 5 except that the same molar amount of the starting material in the column "Starting material" was used instead of dihydro-3(2H)-furanone in Example 5:

| Compound No. | Name | Structure | Starting material |
|---|---|---|---|
| 15 | 3-(4-fluorophenyl)-5-methyl-4-((5-(7-(oxetan-3-yl) -5,6,7,8-tetrahydro-[1,2,4]triazolo[4,3-a]pyrazin-3-yl) pyrazin-2-yl)oxy)methyl)isoxazole | 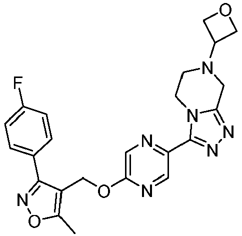 | 3-oxacyclobutanone |
| 16 | 3-(4-fluorophenyl)-5-methyl-4-((5-(7-methyl -5,6,7,8-tetrahydro-[1,2,4]triazolo[4,3-a]pyrazin-3-yl) pyrazin-2-yl)oxy)methyl)isoxazole | 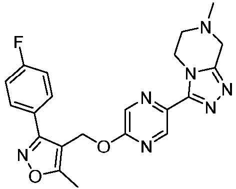 | formaldehyde |

Compound 15: ¹H NMR (400 MHz) CDCl₃: δ 9.08 (br. s., 1H), 8.21 (br. s., 1H), 7.77 (br. s., 2H), 7.22 -7.09 (m, 2H), 5.34 (br. s., 2H), 4.78 (br. s., 2H), 4.67 (br. s., 2H), 4.51 (br. s., 2H), 3.84 (br. s., 3 H), 2.83 (br. s., 2H), 2.60 (br. s., 3 H). LC-MS: m/z [M+H]⁺ =464.

Compound 16: ¹H NMR (400 MHz) CDCl₃: δ 9.08 (s, 1H), 8.22 (s, 1H), 7.78 (d, J = 6.4 Hz, 2H), 7.17 (t, J = 8.3 Hz, 2H), 5.34 (s, 2H), 4.49 (br. s., 2H), 3.89 (br. s., 2H), 2.89 (br. s., 2H), 2.56 (s, 3 H), 2.60 (s, 3 H). LC-MS: m/z [M+H]⁺ =422.

The compounds in the following table were prepared according to the method (Method F) of Example 6 except that the same molar amount of the starting material 1 and the same molar amount of the starting material 2 in the column "Starting material" were used instead of 4-(((5-bromopyrazin-2-yl)oxy)methyl)-3-(4-fluorophenyl)-5-methylisoxazole and (S)-hexahydro-2H-pyrazino[1,2-a]pyrazin-1(6H)-one hydrochloride in Example 6, respectively:

| Compound No. | Name | Structure | Starting material 1 | Starting material 2 |
|---|---|---|---|---|
| 27 | 3-(4-fluorophenyl)-5-methyl-4-((5-(3-methyl-5,6-dihydro-[1,2,4]triazolo [4,3-a]pyrazin-7(8H)-yl)pyrazin -2-yl)oxy)methyl)isoxazole | 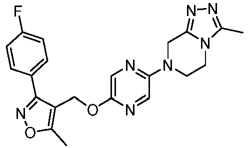 | Intermediate 5 | 3-methyl-5,6,7,8-tetrahydro- [1,2,4] triazolo [4,3-A]pyrazine (CAS: 886886-04-0) |
| 34 | 3-(4-fluorophenyl)-5-methyl -4-(((6-(3-methyl-5,6-dihydro - [1,2,4]triazolo[4,3-a]pyrazin-7(8H)-yl) pyridazin-3-yl)oxy)methyl)isoxazole | 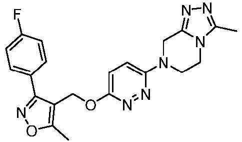 | Intermediate 6 | 3-methyl-5,6,7,8-tetrahydro- [1,2,4] triazolo [4,3-A]pyrazine (CAS: 886886-04-0) |
| 35 | 4-(((6-(5,6-dihydroimidazo [1,2-a]pyrazin-7(8H)-yl)pyridazin-3 -yl) oxy)methyl)-3-(4-fluorophenyl) -5-methylisoxazole | 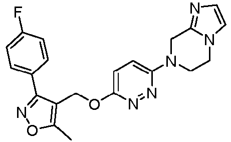 | Intermediate 6 | 5,6,7,8-tetrahy dro imidazolo[1,2-A] pyrazine (CAS: 91476-80-1) |
| 40 | (S)-8-(5-((3 -(4-fluorophenyl) -5-methylisoxazol -4-yl)methoxy)pyrazin-2-yl) octahydropyrazino[2,1-c][1,4]oxazine | 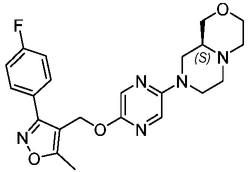 | Intermediate 5 | (9aS)-octahydropyrazino [2,1-c][1,4]oxazine (CAS: 1089759-42-1) |
| 45 | 2-(5-((3-(4-fluorophenyl) -5-methylisoxazol-4-yl)methoxy) pyrazin-2-yl)-3,4-dihy dropyrrolo [1,2-a]pyrazin-1(2H)-one | 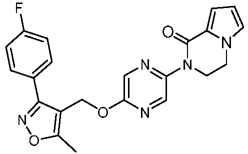 | Intermediate 5 | 3,4-dihydropyrrolo[1,2-a] pyrazin-1(2H)-one (CAS: 54906-42-2) |
| 46 | 3 -(4-fluorophenyl) -5-methyl-4-(((5-(2-methyl -5,6-dihydroimidazo [1,2-a]pyrazin-7(8H)-yl) pyrazin-2 -yl)oxy)methyl)isoxazole | 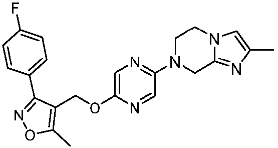 | Intermediate 5 | Intermediate 60 |
| 48 | 4-(((5-(5,6-dihydroimidazo [1,2-a]pyrazin-7(8H)-yl)pyrazin-2-yl) oxy)methyl)-3-(4-fluorophenyl) -5-methylisoxazole | 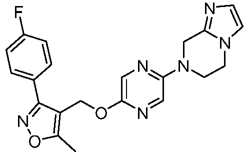 | Intermediate 5 | 5,6,7,8-tetrahydroimidazolo [1,2-A]pyrazine (CAS: 91476-80-1) |
| 49 | (S)-8- [6-((3 -(4-fluorophenyl) -5-methylisoxazol -4-yl)methoxy)pyridazin-3-yl] octahydropyrazino[2,1-c][1,4]oxazine | 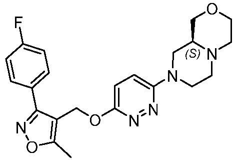 | Intermediate 6 | (9aS)-octahydropyrazino [2,1-c][1,4]oxazine (CAS: 1089759-42-1) |

Compound 27: ¹H NMR (400 MHz) CDCl₃: δ 7.90 (s, 1H), 7.77 (dd, J = 8.5, 5.4 Hz, 2H), 7.73 (s, 1H), 7.15 (t, J = 8.5 Hz, 2H), 5.17 (s, 2H), 4.76 (s, 2H), 4.07 - 4.04 (m, 2H), 4.00 (d, J = 5.3 Hz, 2H), 2.56 (s, 3H), 2.44 (s, 3H). LC-MS: m/z [M+H]⁺ =422.

Compound 34: ¹H NMR (400 MHz) DMSO-d6: δ 7.80 -7.72 (m, 2H), 7.62 (d, J = 9.8 Hz, 1H), 7.34 (t, J = 8.6 Hz, 2H), 7.13 (d, J = 9.8 Hz, 1H), 5.28 (s, 2H), 4.82 (s, 2H), 3.98 (s, 4 H), 2.54 (s, 3 H), 2.29 (s, 3 H). LC-MS: m/z [M+H]⁺ =422.

Compound 35: ¹H NMR (400 MHz) DMSO-d6: δ 7.81 -7.69 (m, 2H), 7.58 -7.52 (m, 1H), 7.34 (t, J = 8.6 Hz, 2H), 7.20 -7.09 (m, 2H), 6.94 (s, 1H), 5.28 (s, 2H), 4.74 (s, 2H), 4.10 (d, J = 4.4 Hz, 2H), 4.00 (d, J = 4.9 Hz, 2H), 2.54 (s, 3 H). LC-MS: m/z [M+H]⁺ =407.

Compound 40: ¹H NMR (400 MHz) CD₃OD: δ 7.89 -7.70 (m, 4 H), 7.33 -7.12 (m, 2H), 5.33 -5.13 (m, 2H), 4.05 (d, J = 12.2 Hz, 1H), 3.93 (d, J = 11.7 Hz, 1H), 3.89 -3.75 (m, 2H), 3.70 (t, J = 10.8 Hz, 1H), 3.23 -3.12 (m, 1H), 3.04 -2.85 (m, 2H), 2.85 -2.72 (m, 1H), 2.63 -2.52 (m, 3 H), 2.50 -2.32 (m, 4 H). LC-MS: m/z [M+H]⁺ =426.

Compound 45: ¹H NMR (400 MHz) CD₃OD: δ 2.60 (s, 3 H), 4.33 (br. s, 2H), 4.58 (br. s, 2H), 5.36 (s, 2H), 6.28 (br. s, 1H), 6.93 -7.06 (m, 2H), 7.25 (t, J=8.56 Hz, 2H), 7.74 -7.85 (m, 2H), 8.10 (s, 1H), 8.65 (s, 1H). LC-MS: m/z [M+H]⁺ =420.

Compound 46: ¹H NMR (400 MHz) CD₃OD: δ 2.17 (s, 3 H), 2.58 (s, 3 H), 3.96 -4.02 (m, 2H), 4.05 -4.12 (m, 2H), 4.60 (s, 2H), 5.24 (s, 2H), 6.76 (s, 1H), 7.24 (t, J=8.56 Hz, 2H), 7.77 -7.84 (m, 2H), 7.89 (d, J=3.42 Hz, 2H). LC-MS: m/z [M+H]⁺ =421.

Compound 48: ¹H NMR (400 MHz) CDCl₃: δ 7.91 (s, 1H), 7.83 -7.73 (m, 2H), 7.71 (s, 1H), 7.16 (t, J = 8.3 Hz, 2H), 7.09 (s, 1H), 6.91 (s, 1H), 5.17 (s, 2H), 4.68 (s, 2H), 4.14 (d, J = 4.9 Hz, 2H), 4.06 (d, J = 4.9 Hz, 2H), 2.57 (s, 3 H). LC-MS: m/z [M+H]⁺ =407.

Compound 49: ¹H NMR (400 MHz) DMSO-d6: δ 7.80 - 7.72 (m, 2H), 7.42 - 7.30 (m, 3 H), 7.04 (d, J = 9.6 Hz, 1H), 5.27 (s, 2H), 4.08 - 4.05 (m, 1H), 3.98 - 3.95 (m, 1H), 3.76 - 3.70 (m, 2H), 3.55 - 3.50 (m, 1H), 3.17 - 3.13 (m, 1H), 2.92 - 2.86 (m, 1H), 2.81-2.78 (m, 1H), 2.67 - 2.65 (m, 1H), 2.54 (s, 3H), 2.44 - 2.38 (m, 1H), 2.26 - 2.11 (m, 3 H). LC-MS: m/z [M+H]⁺ =426.

The compound in the following table was prepared according to the method (Method G) of Example 7 except that the same molar amount of the starting material in the column "Starting material" was used instead of 4-(((5-(5,6-dihydro-8H-[1,2,4]triazolo[3,4-c][1,4]thiazin-3-yl)pyridin-2-yl)oxy)methyl)-3-(4-fluorophenyl)-5-methylisoxazole in Example 7:

| Compound No. | Name | Structure | Starting material |
|---|---|---|---|
| 29 | 3-(6-((3-(4-fluorophenyl)-5-methylisoxazol-4-yl) methoxy)pyridazin-3-yl)-5,6-dihydro-8H -[1,2,4]triazolo[3,4-c][1,4]thiazine 7,7-dioxide | 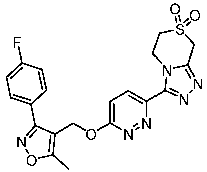 | Intermediate 49 |

Compound 29: ¹H NMR (400 MHz) DMSO-d6: δ 8.30 (d, J = 9.3 Hz, 1H), 7.81 -7.73 (m, 2H), 7.45 (d, J = 9.3 Hz, 1H), 7.36 (t, J = 8.8 Hz, 2H), 5.52 (s, 2H), 5.04 (s, 2H), 4.94 (t, J = 5.9 Hz, 2H), 3.94 -3.84 (m, 2H), 2.60 (s, 3 H). LC-MS: m/z [M+H]⁺ =457.

The compound in the following table was prepared according to the method (Method H) of Example 8 except that the same molar amount of the starting material in the column "Starting material" was used instead of 6-((3-(4-fluorophenyl)-5-methylisoxazol-4-yl)methoxy)-2-methoxynicotinohydrazine in Example 8:

| Compound No. I | Name | Structure | Starting material |
|---|---|---|---|
| 51 | 3-(6-((3-(4-fluorophenyl)-5-methylisoxazol-4-yl) methoxy)-5-methoxypyridin-3-yl)-5,6-dihydro -8H-[1,2,4]triazolo[3,4-c][1,4]oxazine | 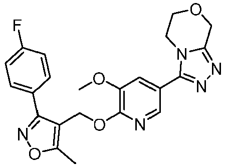 | Intermediate 21 |

Compound 51: ¹H NMR (400 MHz) CD₃OD: δ 7.56 (dd, J = 5.4, 8.8 Hz, 2H), 7.19 (t, J = 8.8 Hz, 2H), 7.12 (d, J = 5.9 Hz, 2H), 5.23 (s, 2H), 4.98 (s, 2H), 4.07 -4.02 (m, 2H), 3.88 (t, J = 4.9 Hz, 2H), 3.85 (s, 3 H), 2.59 (s, 3 H). LC-MS: m/z [M+H]⁺ =438.

The compound in the following table was prepared according to the method (Method I) of Example 9 except that the same molar amount of the starting material in the column "Starting material" was used instead of 3-chloro-7,8-dihydro-1H,6H,9H-7,8a-methanopyrrolo[1',2':3,4]imidazo[1,2-c]pyrimidin-1-on e in Example 9:

| Compound No. | Name | Structure | Starting material |
|---|---|---|---|
| 47 | 7-(5-((3-(4-fluorophenyl)-5-methylisoxazol -4-yl)methoxy)pyrazin-2-yl)-1-methyl -2,3-dihydroimidazo[1,2-c]pyrimidin-5(1H)-one | 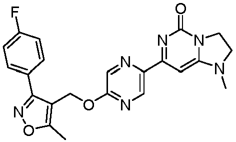 | Intermediate 58 |

Example 47: ¹H NMR (400 MHz) CDCl₃: δ 9.05 -8.94 (m, 1H), 8.35 -8.22 (m, 1H), 7.87 -7.70 (m, 2H), 7.23 -7.12 (m, 2H), 6.85 -6.72 (m, 1H), 5.32 (br. s., 2H), 4.18 (br. s., 2H), 3.73 (br. s., 2H), 3.12 (br. s., 3 H), 2.61 (br. s., 3 H). LC-MS: m/z [M+H]⁺ = 435.

### I. Biological experimental method:

### 1. Cell line construction and subculture

α, β and γ subunits are essential for the formation of a complete functional GABA_{A} receptor. In this example, HEK293 cell strains stably expressing human α5-GABA_{A} receptor (comprising α5β3γ2 subunit) and α2-GABA_{A} receptor (comprising α2β3γ2 subunit) were constructed and screened, respectively, by liposome transfection method (Felgner, P. L., et al., Proceedings of the National Academy of Sciences, 1987, 84: 7413-7417). The specific subunit protein sequences are as follows: α5 subunit (protein sequence can be found in GenBank accession number: NM_000810.3); α2 subunit (protein sequence can be found in GenBank accession number: NM_000807.4); β3 subunit (protein sequence can be found in GenBank accession number: NM_000814.5); γ2 subunit (protein sequence can be found in GenBank accession number: NM_000816.3).

The above cell lines were subcultured. Amplification of α5β3γ2-HEK293 and α2β3γ2-HEK293 cells was used to test the affinity of the compounds to benzodiazepine site (BZD) of the GABA_{A} receptor. During the passage of α5β3γ2-HEK293 cells, some of the suspended cells were spread on a glass slide pretreated with Poly-D-Lysine for electrophysiological testing.

### 2. Affinity activity of compounds of the invention to α5-GABA_{A} receptor and α2-GABA_{A} receptor

Flunitrazepam is a non-specific reagent that binds to the BZD site efficiently. The affinity of the compounds to the BZD sites of the human α5β3γ2 and α2β3γ2 receptors can be determined by competing the compound with ³H-flunitrazepam (isotope ³H-labeled flunitrazepam) for binding to these two receptors.

Membrane preparation: HEK293 cells stably expressing human α5β3γ2 receptor or α2β3γ2 receptor were collected, suspended in 50 mM Tris-HCl buffer (pH = 7.4), homogenized on ice by a homogenizer for 20 seconds 10 times, and centrifuged at 4°C, 1000 g for 10 min. The supernatant was collected, and the above steps were repeated. The supernatant was centrifuged (33800 g; centrifuge purchased from Thermo, rotor model: A27-8x50) at 4°C for 60 min, and the precipitate was resuspended in Tris buffer (50 mM Tris-HCl, 10 mM MgCl₂, 0.5 mM EDTA, 10% glycerol). The precipitate was the cell membrane. Protein content was determined by BCA (Bicinchoninic Acid) method (BCA kit from Pierce (Annoron)). The cell membrane was prepared into 1 ml aliquot and stored at -80°C.

Radioligand competition binding assay: the assay was carried out in a 200 µL system (96-well plate from Agilent) with 100 µL of cell membrane. The concentration of ³H-flunitazepam (from PerkinElmer, lot: NET567250 UC) was 1 nM and the concentration of test compound was in the range 1 × 10⁻⁵-10⁻⁶ M. Flumazenil (from Tocris; lot: 1318) was used as a control. 1 µL of 2 mM flumazenil (final concentration 10 µM) was added to the Low singal control well (Low control, LC) and 1 µL of DMSO (from Sigma aldrich, lot: 472301) was added to the High signal control well (High control, HC). The final target membrane protein concentration was 5 µg/well. All test compound sample stocks were 10 mM DMSO solutions. The working concentration of the samples is to dilute all samples with DMSO to 0.2 mM, and then perform 4-fold continuous gradient dilution for a total of 8 concentration gradients. The 96-well plate was sealed with a plate sealing membrane and then incubated on a shaker at room temperature for 1 hour. Meanwhile, GF/C filter plate was soaked with a plate soaking buffer (0.3% PEI, polyethyleneimine, purchased from Sigma aldrich, model: P314, stored at 4°C) for at least 0.5 hours. After completion of binding incubation, the cells were collected with a cell collector onto the GF/C filter plate and washed four times with a plate washing buffer (50 mM Tris-HCl, pH 7.4, stored at 4°C). After drying in an oven at 50°C for 1 hour, the dried GF/C filter plate was bottom-sealed with a membrane, and the residual radioactivity of the filter membrane was detected by liquid scintillation counting method with 50 µL of scintillation fluid added per well and sealed, and the use of Microbeta² (Microplate counter, purchased from PerkinElmer, Model: CNLL0153) for reading. The inhibitory activity of the test samples on the binding of ³H-flunitrazepam to the GABA_{A} receptor membrane protein was calculated, the IC₅₀ of each test sample was calculated by dose-effect curve fitting (by GraphPad Prism 5 software), and the Ki (inhibition constant) of the sample was calculated by IC₅₀, thereby evaluating the binding ability of the compounds to BZD sites of α5-GABA_{A} receptor and α2-GABA_{A} receptor.

### 3. Functional activity of compounds of the invention to α5-GABA_{A} receptor

The inverse regulation activity of the test drugs on α5-GABA_{A} receptor was detected by an electrophysiological method. The specific method was as follows:
Compound concentration setting: the final concentration of the compounds used for compound screening was 100 nM. GABA concentration was 0.05-0.06 µM (about EC₇₋₈). The electrophysiological assay was performed using the whole-cell patch clamp technique, for which a reference could be made to the method reported in the literature (Nickolls, S.A., et al., British Journal of Pharmacology, 2018, 175: 708-725). The extracellular fluid (ECS) ingredients for electrophysiology were as follows: 150 mM NaCl, 5 mM KCl, 2.5 mM CaCl₂, 1 mM MgCl₂, 10 mM HEPES and 10 mM glucose (pH 7.4); the electrode internal solution (ICS) formula for electrophysiology was as follows: 140 mM CsCl, 11 mM EGTA, 10 mM HEPES, 2 mM CaCl₂, 1 mM MgCl₂, 4 mM MgATP, 2 mM TEA (pH 7.3). The above reagents were all purchased from Sigma aldrich. GABA (γ-aminobutyric acid, from sigma aldrich, lot: A5385) powder was prepared into a stock solution with pure water and then diluted in ECS. The compound was first prepared into a 4 mM stock solution with DMSO, and then gradually diluted in corresponding concentrations of GABA-ECS. The solutions were all prepared fresh prior to electrophysiological testing.

Electrophysiological signal acquisition were performed by using EPC 10 amplifier and PatchMaster software (HEKA) or Axon 700 B amplifier and Clampex software (AXON). The recording electrode was made by drawing borosilicate glass (purchased from sutter), and has an electrode resistance of 4 ~ 6 MΩ. Extracellular administration was carried out using ALA-VC-8PG (purchased from ALA, USA) system. A single independently grown cell was selected, and lysis was performed after the glass electrode and the cell forming a good seal, to form a whole cell mode. With the cellular membrane potential clamped at -60 mV, recording was carried out in the Gap-free mode. For the test, extracellular fluid was first applied extracellularly for about 20 seconds. After baseline (I_{prebaseline}) was stabilized, the extracellular fluid was switched to GABA-ECS. At this time, the current (I_{gaba}) caused by GABA can be detected. At about 20-40 seconds, after the current stabilized, the extracellular fluid was switched to a mixed solution of the compound and GABA-ECS, until the current (Iₜᵣₑₐₜₘₑₙₜ) caused by both the compound and GABA can be detected. Finally, the solution was switched to the extracellular fluid and the test was terminated after recording for 20-40 seconds. Only cells with a stable baseline, an absolute control current level (I_{gaba}-I_{prebasepine}) greater than 40 pA, and a relatively stable current within 10-20 seconds were used for compound testing.

The experimental results were analyzed using PatchMaster v2x90.1 or PatchMaster v2x90.3 software (EPC 10 amplifier) and Clampex10.6 software (Axon700B amplifier). The current value of each stage was calculated according to the average value of the stabilized current under the corresponding conditions. We defined the control current as I_{gaba}- I_{prebaseline}, and the current after compound treatment as Iₜᵣₑₐₜₘₑₙₜ - I_{prebaseline}. The inverse regulation activity of the compounds is expressed as a percentage and is calculated according to the following formula: Inverse regulation efficiency = [(Iₜᵣₑₐₜₘₑₙₜ-I_{gaba})/(I_{gaba}-I_{prebaselme})]×100%.

**Table 1 Affinity activity and inverse regulation activity of compounds to α5-GABA_{A} receptor**

| Compound No. | Structure | α5-GABA_{A} receptor Ki (nM) | Inverse regulation efficiency to α5-GABA_{A} receptor (%) |
|---|---|---|---|
| 1 | 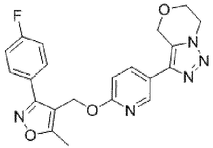 | 1.20 | -38 |
| 2 | 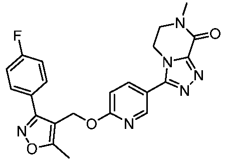 | 7.70 | -29 |
| 3 | 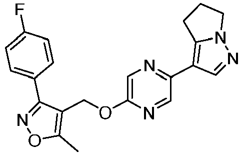 | 1.66 | -24 |
| 6 | 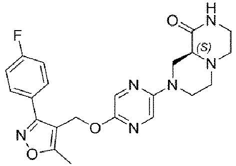 | 103.19 | -16 |
| 7 | 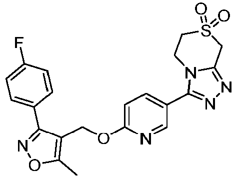 | 2.14 | -37 |
| 10 | 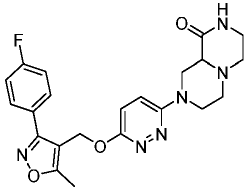 | 3.20 | -66 |
| 11 | 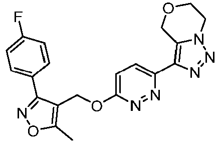 | 0.60 | -45 |
| 12 | 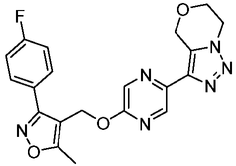 | 2.60 | -24 |
| 13 | 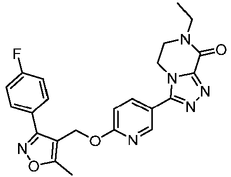 | 12.52 | -43 |
| 14 | 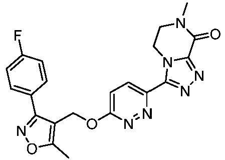 | 2.50 | -49 |
| 16 | 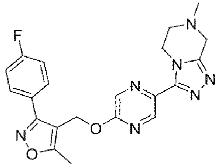 | 6.93 | -15 |
| 17 | 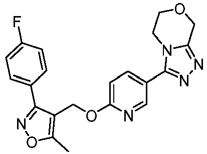 | 2.59 | -49 |
| 18 | 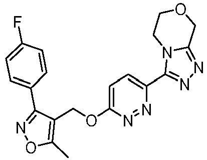 | 0.32 | -57 |
| 19 | 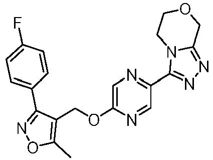 | 1.62 | -54 |
| 20 | 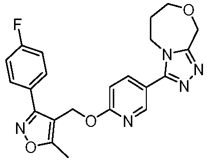 | 11.19 | -45 |
| 21 | 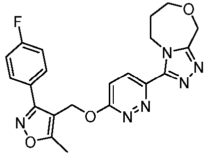 | 0.91 | -46 |
| 22 | 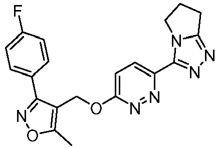 | 0.54 | -50 |
| 23 | 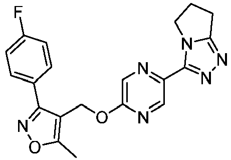 | 3.06 | -45 |
| 24 | 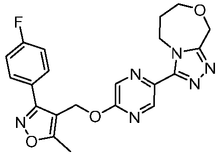 | 6.75 | -44 |
| 25 | 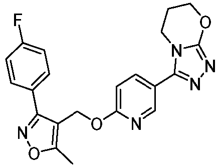 | 22.08 | -50 |
| 27 | 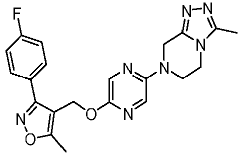 | 3.90 | -39 |
| 28 | 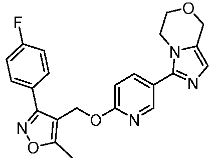 | 32.95 | -42 |
| 29 | 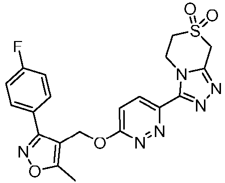 | 0.77 | -45 |
| 30 | 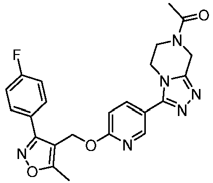 | 9.86 | -41 |
| 32 | 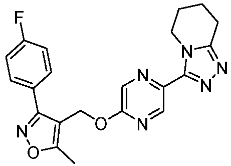 | 4.93 | -35 |
| 33 | 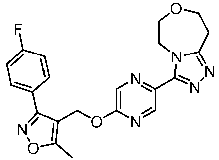 | 9.74 | -48 |
| 34 | 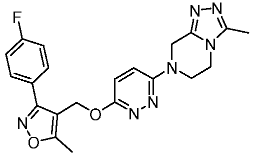 | 2.44 | -39 |
| 35 | 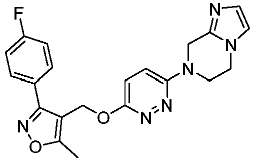 | 1.44 | -49 |
| 36 | 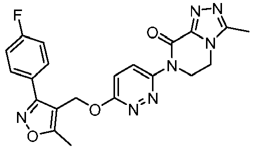 | 3.39 | -45 |
| 37 | 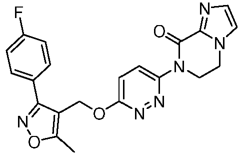 | 5.37 | -54 |
| 38 | 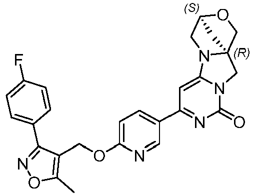 | 2.24 | -56 |
| 39 | 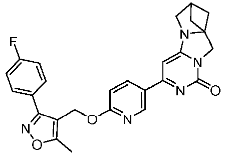 | 6.31 | -44 |
| 40 | 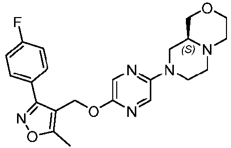 | 21.80 | -18 |
| 41 | 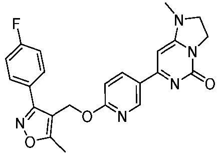 | 0.99 | -47 |
| 44 | 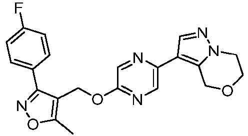 | 0.99 | -36 |
| 46 | 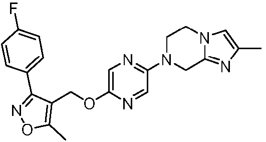 | 3.09 | -21 |
| 47 | 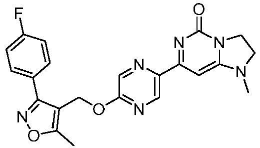 | NA | -31 |
| 48 | 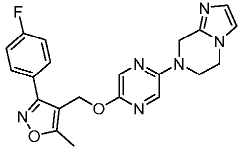 | 68.48 | -25 |
| 49 | 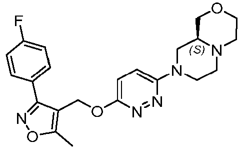 | 3.84 | -37 |
| 50 | 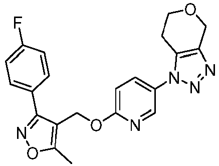 | 8.63 | -49 |

The results indicated that the compounds of the invention have good affinity activity and inverse regulation activity to α5-GABA_{A} receptor.

**Table 2 Receptor binding selectivity of compounds**

| Compound No. | Structure | Selectivity α2 ki/α5 ki (folds) |
|---|---|---|
| 7 | 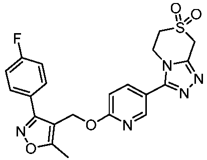 | 110.1 |
| 10 | 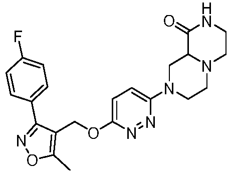 | 63.3 |
| 17 | 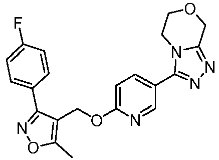 | 23.1 |
| 18 | 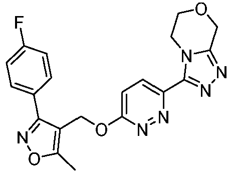 | 41.4 |
| 19 | 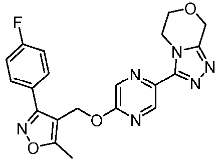 | 93.8 |
| 20 | 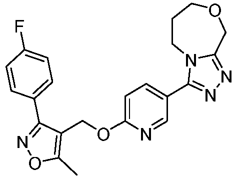 | 109 |
| 21 | 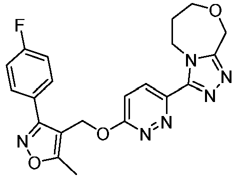 | 51 |
| 23 | 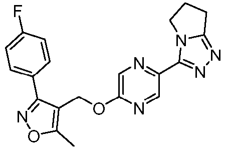 | 90.2 |
| 24 | 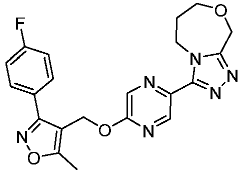 | 39.2 |
| 33 | 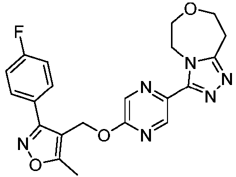 | 86.7 |
| 35 | 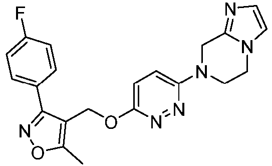 | 35.8 |
| 36 | 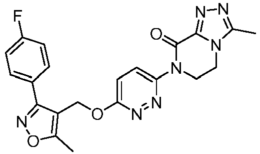 | 46.9 |
| 37 | 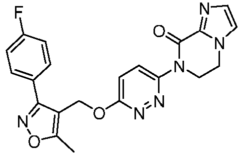 | 48.6 |
| 38 | 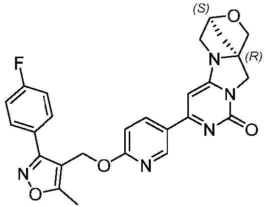 | 122.2 |
| 41 | 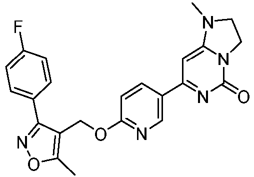 | 75.8 |
| WO2020016443A1, Example 3 | 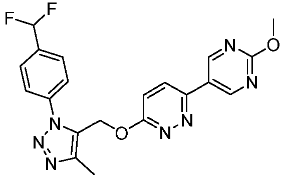 | 14.5 |
| WO2020016443A1, Example 6 | 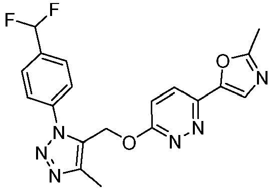 | 13.2 |
| WO2020016443A1, Example 15 | 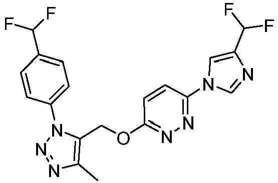 | 7.67 |

Compared with the comparative compounds, the compounds of the invention have better α5-GABA_{A} receptor subunit selectivity, less potential side effects caused by inverse regulation of α2-GABA_{A} receptor, and better safety.

### II. Solubility and stability in simulated gastric fluid (SGF) of the compounds of the invention

### 1. Thermodynamic solubility of the compounds in artificial simulated intestinal fluid

### (1) Experimental materials and equipment:

50 mM of phosphate buffer, pH = 7.4: 0.39 g of NaH₂PO₄•2H₂O, 1.4025 g of Na₂HPO₄ were weighed, placed into an Erlenmeyer flask, dissolved with the addition of 240 ml of water, homogenously mixed, adjusted to pH 7.4 with 10 M NaOH solution, transferred into a 250 ml volumetric flask and diluted with water to the scale.

Simulated intestinal fluid FaSSIF: 54.76 mg of FaSSIF-V2 (Biorelevant, batch. V2FAS-0619-A, lot. V2FAS01) was weighed, and added to 15 ml of buffer, and after dissolution, the solution was made up to 30 ml and left to stand at room temperature for 1 hour for use.

Waters e2695 HPLC high performance liquid chromatography, Mettler XSE105 analytical balance.

### (2) Experimental method:

Stock solution containing 10 mM of each test compound was formulated using DMSO, diluted into 1-200 M standard curve solution using diluent (ACN:PB buffer with a volume ratio of 50:50), and tested by HPLC to obtain a standard curve of concentration vs peak area of each test compound.

Thermodynamic solubility in simulated intestinal fluid (TS in FaSSIF). About 0.3 mg of each sample (test compound) was weighed, added into 1.5 mL of FaSSIF solution, two samples in parallel, shaken at 1000 rpm and 37°C for 4 hours, and filtered, 1 mL of primary filtrate was discarded, then 400 µL of subsequent filtrate was taken, and tested by using a high performance liquid chromatograph (UV). The test results of the compound of the invention are shown in the following table.

**Table 3 Test results of solubility**

| Compound No. | Structure | Thermodynamic solubility µg/mL |
|---|---|---|
| 7 | 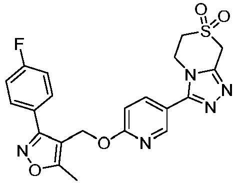 | 156.64 |
| 10 | 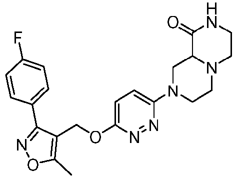 | 19.35 |
| 17 | 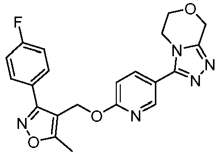 | 33.10 |
| 19 | 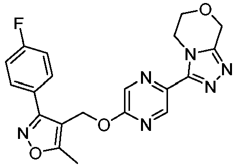 | 15.25 |
| 23 | 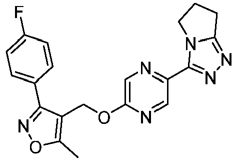 | 39.58 |
| 24 | 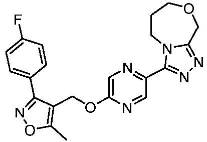 | 18.27 |
| 30 | 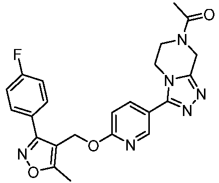 | 191.05 |
| 31 | 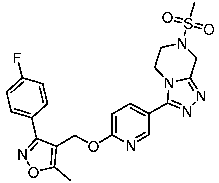 | 32.86 |
| 33 | 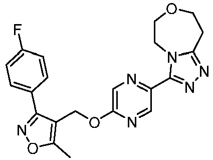 | 39.51 |
| 37 | 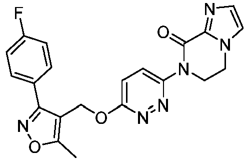 | 60.21 |
| WO2009071476, Example 245 | 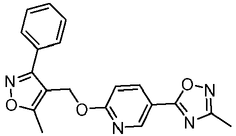 | 0.9 |

Compared with the comparative compound, the compounds of the invention have better solubility and are more suitable for the development of oral drugs and oral absorption.

### 2. Stability of compounds in simulated gastric fluid (SGF)

### (1) Reagent

Simulated gastric fluid (SGF), Manufacturer: Dongguan Chuangfeng Automation Technology Co., Ltd.; Product model: CF-006.

### (2) Instrument

Oscillating constant temperature metal bath, Model: Thermo; Manufacturer: Digital Shaking Drybath.

### (3) Test conditions

Temperature: 37°C; rotating speed: 1000 rpm; sampling time: 0, 4 hours.

### (4) Liquid phase conditions

Chromatographic column: Welch Xtimate C18 3.0 × 100 mm, 5 µm. Mobile phase: A: 0.04% trifluoroacetic acid (TFA)/H₂O (v/v), B: acetonitrile (ACN). Gradient elution was performed according to the following table, flow rate: 1.0 mL/min; injection volume: 5 µL; column temperature: 35°C; wavelength: 254 nm.

| Time (min) | Mobile phase A (%) | Mobile phase B (%) |
|---|---|---|
| 0-10 | 90-25 | 10-75 |
| 10-12 | 25-0 | 75-100 |
| 12-12.1 | 0-90 | 100-10 |
| 15 | STOP | |

### (5) Experimental procedures

(a) preparation of 0.04% TFA/H₂O: 400 µL of TFA was measured, placed into 1L of H₂O, and homogenously mixed;
(b) Sample stock solution: about 3 mg of test sample was weighed, and prepared into a stock solution of 10 mmol/L using DMSO;
(c) 50 µL of the sample stock solution was taken, placed into a 5 mL volumetric flask, made up to the scale with the addition of SGF, and homogenously mixed. 400 µL of the solution was taken, four samples in parallel, and shaken at 1000 rpm and 37°C;
(d) every two samples were taken after 0 and 4 hours respectively, 400 µL of acetonitrile was added, and homogenously mixed, followed by liquid phase injection, and calculation of the change % of the main peak area in the solution after 4 hours relative to 0 hour, two average value reports being taken at each time point.

The test results of the compounds of the invention are shown in the following table.

**Table 4 Stability of compounds in simulated gastric fluid after 4 hours**

| Compound No. | Structure | Residual content in simulated gastric juice after 4 hours (compared with 0 hour content). |
|---|---|---|
| 2 | 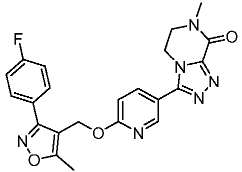 | 46% (4 hours) |
| 3 | 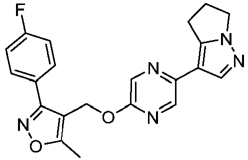 | 102% (4 hours) |
| 4 | 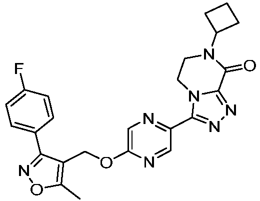 | 89% (4 hours) |
| 5 | 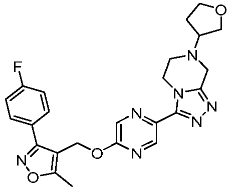 | 43% (4 hours) |
| 9 | 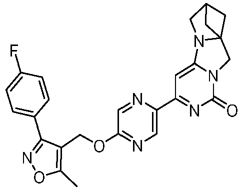 | 64% (4 hours) |
| 12 | 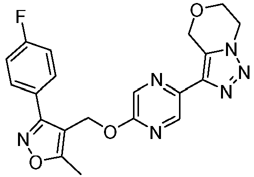 | 93% (4 hours) |
| 16 | 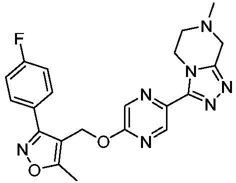 | 76% (4 hours) |
| 17 | 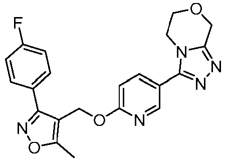 | 65% (4 hours) |
| 18 | 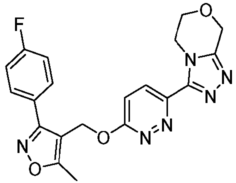 | 51% (4 hours) |
| 19 | 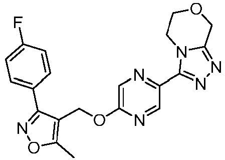 | 94% (4 hours) |
| 23 | 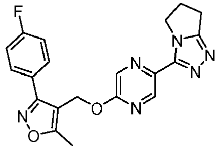 | 80% (4 hours) |
| 24 | 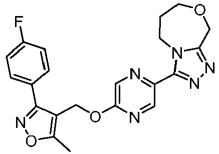 | 89% (4 hours) |
| 26 | 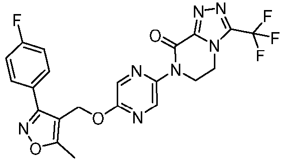 | 80% (4 hours) |
| 27 | 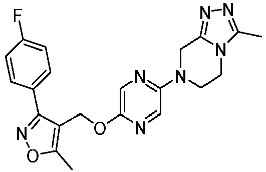 | 63% (4 hours) |
| 32 | 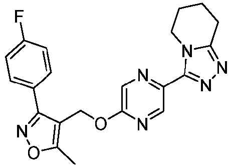 | 83% (4 hours) |
| 33 | 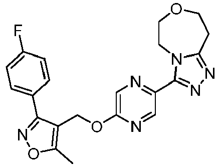 | 91% (4 hours) |
| 38 | 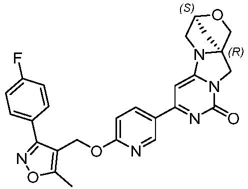 | 75% (4 hours) |
| 40 | 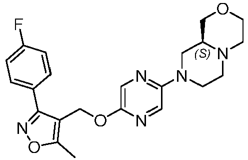 | 98% (4 hours) |
| 41 | 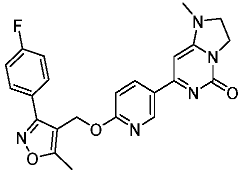 | 35% (4 hours) |
| 44 | 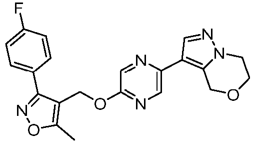 | 94% (4 hours) |
| 45 | 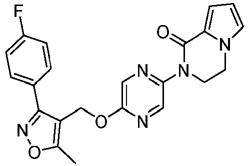 | 87% (4 hours) |
| 46 | 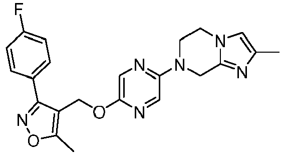 | 87% (4 hours) |
| 47 | 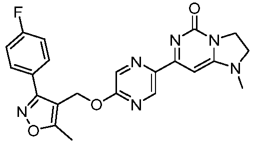 | 82% (4 hours) |
| 48 | 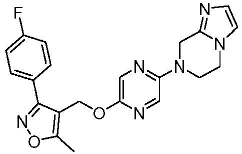 | 88% (4 hours) |
| 49 | 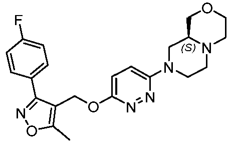 | 31% (4 hours) |
| 50 | 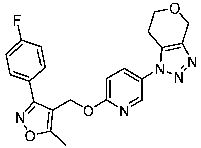 | 82% (4 hours) |
| WO2009071476, Example 112 | 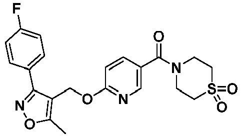 | 2% (4 hours) |
| WO2009071476, Example 109 | 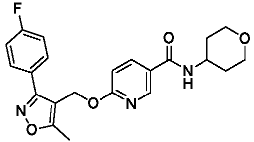 | 15% (4 hours) |
| WO2009071477, Example 63 | 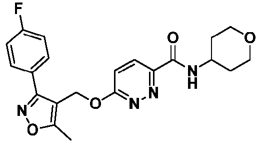 | 0% (4 hours) |

Compared with the coparative compound, the compounds of the invention have better stability in simulated gastric juice, and are more suitable for the development of oral drugs and oral absorption.

Obviously, the above examples are only listed to clearly illustrate, not to limit the embodiments. For those ordinary skilled in the art, other forms of changes or variations can be made based on the above illustration. It is not necessary and impossible to exhaustively list all embodiments here. While obvious changes or variations derivatd therefrom are still within the scope of protection of the invention.

## Claims

1. A compound represented by formula (I) or a pharmaceutically acceptable salt, stereoisomer, tautomer, prodrug, amorphous form, isotopologue, polymorph or solvate thereof: 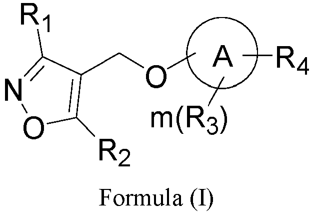
wherein, R₁ is selected from phenyl or pyridyl, wherein phenyl or pyridyl is optionally substituted with 1, 2 or 3 halogens;
R₂ is selected from H, linear or branched C1-C4 alkyl, C1-C4 alkoxy or C3-C6 cycloalkyl;
ring A is selected from a 5-10 membered heterocyclic group containing 1, 2 or 3 ring heteroatoms selected from N, P, O or S;
R₄ is selected from a fused group formed by heterocycle and heterocycle, the heterocycle for forming the fused group being selected from a 5-10 membered heterocyclic group containing 1, 2 or 3 heteroatoms selected from N, P, O or S; the fused group is unsubstituted or is substituted with one or more substituents selected from: optionally substituted alkyl, optionally substituted cycloalkyl, optionally substituted formyl, optionally substituted sulfonyl, or optionally substituted heterocycle containing 1, 2 or 3 heteroatoms; wherein the heteroatom is selected from N, P, O or S;
R₃ is selected from hydrogen, halogen, linear or branched C1-C4 alkyl, C1-C4 alkoxy, C3-C6 cycloalkyl;
m is selected from 0, 1 or 2.

2. The compound as claimed in claim 1, **characterized in that**, ring A is selected from 5-6 membered heterocyclic group containing 1, 2 or 3 nitrogen atoms as ring heteroatoms, preferably ring A is selected from pyridine, pyridazine, pyrazine, pyrimidine, piperidine or piperazine, more preferably ring A is selected from pyridine, pyridazine or pyrazine.

3. The compound as claimed in claim 1, **characterized in that**, R₃ is selected from hydrogen, methyl or methoxy.

4. The compound as claimed in claim 1, **characterized in that**, m is 0 or 1, preferably 0.

5. The compound as claimed in claim 1, **characterized in that**, R₄ has a structure represented by formula (II) or (III): 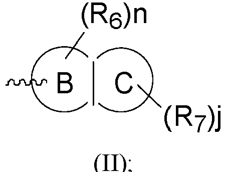 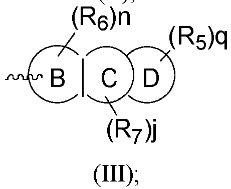
wherein, ring B, ring C or ring D is independently selected from a 5-10 membered heterocyclic group containing 1, 2 or 3 heteroatoms selected from N, P, O or S;
R₅, R₆ or R₇ is independently selected from oxo, optionally substituted alkyl, optionally substituted cycloalkyl, optionally substituted formyl, optionally substituted sulfonyl, or optionally substituted heterocycle containing 1, 2 or 3 heteroatoms; wherein the heteroatom is selected from N, O, S; preferably, R₅, R₆ or R₇ is independently selected from oxo, C1-C6 alkyl, C3-C8 cycloalkyl, formyl, sulfonyl or C3-C8 heterocycle containing 1, 2 or 3 heteroatoms, wherein the heteroatom is selected from N, O, S; wherein the alkyl, cycloalkyl, formyl, sulfonyl or heterocycle is unsubstituted or substituted with 1, 2 or 3 substituents selected from halogen or C1-C3 alkyl;
n, j or q is independently selected from 0, 1 or 2.

6. The compound as claimed in claim 5, **characterized in that**, ring B is selected from a 5-7 membered heterocycle containing 1, 2 or 3 nitrogen atoms as the heteroatoms constituting the ring; preferably, ring B is selected from triazole, pyrazole, imidazole, 6-membered cyclic lactam or diazacyclopentane, wherein the 6-membered cyclic lactam contains two nitrogen atoms as the heteroatoms constituting the ring, more preferably, ring B is selected from

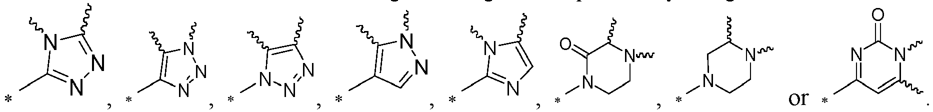

7. The compound as claimed in claim 5, **characterized in that**, ring C is selected from a 5-7 membered heterocycle containing 1-2 heteroatoms independently selected from nitrogen atom, oxygen atom or sulfur atom as the heteroatoms constituting the ring; preferably, ring C is selected from the heterocycles as described in any one of 1)-2) below:
1) pyrrole, imidazole, triazole or 6-membered cyclic lactam, wherein the 6-membered cyclic lactam contains two nitrogen atoms as the heteroatoms constituting the ring;
2) 5-7 membered heterocycloalkanes or heterocycloalkenes containing 1-2 heteroatoms independently selected from nitrogen atom, oxygen atom or sulfur atom as the heteroatoms constituting the ring; more preferably, ring C is selected from

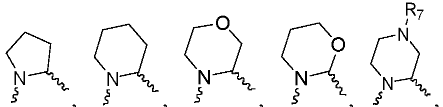

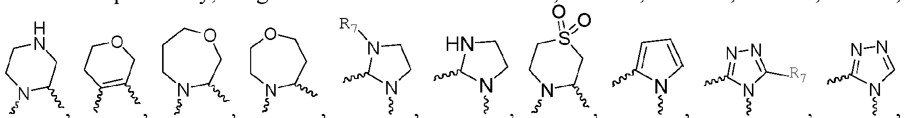

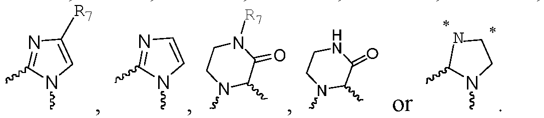

8. The compound as claimed in claim 5, **characterized in that**, ring D is selected from a 5-7 membered heterocycle containing 1-2 heteroatoms independently selected from nitrogen atom, oxygen atom or sulfur atom as the heteroatoms constituting the ring; preferably, ring D is selected from a 5-7 membered bridged ring containing 1 nitrogen atom and 1 oxygen atom or a 5-6 membered bridged ring containing 1 nitrogen atom; more preferably, ring D is selected from

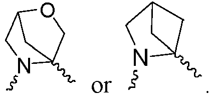

9. The compound as claimed in claim 5 or 7, **characterized in that**, R₇ is selected from C1-C6 alkyl, C3-C8 cycloalkyl, formyl substituted with C1-C3 alkyl, sulfonyl substituted with C1-C3 alkyl, C3-C6 heterocyclic group containing one O atom or one S atom; wherein the alkyl, cycloalkyl or heterocyclic group is unsubstituted or substituted with 1 2 or 3 halogens; preferably, R₇ is selected from methyl, ethyl, -CF₃, -COCH₃,

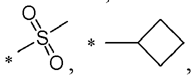

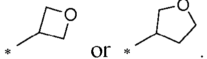

10. The compound as claimed in any one of claims 1-9, **characterized in that**, when R₄ has the structure of formula (II), n is 0 or 1; when R₄ has the structure of formula (III), n is 0 or 1, and q is 0.

11. The compound as claimed in any one of claims 1-10, **characterized in that**, R₁ is selected from phenyl substituted with 1, 2 or 3 halogens.

12. The compound as claimed in any one of claims 1-11, **characterized in that**, R₂ is selected from H, and linear or branched C1-C4 alkyl.

13. The compound as claimed in any one of claims 1-10 **characterized in that** R₁ is 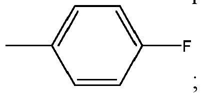 and R₂ is selected from methyl.

14. The compound as claimed in claim 1, **characterized in that**, the compound has a structure represented by the following formula (VI), (V), (IV) or (IIV): 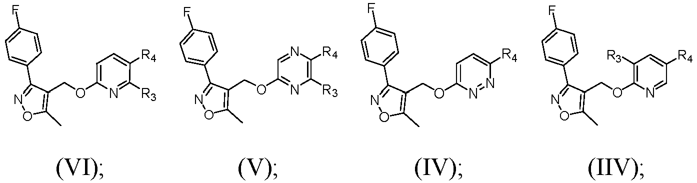 R₃ is as defined in claim 1 or 3, R₄ is as defined in any one of claims 1, 5, 6, 7, 8, 9 or.

15. The compound as claimed in claim 1, **characterized in that**, the compound is selected from any one of the following compounds:

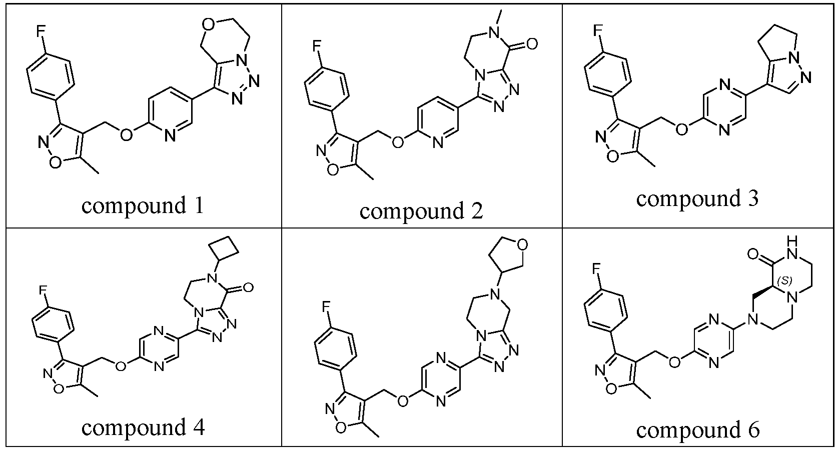

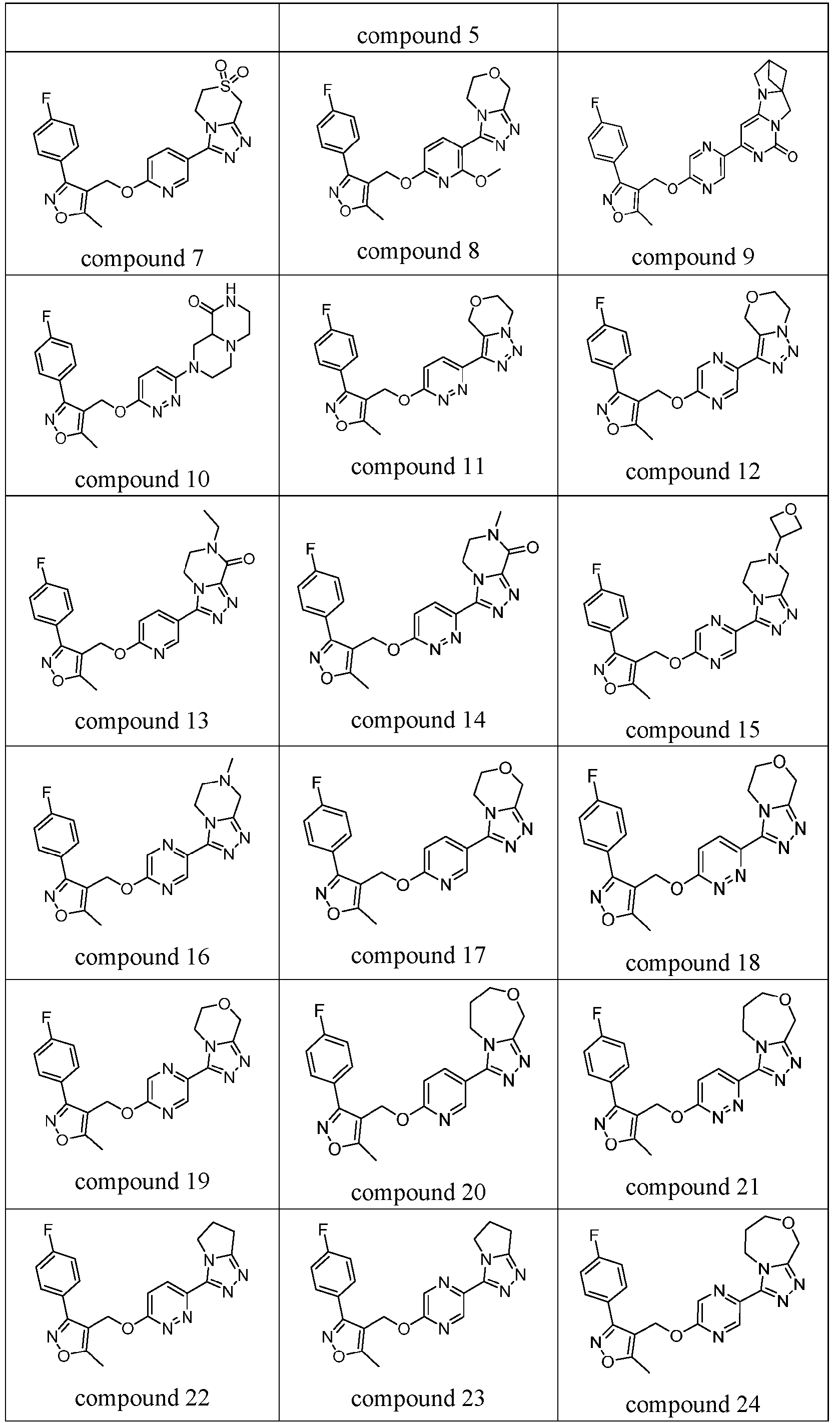

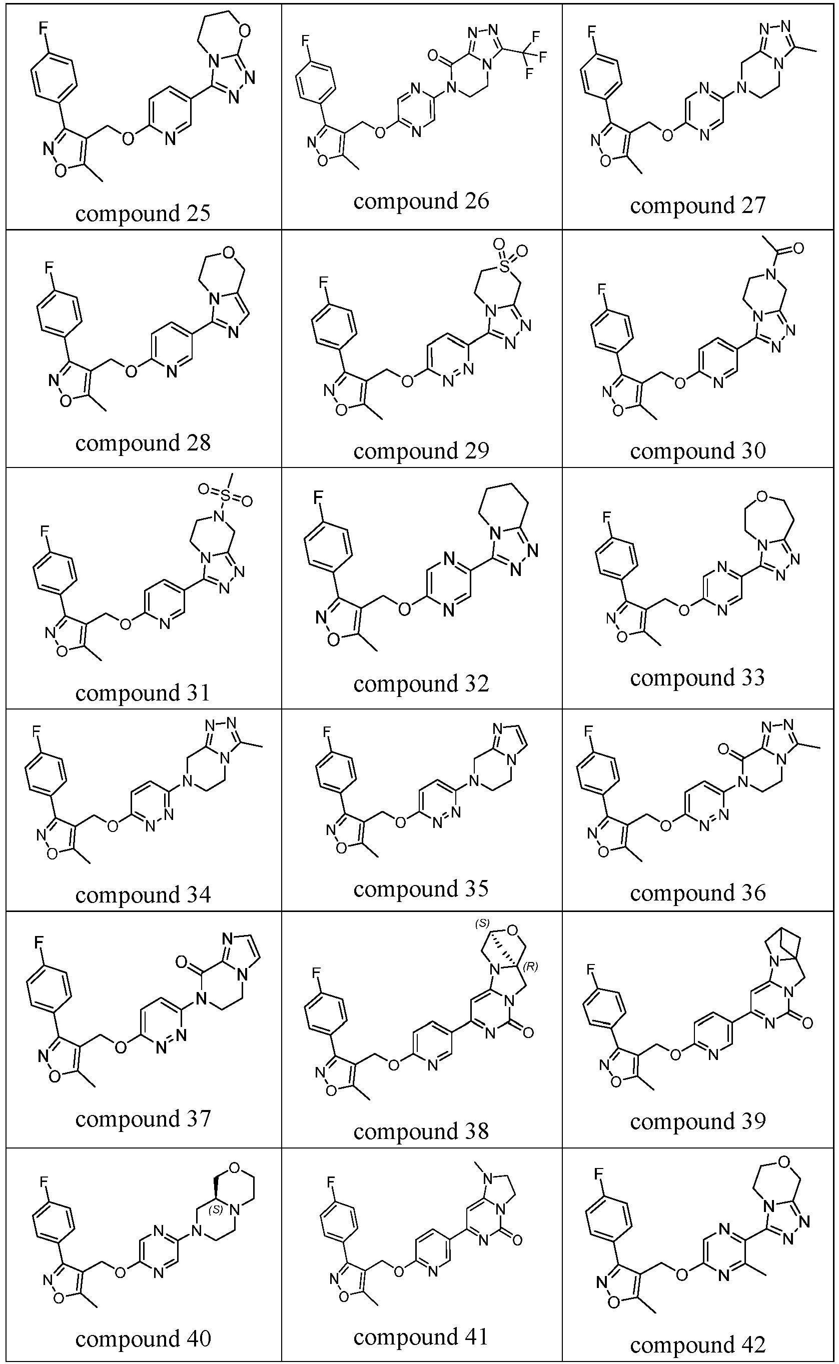

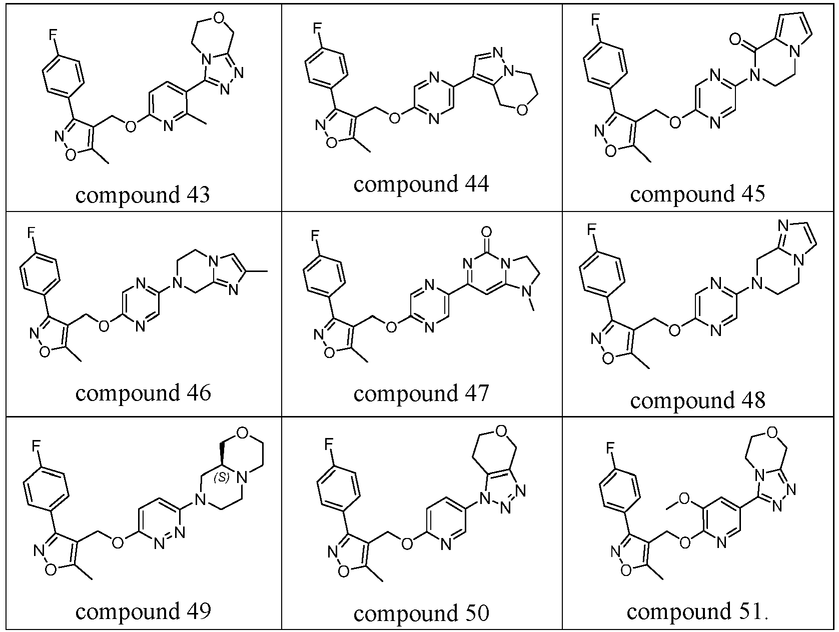

16. A pharmaceutical composition comprising at least one of the compound or a stereoisomer, tautomer, prodrug, pharmaceutically acceptable salt, amorphous form, isotopologue, polymorph or solvate thereof according to any one of claims 1-15, and a pharmaceutically acceptable carrier and/or adjuvant.

17. Use of the compound or a stereoisomer, tautomer, prodrug, pharmaceutically acceptable salt, amorphous form, isotopologue, polymorph or solvate thereof according to any one of claims 1-15, or a pharmaceutical composition according to claim 16 for the preparation of a medicament for treating or preventing a disease associated with α5-GABA_{A} receptor.

18. Use of the compound or a stereoisomer, tautomer, prodrug, pharmaceutically acceptable salt, amorphous form, isotopologue, polymorph or solvate thereof according to any one of claims 1-15, or a pharmaceutical composition according to claim 16 for the preparation of a medicament for treating or preventing the following diseases: depression, pain, Alzheimer's disease, multi-infarct dementia, stroke, anxiety disorder, generalized anxiety disorder, panic disorder, agoraphobia, post-traumatic stress disorder, premenstrual dysphoric disorder, fibromyalgia, attention deficit hyperactivity disorder, obsessive-compulsive disorder, social anxiety disorder, autism, autistic disorder, schizophrenia, obesity, bulimia nervosa or anorexia nervosa, Tourette's syndrome, vasomotor flushing, sexual dysfunction, borderline personality disorder, chronic fatigue syndrome, Reynaud's syndrome, Parkinson's disease, epilepsy, or mood disorder following head injury.
